# EUROPÄISCHE PATENTSCHRIFT

(11) **EP 1 759 735 B1**
(45) Veröffentlichungstag und Bekanntmachung des Hinweises auf die Patenterteilung: **05.12.2007**
(21) Anmeldenummer: 06017849.8
(22) Anmeldetag: 26.08.2006
(51) Int. Cl.: A61Q 5/06, A61Q 5/08, A61Q 5/10

(54) **Verfahren zur Selektiven Färbung keratinhaltiger Fasern**
Method of selectively dyeing portions of hair
Méthode pour la coloration sélective des cheveux

(30) Priorität: 02.09.2005 DE 102005042051
(43) Veröffentlichungstag der Anmeldung: 07.03.2007
(73) Patentinhaber: Henkel Kommanditgesellschaft auf Aktien, 40589 Düsseldorf (DE)
(72) Erfinder: Seiler, Martina, 47228 Duisburg (DE); Günther, Katja, 40721 Hilden (DE); Wacquez, Anne-Laure, 142890 Saint Contesth (FR)

(56) Entgegenhaltungen:
- EP-A- 1 419 709
- DE-C- 853 806
- US-A- 2 655 924
- US-A- 2 819 721
- US-A- 5 146 937
- US-A1- 2003 135 937

## Beschreibung

Die vorliegende Erfindung betrifft ein Verfahren zur Färbung keratinhaltiger Fasern, insbesondere menschlicher Haare, in welchem selektiv aus einer Menge keratinhaltiger Fasern eine oder mehrere Teilmengen der Fasern mit einem Nuanciermittel behandelt und jede dieser behandelten Teilmengen jeweils in eine röhrenförmige Hülse eingebracht wird. Weiterhin ist eine Verpackungseinheit (Kit), enthaltend ein Nuanciermittel, sowie entsprechende Röhren Gegenstand der Erfindung sowie die Verwendung dieses Kits in dem erfindungsgemäßen Färbeverfahren.

Menschliches Haar wird heute in vielfältiger Weise mit haarkosmetischen Zubereitungen behandelt. Dazu gehören etwa die Reinigung der Haare mit Shampoos, die Pflege und Regeneration mit Spülungen und Kuren sowie das Bleichen, Färben und Verformen der Haare mit Bleich-, Tönungs-, Well- bzw. Stylingmitteln.

Die Veränderung oder Nuancierung der Farbe des Kopfhaares spielt in der Haarbehandlung eine herausragende Rolle. Bekanntermaßen existieren zahlreiche Verfahren und Hilfsmittel mit Hilfe derer sich eine Farbveränderung unifarben am gesamten Kopfhaar, oder an Teilbereichen unter Erhalt einer Multiton-Haarfarbe bewerkstelligen lässt. Für die Farbveränderung stehen dem Anwender viele kosmetische Zubereitungen zu Verfügung. Sieht man von den Blondiermitteln, die eine oxidative Aufhellung der Haare durch Abbau der natürlichen Haarfarbstoffe bewirken, ab, so sind im Bereich der Haarfärbung im wesentlichen vier Typen von Haarfärbemitteln von Bedeutung:

Für dauerhafte, intensive Färbungen mit entsprechenden Echtheitseigenschaften werden sogenannte Oxidationsfärbemittel verwendet. Solche Färbemittel enthalten üblicherweise Oxidationsfarbstoffvorprodukte, sogenannte Entwicklerkomponenten und Kupplerkomponenten. Die Entwicklerkomponenten bilden unter dem Einfluß von Oxidationsmitteln oder von Luftsauerstoff untereinander oder unter Kupplung mit einer oder mehreren Kupplerkomponenten die eigentlichen Farbstoffe aus. Die Oxidationsfärbemittel zeichnen sich zwar durch hervorragende, lang anhaltende Färbeergebnisse aus. Für natürlich wirkende Färbungen muß aber üblicherweise eine Mischung aus einer größeren Zahl von Oxidationsfarbstoffvorprodukten eingesetzt werden; in vielen Fällen werden weiterhin direktziehende Farbstoffe zur Nuancierung verwendet.

Als Entwicklerkomponenten werden üblicherweise primäre aromatische Amine mit einer weiteren, in para- oder ortho-Position befindlichen, freien oder substituierten Hydroxy- oder Aminogruppe, Diaminopyridinderivate, heterozyklische Hydrazone, 4-Aminopyrazolonderivate sowie 2,4,5,6-Tetraaminopyrimidin und dessen Derivate eingesetzt.

Spezielle Vertreter sind beispielsweise p-Phenylendiamin, p-Toluylendiamin, 2,4,5,6-Tetraaminopyrimidin, p-Aminophenol, N,N-Bis(2-hydroxyethyl)-p-phenylendiamin, 2-(2,5-Diaminophenyl)-ethanol, 2-(2,5-Diaminophenoxy)-ethanol, 1-Phenyl-3-carboxyamido-4-amino-pyrazolon-5, 4-Amino-3-methylphenol, 2-Aminomethyl-4-aminophenol, 2-Hydroxy-4,5,6-triaminopyrimidin, 2,4-Dihydroxy-5,6-diaminopyrimidin, 2,5,6-Triamino-4-hydroxypyrimidin und 1,3-N,N'-Bis(2-hydroxyethyl)-N,N'-bis(4-aminophenyl)-diaminopropan-2-ol.

Als Kupplerkomponenten werden in der Regel m-Phenylendiaminderivate, Naphthole, Resorcin und Resorcinderivate, Pyrazolone und m-Aminophenole verwendet. Als Kupplersubstanzen eignen sich insbesondere 1-Naphthol, 1,5- Dihydroxynaphthalin, 2,7-Dihydroxynaphthalin und 1,7-Dihydroxynaphthalin, 5-Amino-2-methylphenol, m-Aminophenol, Resorcin, Resorcinmonomethylether, m-Phenylendiamin, 1-Phenyl-3-methylpyrazolon-5, 2,4-Dichlor-3-aminophenol, 1,3-Bis-(2,4-diaminophenoxy)-propan, 2-Chlorresorcin, 4-Chlorresorcin, 2-Chlor-6-methyl-3-aminophenol, 2-Methylresorcin, 5-Methylresorcin und 2-Methyl-4-chlor-5-aminophenol.

Für temporäre Färbungen werden üblicherweise Färbe- oder Tönungsmittel verwendet, die als färbende Komponente sogenannte Direktzieher enthalten. Hierbei handelt es sich um Farbstoffmoleküle, die direkt auf das Haar aufziehen und keinen oxidativen Prozeß zur Ausbildung der Farbe benötigen. Zu diesen Farbstoffen gehört beispielsweise das bereits aus dem Altertum zur Färbung von Körper und Haaren bekannte Henna. Diese Färbungen sind gegen Shampoonieren in der Regel deutlich empfindlicher als die oxidativen Färbungen, so daß dann sehr viel schneller eine vielfach unerwünschte Nuancenverschiebung oder gar eine sichtbare "Entfärbung" eintritt.

Schließlich hat weiteres Färbeverfahren große Beachtung gefunden. Bei diesem Verfahren werden Vorstufen des natürlichen Haarfarbstoffes Melanin auf das Haar aufgebracht; diese bilden dann im Rahmen oxidativer Prozesse im Haar naturanaloge Farbstoffe aus. Ein solches Verfahren mit 5,6-Dihydroxyindolin als Farbstoffvorprodukt wurde in der EP-B1-530 229 beschrieben. Bei, insbesondere mehrfacher, Anwendung von Mitteln mit 5,6-Dihydroxyindolin ist es möglich, Menschen mit ergrauten Haaren die natürliche Haarfarbe wiederzugeben. Die Ausfärbung kann dabei mit Luftsauerstoff als einzigem Oxidationsmittel erfolgen, so daß auf keine weiteren Oxidationsmittel zurückgegriffen werden muß. Bei Personen mit ursprünglich mittelblondem bis braunem Haar kann das Indolin als alleinige Farbstoffvorstufe eingesetzt werden. Für die Anwendung bei Personen mit ursprünglich roter und insbesondere dunkler bis schwarzer Haarfarbe können dagegen befriedigende Ergebnisse häufig nur durch Mitverwendung weiterer Farbstoffkomponenten, insbesondere spezieller Oxidationsfarbstoffvorprodukte, erzielt werden.

Eine weitere Möglichkeit keratinhaltige Fasern zu färben, bietet die Verwendung von Färbemitteln, die eine Kombination aus Komponente
- A: Verbindungen, die eine reaktive Carbonylgruppe enthalten mit Komponente
- B: Verbindungen, ausgewählt aus (a) CH-aciden Verbindungen, (b) Verbindungen mit primärer oder sekundärer Aminogruppe oder Hydroxygruppe, ausgewählt aus primären oder sekundären aromatischen Aminen, stickstoffhaltigen heterozyklischen Verbindungen und aromatischen Hydroxyverbindungen, (c) Aminosäuren, (d) aus 2 bis 9 Aminosäuren aufgebauten Oligopeptiden
enthalten. Die entsprechende Färbemethode (im folgenden Oxofärbung genannt) wird beispielsweise in den Druckschriften WO-A1-99/18916, WO-A1-00/38638, WO-A1-01/34106 und WO-A1-01/47483 beschrieben. Die resultierenden Färbungen besitzen teilweise Farbechtheiten auf der keratinhaltigen Faser, die mit denen der Oxidationsfärbung vergleichbar sind. Das mit der schonenden Oxofärbung erzielbare Nuancenspektrum ist sehr breit und die erhaltene Färbung weist oftmals eine akzeptable Brillanz und Farbtiefe auf. Die vorgenannten Komponenten A und B, im weiteren als Oxofarbstoffvorprodukte bezeichnet, sind im allgemeinen selbst keine Farbstoffe, und eignen sich daher jede für sich genommen allein nicht zur Färbung keratinhaltiger Fasern. In Kombination bilden sie in einem nichtoxidativen Prozess Farbstoffe aus. Unter Verbindungen der Komponente B können allerdings auch entsprechende Oxidationsfarbstoffvorprodukte vom Entwickler- und/oder Kupplertyp mit oder ohne Einsatz eines Oxidationsmittels Verwendung finden. Somit läßt sich die Methode der Oxofärbung ohne weiteres mit dem oxidativen Färbesystem kombinieren.

Dem Fachmann sind Verfahren bekannt, in denen das Haar an selektiven Stellen mit einem Färbemittel unter Ausbildung farbiger Strähnen behandelt wird. Ferner sind Verfahren bekannt, in denen das Haar an selektiven Stellen durch Applikation von sogenannten Blondiermitteln aufgehellt bzw. gebleicht wird. Das Resultat dieser Verfahren sind gefärbte bzw. aufgehellte Strähnchen oder Haarspitzen.

Die in Bleichmitteln enthaltenen Oxidationsmittel wirken auf den natürlichen Haarfarbstoff Melanin und gegebenenfalls auf in der Faser befindliche synthetische Farbstoffe oxidativ ein und verursachen dadurch eine Farbveränderung und optimalerweise eine Aufhellung der Haarfarbe. Die Grundlagen der Blondier- und oxidativen Färbeverfahren sind dem Fachmann bekannt und in einschlägigen Monographien, z.B. von K. Schrader, Grundlagen und Rezepturen der Kosmetika, 2. Auflage, 1989, Dr. Alfred Hüthig Verlag, Heidelberg, oder W. Umbach (Hrg.), Kosmetik, 2. Auflage, 1995, Georg Thieme Verlag, Stuttgart, New York, zusammenfassend beschrieben.

Zum Aufhellen bzw. Ultrablondieren menschlicher Haare - insbesondere für die Strähnchenapplikation - werden üblicherweise feste oder pastenförmige Zubereitungen mit festen Oxidationsmitteln unmittelbar vor der Anwendung mit einer verdünnten Wasserstoffperoxidlösung vermischt. Diese Anwendungsmischung wird dann auf das Haar aufgebracht und nach einer bestimmten Einwirkzeit wieder ausgespült. Die herkömmlichen gebrauchsfertigen Bleichmittel für keratinhaltige Fasern enthalten neben Wasserstoffperoxid meist Peroxidisulfatverbindungen zur Steigerung der Aufhellleistung und besitzen bei der Anwendung auf der Faser einen pH-Wert von größer pH 9. Allerdings ist das Resultat eines Aufhellvorgangs von dem Resultat des Ultrablondierens zu unterscheiden. Während beim Ultrablondiervorgang möglichst alle Farbpigmente unter Erhalt einer nahezu weiß gefärbten Haarfaser oxidativ beeinflußt werden, soll bei einer oxidativen Aufhellung die existierende Färbung derart nuanciert werden, daß die Ausgangsfarbe nach wie vor erkennbar ist, jedoch für den Betrachter hellergefärbt erscheint.

Die gezielte Nuancierung keratinhaltiger Fasern läuft in ihrer Durchführung nicht ohne Probleme ab. Oftmals lassen sich keine scharfen Farbkontraste erzielen, weil die nuancierten Haarbereiche während der Einwirkzeit des Nuanciermittels mit dem restlichen Haar in Kontakt treten. Ferner ist es schwierig, wohldefinierte Teile des Kopfhaares wie z.B. Strähnchen gleichmäßig zu nuancieren und diese wohldefinierten Teile dabei möglichst gleichmäßig über den gesamten Kopfhaarbereich zu verteilen. Zusammenfassend bedeutet dies beispielsweise für eine Strähnchennuancierung, daß eine Haarfaser gleichmäßig vom Haaransatz bis in die Spitzen nuanciert werden muß. Dabei soll beachtet werden, dass die benachbarten keratinhaltigen Fasern nicht mit dem Nuanciermittel in Berührung kommen, um scharfe Kontraste zu erhalten.

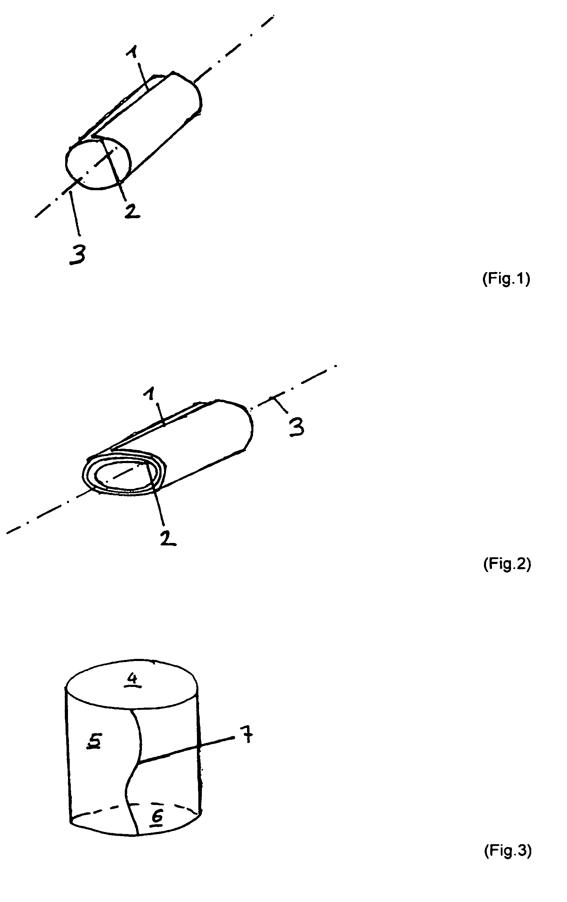

Aufgrund der Komplexität der Verfahrensschritte einer Nuancierung von Teilbereichen des Haars, führt meist ein Frisör diese Nuancierung durch. Eine Nuancierung kann ebenso durch Anwendung der im Handel angebotenen Nuanciermittel für den Heimgebrauch erzielt werden. Letztere Mittel werden meist zusammen mit Applikationshilfen bereitgestellt, die die selektive Nuancierung einzelner Haarbereiche mittels Selbstapplikation durch den Heimanwender erleichtern sollen. Zu diesen Hilfen zählt z.B. eine Strähnenhaube, beispielsweise eine Haube mit Lochraster gemäß Fig. 1 der US-A-3468318, Folien, oder ein kleiner Pinsel bzw. eine Mascarabürste.

Die Anwendung eines Pinsels bzw. einer Mascarabürste erleichtert das Auftragen des Nuanciermittels auf den ausgewählten Haarbereich. Allerdings treten die mit dem Nuanciermittel benetzten Haarbereiche während der Einwirkungszeit mit den nicht benetzten Haarbereichen in Kontakt, wodurch die Nuancierung kontrastarm ausfällt.

Die Anwendung unter Zuhilfenahme einer Strähnenhaube liefert kontrastreiche Strähnen. Der Proband setzt sich die Haube mit Lochraster auf den Kopf. Anschließend muß er mit einem Haken in Form einer Häkelnadel Haarsträhnen durch ein dafür vorgesehenes Loch des Lochrasters der Haube ziehen. Die Durchführung dieses Haubenverfahrens ist für den Probanden selbst, insbesondere im Rahmen der Auswahl von Strähnen am Hinterkopf, schwierig.

Die Verwendung von Folien zur Abdeckung der mit dem Nuanciermittel behandelten Faserbereiche bietet zwar eine weitere Möglichkeit der Erzeugung kontrastreicher Strähnen. Allerdings können die Folien schlecht am Haar befestigt werden. Bei Anwendung auch von teilweise mit Kunststoffen beschichteten Metallfolien kann insbesondere bei oxidationsmittelhaltigen Nuanciermitteln eine unerwünschte Zersetzungsreaktion des Oxidationsmittels unter Wärmeentwicklung einsetzen. In einem solchen Fall kann sich unter der metallhaltigen Folie ein Hitzestau bilden, der zu unerwünschten Nuancierergebnissen, zu Kopfhautreizungen sowie zu einer stark beschleunigten Degenerierung der keratinhaltigen Faser führen kann.

Aufgabe der vorliegenden Erfindung ist es daher, ein sicheres Verfahren für den Heimanwender bereitzustellen, welches eine gleichmäßig nuancierte Farbgebung von Teilbereichen keratinhaltiger Fasern gewährleistet und über den gesamten Kopfhaarbereich gleichmäßige und kontrastreiche Farbakzente unter Erhalt einer Multitonhaarfarbe ergibt. Die Durchführung sollte einfach sein und dem Heimanwender eine Selbstapplikation ermöglichen.

Es wurde überraschenderweise gefunden, daß sich, insbesondere auch durch Selbstapplikation des Heimanwenders, über den gesamten Fasermengenbereich gleichmäßige und kontrastreiche Farbakzente unter Erhalt einer Multitonhaarfarbe erzielen lassen, wenn der Anwender die Teilbereiche des Haars während der Einwirkzeit des Nuanciermittels in eine spezielle Hülse in Form eines rohrförmigen Körpers einbringt.

Ein erster Gegenstand der Erfindung ist daher ein Verfahren zum Färben keratinhaltiger Fasern, insbesondere menschlicher Haare, in welchem
(i) aus einer Menge keratinhaltiger Fasern eine Teilmenge abgeteilt wird,
(ii) auf diese Teilmenge vollständig oder teilweise ein Nuanciermittel aufgetragen wird,
(iii) die Teilmenge in einen rohrförmigen Körper eingebracht wird,
(iv) gegebenenfalls die Schritte (i) bis (iii) unter Auswahl weiterer Teilmengen und Verwendung weiterer rohrförmiger Körper wiederholt werden,
(v) nach Ablauf einer Einwirkzeit Z1 alle rohrförmigen Körper entfernt werden und das Nuanciermittel abgespült wird,
wobei
- die rohrförmigen Körper als Ausgangsform die Form eines bereits vorgefertigten Hohlzylinders besitzen, der an Grund- und Deckfläche offen ist und durch einen Mantel begrenzt wird, wobei der Mantel eine Naht, verlaufend über die vollständige Höhe des Hohlzylinders entlang der äußeren Fläche des Mantels (Mantelfläche) aufweist,
- die Ausgangsform ohne äußere Krafteinwirkung beständig ist,
- die rohrförmigen Körper sich zusätzlich unter äußerer Krafteinwirkung zu einer geöffneten Form entlang der Naht öffnen lassen und
- die rohrförmigen Körper aus der geöffneten Form heraus ohne äußere Krafteinwirkung selbsttätig ihre Ausgangsform annehmen.

Unter keratinhaltigen Fasern werden im Rahmen dieser Anmeldung Pelze, Wolle, Federn und insbesondere menschliche Haare verstanden.

Das Nuanciermittel wird im Rahmen des erfindungsgemäßen Verfahrens auf eine oder mehrere Teilmengen der Gesamtmenge an keratinhaltigen Fasern aufgetragen. Dies bedeutet, daß aus der Menge aller Fasern eine oder bei Wiederholungen der Schritte (i) bis (iii) in Summe mehrere Teilmengen in Form eines Faserbündels (Strähne) behandelt werden. Zur Erzielung gleichmäßig verteilter Farbeffekte auf menschlichem Haar sollte darauf geachtet werden, dass bei der Abteilung der Faserbündel nicht nur Haare aus dem Deckhaar, sondern auch aus den unter dem Deckhaar liegenden Haarbereichen ausgewählt werden. Ferner werden bei Wiederholung der Schritte (i) bis (iii) die ausgewählten Faserbündel bevorzugt möglichst gleichmäßig über die Fläche des gesamten Kopfhaarbereichs selektiert.

Das ausgewählte Faserbündel wird entweder vollständig oder teilweise mit dem Nuanciermittel behandelt. Bei einer vollständigen Behandlung handelt es sich um eine so genannte Strähnchenapplikation. Bei einer partiellen Behandlung lassen sich beispielsweise im Rahmen des so genannten "Tipping" bzw. "Frosting" die Faserspitzen oder andere ausgewählte Bereiche des Faserbündels selektiv nuancieren. Wenn mehrere Teilmengen nuanciert werden, so ist es möglich, jede Teilmenge mit einem anderen Nuanciermittel zu behandeln. Es ist aber erfindungsgemäß bevorzugt, die Nuancierung einer abgeteilten Teilmenge unter Anwendung eines Nuanciermittels vorzunehmen, welches aus einer Menge von einem bis höchstens vier voneinander verschiedenen Nuanciermitteln ausgewählt wird. Bei Anwendung von mehr als einem Nuanciermittel in dem erfindungsgemäßen Verfahren kann jedem Nuanciermittel eine Kennzeichnung zugeordnet werden, wie beispielsweise ein Farbcode oder ein Zahl- oder Buchstabencode. Diese Kennzeichnung ist auf dem Container angebracht, der das entsprechende Nuanciermittel enthält.

Das Nuanciermittel kann prinzipiell mit den Händen unter Verwendung von entsprechend geeigneten Schutzhandschuhen auf das selektierte Faserbündel appliziert werden. Das Nuanciermittel wird jedoch bevorzugt mit einem Applikator, wie beispielsweise einer Bürste, einem Pinsel oder einer Applicette auf die zur Nuancierung vorgesehenen Haarbereiche aufgetragen. Unter einer Applicette wird ein breiter Pinsel verstanden, an dessen Stielende sich eine Spitze befindet, die das Abteilen von Faserbündeln bzw. Strähnchen aus der Gesamtmenge der Fasern erlaubt und vereinfacht.

Es ist bevorzugt, insbesondere bei der Durchführung der Nuancierung an kurzem Haar bis 20 cm Länge oder zur Nuancierung der Haarspitzen, als Applikator eine Rundbürste zu verwenden. Eine Rundbürste besitzt einen Stiel und einen Borstenkopf. Die Borsten des Borstenkopfes sind bei einer Rundbürste in Form eines Zylinders um einen zentralen Körper des Borstenkopfes angeordnet, wobei die Summe der befestigten Borsten mit einem Borstenende eine im wesentlichen zylindrische Außenfläche beschreibt während sie mit dem anderen Ende am Körper des Borstenkopfes befestigt ist. Der Körper des Borstenkopfes ist wiederum an einem Ende des Stils der Rundbürste positioniert oder ist ein Teil eines Endbereichs des Stils. Die in dem erfindungsgemäßen Verfahren verwendete Rundbürste besitzt bevorzugt Borsten mit einer Länge von maximal 2 cm und hat einen Durchmesser des zylindrischen Borstenkopfes von maximal 2,5 cm. Der Zylinder des Borstenkopfes hat eine Länge von maximal 4 cm. Zur Auftragung des Nuanciermittels eignet sich hervorragend eine Mascarabürste.

Wenn das Nuanciermittel auf das Faserbündel aufgetragen wurde, wird das Faserbündel in einen rohrförmigen Körper eingebracht. Zu diesem Zweck wird der rohrförmige Körper durch äußere Krafteinwirkung an der Naht des Mantels geöffnet und das Faserbündel durch die dabei entstehende Öffnung in das innere des rohrförmigen Körpers eingebracht. Ohne die äußere Krafteinwirkung verschließt sich der geöffnete rohrförmige Körper selbsttätig und nimmt seine Ausgangsform ein. Das Faserbündel wird von dem Mantel des rohrförmigen Körpers umschlossen, d.h. es orientiert sich mit seiner längsten Ausdehnung im Inneren des rohrförmigen Körpers entlang der Höhe des Hohlzylinders und durchstößt die Grund- und/oder Deckfläche an deren Öffnung.

Erfindungsgemäß ist es auch möglich, den rohrförmigen Körper entlang der Naht durch äußere Krafteinwirkung zu öffnen, das abgeteilte Faserbündel unter Beibehaltung der Krafteinwirkung durch die Öffnung derart auf die Mantelfläche des nach wie vor offenen röhrenförmigen Körpers aufzulegen, dass die längste Ausdehnung des Faserbündels in etwa entlang der Höhe der Mantelfläche liegt. Anschließend kann das Nuanciermittel durch die Öffnung entlang der Naht auf das Faserbündel appliziert werden. Nach vollendeter Applikation wird die Krafteinwirkung auf den rohrförmigen Körper beendet. Der rohrförmige Körper verschließt sich sodann selbsttätig, nimmt seine Ausgangsform ein und umschließt dabei das Faserbündel. Das mit dem Nuanciermittel behandelte Faserbündel wurde auf diese Weise in den rohrförmigen Körper eingebracht.

Bevorzugt ist zu beachten, dass sich wenigstens derjenige Teil des Faserbündels in dem rohrförmigen Körper befindet, der mit dem Nuanciermittel behandelt wurde. Diese bevorzugte Maßgabe sollte bei einer Haarnuancierung am Probanden mit langem Haar für eine quantitative Abdeckung der Haarlängen beachtet werden. Wenn sich am Haaransatz bis zu 1 cm behandeltes Haar nicht in dem röhrenförmigen Körper befindet, ist dies mit Blick auf die Lösung der erfindungsgemäßen Aufgabe tolerabel. Gleiches gilt für die behandelten Haarspritzen.

Wenn in dem erfindungsgemäßen Verfahren mehrere Nuanciermittel Anwendung finden, ist es bevorzugt, das Faserbündel in einen rohrförmigen Körper einzubringen, der die gleiche Kennzeichnung, bzw. den gleichen Farb-, Zahlen oder Buchstabencode, trägt, wie der Container des auf dieses Faserbündel applizierten Nuanciermittels. Die Kennzeichnung befindet sich bevorzugt auf der Mantelfläche des rohrförmigen Körpers. Auf diese Weise wird jede behandelte Teilmenge der keratinhaltigen Fasern entsprechend gekennzeichnet. Der Anwender behält dadurch den Überblick über die Verteilung der einzelnen Nuanciermittel über die gesamte Menge keratinhaltiger Fasern und ist somit in der Lage, das farbliche Gesamtergebnis der Multitonhaarfarbe besser zu steuern.

In Schritt (v) können entweder die Hülsen abgenommen und dann gespült werden. Es ist aber auch denkbar, die Hülsen gleichzeitig während des Spülvorganges abzuziehen.

Der im erfindungsgemäßen Verfahren verwendete rohrförmige Körper besitzt erfindungsgemäß die vorgefertigte Form eines Hohlzylinders, die ohne eine äußere Krafteinwirkung beständig ist.

Ein Hohlzylinder ist erfindungsgemäß der hohle Abschnitt innerhalb eines geometrischen Körpers, der die Form eines Zylinders aufweist. Ein Zylinder ist erfindungsgemäß ein hohler Körper, der von einem Mantel, einer Grundfläche und einer Deckfläche begrenzt wird. Die Grundfläche hat einen Abstand von der Deckfläche, der als Höhe definiert wird. Die Grund- und Deckfläche sind erfindungsgemäß bevorzugt kongruent. Wiederum bevorzugt sind Grund- und Deckfläche parallel zueinander angeordnet.

Der Hohlzylinder des in dem erfindungsgemäßen Verfahren verwendeten rohrförmigen Körpers ist an den durch die Grund- und Deckfläche begrenzten Seiten offen. Die Öffnung an der Grundfläche und die Öffnung an der Deckfläche des Hohlzylinders besitzen bevorzugt jeweils eine Größe von mindestens 1 mm² bis zur gesamten Größe der Grund- beziehungsweise Deckfläche. Wenn sich die Öffnung über die gesamte Grund- bzw. Deckfläche erstreckt ist es besonders gut möglich, mehrere rohrförmige Körper ineinander zu stecken. Dies kann erforderlich sein, wenn die keratinhaltigen Fasern länger als die Höhe des Hohlzylinders sind.

Die Grund- und die Deckfläche können beliebige Formen annehmen, wie beispielsweise Dreieck, Viereck (Rechteck oder Quadrat), Fünfeck, Sechseck, Siebeneck, n-Eck mit n = 12 bis 20, Ellipse, Kreis, Tropfenform oder dem Schattenriss eines Gegenstandes oder eines Lebewesens. Erfindungsgemäß bevorzugte Formen der besagten Flächen sind runde Formen, wie die die Ellipse oder der Kreis sowie das n-Eck mit n=12 bis 20. Besonders bevorzugt sind die Flächen elliptisch oder kreisrund. Das n-Eck mit n=12 bis 20 Ecken bietet einen erfindungsgemäßen rohrförmigen Körper, der sich insbesondere mit nassen Händen im Rahmen des erfindungsgemäßen Verfahrens perfekt handhaben lässt.

Die Mantelfläche ist die äußere Fläche des Mantels. Sie bildet erfindungsgemäß die Summe der Seitenflächen des rohrförmigen Körpers, die den Umfang der Grundfläche und zugleich den Umfang der Deckfläche umschließt und durch Grund- und Deckfläche begrenzt werden.

Darüber hinaus besitzt der rohrförmige Körper eine Naht über die vollständige Höhe des Hohlzylinders entlang seiner Mantelfläche. Unter einer Naht wird erfindungsgemäß ein Bereich des Mantels verstanden, an dem das Material des Mantels eine Öffnung des Mantels entlang der Mantelfläche zulässt. Entlang der Naht kann sich ein Verschluß, beispielsweise ein Klettverschluß oder ein Klemmverschluß, befinden.

Exemplarisch wird in Fig. 3 ein rohrförmiger Körper mit einer runden Grundfläche (6) und einer runden Deckfläche (4) dargestellt. Grund- und Deckfläche sind immateriell, d.h. vollständig geöffnet. Die Mantelfläche (5) besitzt eine Naht (7), welche sich über die vollständige Höhe entlang der Mantelfläche (5) erstreckt.

In einer bevorzugten Ausführungsform wird der Mantel des rohrförmigen Körpers aus einem planaren Material durch Aufrollen gebildet. Das planare Material hat bevorzugt die Form eines Rechtecks. Mindestens zwei Möglichkeiten führen zu einem rohrförmigen Körper durch Aufrollen:

Das planare Material kann während des Aufrollvorgangs einerseits entsprechend (beispielsweise um ca. 360° bis 380°) um eine imaginäre Achse, die das planare Material nicht schneidet, herumgebogen werden, so dass sich zwei gegenüberliegende (bevorzugt parallel zueinander stehende) Kanten des planaren Materials berühren oder sich vollständig überlappen. Dabei haben besagte Kanten in der Ebene der Deck- bzw. Grundfläche, sowie in der Ebene der Mantelfläche des resultierenden Hohlzylinders einen geringfügigen Abstand von höchstens 0,35 cm, bevorzugt von höchstens 0,2 cm. Bei der Überlappung ist es bevorzugt, dass eine der beiden besagten Kanten die innere Fläche des Mantels, bevorzugt vollständig, berührt. Die Überlappung beträgt in dieser Ausführungsform bevorzugt nicht mehr als 0,5 cm von besagter Kante zu besagter Kante entlang der Ebene der Mantelfläche. Die Berührungs- oder Überlappungszone bildet die Naht, verlaufend über die vollständige Höhe des Hohlzylinders entlang der Mantelfläche.

Ein resultierender rohrförmiger Körper wird in Fig. 1 exemplarisch illustriert. Ein rechteckiges, planares Material wurde hier um die imaginäre Achse (3) wie zuvor beschrieben um mehr als 360° gebogen, so dass die Kante (1) auf der Mantelfläche aufliegt und die Kante (2) die innere Fläche des Mantels vollständig berührt.
Beispiel für einen entsprechenden rohrförmigen Körper ist ein Trinkhalm, der über seine vollständige Länge in seiner Wandung einen Schnitt als Naht im Sinne der Erfindung besitzt.

Das planare Material kann andererseits in einer besonders bevorzugten Ausführungsform des Aufrollvorgangs mehr als 380°, bevorzugt mehr als 540° um eine imaginäre Achse, die das planare Material nicht schneidet, gebogen bzw. aufgerollt werden, so dass sich eine erste Kante des planaren Materials im inneren des Mantels (innere Kante) und die in der planaren Form des Materials der ersten Kante gegenüberliegende zweite Kante außen auf der Mantelfläche (äußere Kante) befindet. Die Zone der Mantelfläche, in der sich die äußere Kante befindet, bildet die Naht, verlaufend über die vollständige Höhe des Hohlzylinders entlang der Mantelfläche. Die innere Kante berührt, bevorzugt vollständig, die Fläche im Inneren des Mantels.

In Fig. 2 wird beispielsweise ein rohrförmiger Körper abgebildet, der gemäß zweiter Aufrollvariante eines rechteckigen, planaren Materials um die imaginäre Achse (3) um mehr als 1080° ( 3 x 360°, d.h. mehr als drei Umdrehungen) aufgerollt wird. Die äußere Kante (1) liegt auf der Mantelfläche auf. Die innere Kante (2) liegt im inneren des Hohlzylinders.

Das Material des Mantels besteht bevorzugt aus mindestens einem Kunststoff, welcher dem röhrenförmigen Körper Formstabilität gewährt, unter Krafteinwirkung eine elastische Verformung der röhrenförmigen Ausgangsform zulässt und nach der Deformation durch seine Rückstellkraft wieder ein Zurückkehren in die Ausgangsform erlaubt.

Dabei ist es bevorzugt, wenn das Material des Mantels aus einem mehrschichtigen Kunststoff besteht. Die Schichten bestehen jeweils aus einem Kunststoff, wobei die jeweiligen Kunststoffe einen unterschiedlichen Kristallisationsgrad besitzen. Die Kunststoffe sind bevorzugt thermoplastisch und werden im erwärmten, plastischen Zustand zum Verbundwerkstoff verarbeitet. Durch langsames Abkühlen des resultierenden Verbundwerkstoffes rollt sich dieser automatisch unter Erhalt eines erfindungsgemäßen rohrförmigen Körpers ein.
Zwischen den Schichten befindet sich bevorzugt ein Haftvermittler.
Eine erfindungsgemäß bevorzugte Form des Materials, insbesondere für eine aufgerollte Form des erfindungsgemäßen rohrförmigen Körpers (siehe exemplarisch Fig. 2) ist ein schichtenförmiges Verbundmaterial aus einer Schicht Surlyn® und einer Schicht Polyamid mit dazwischen befindlichem Haftvermittler. Surlyn® ist ein Handelsprodukt der Firma DuPont. Surlyn® ist ein Copolymer aus Ethylen/Methacrylsäure, wobei Zink, Natrium, Lithium oder andere Metallsalze zugegen sind und mit dem Copolymer lonencluster, so genannte lonomere, innerhalb der Polymermatrix bilden. Surlyn® ist ein thermoplastisches Harz.

Falls der Mantel aus einem anderen Werkstoff besteht, insbesondere aus Metall, so ist es bevorzugt, dass dieser Werkstoff mit mindestens einem Kunststoff beschichtet ist.

Das Nuanciermittel enthält bevorzugt in einem kosmetischen Träger mindestens eine Komponente, die ausgewählt wird,
(a) aus Oxidationsfarbstoffvorprodukten vom Typ der Entwicklerkomponenten und gegebenenfalls der Kupplerkomponenten und/oder
(b) aus Oxofarbstoffvorprodukten und/oder
(c) aus direktziehenden Farbstoffen und/oder
(d) aus Vorstufen naturanaloger Farbstoffe und/oder
(e) aus Oxidationsmitteln und gegebenenfalls zusätzlichen Bleichverstärkern.

Alle Mittel des erfindungsgemäßen Verfahrens enthalten ihre erfindungsgemäßen Inhaltsstoffe bevorzugt in einem geeigneten kosmetischen Träger. Solche Träger sind beispielsweise Cremes, Emulsionen, Gele oder auch tensidhaltige schäumende Lösungen, wie beispielsweise Shampoos, Schaumaerosole oder andere Zubereitungen, die für die Anwendung auf dem Haar geeignet sind. Es ist aber auch denkbar, die Inhaltsstoffe in eine pulverförmige oder auch tablettenförmige Formulierung zu integrieren. Die kosmetischen Träger können insbesondere wässrig oder wässrigalkoholisch sein.

Ein wäßriger kosmetischer Träger enthält mindestens 50 Gew.-% Wasser.

Unter wäßrig-alkoholischen kosmetischen Trägern sind im Sinne der vorliegenden Erfindung wäßrige Lösungen enthaltend 3 bis 70 Gew.-% eines C₁-C₄-Alkohols, insbesondere Ethanol bzw. Isopropanol, zu verstehen. Die erfindungsgemäßen Mittel können zusätzlich weitere organische Lösemittel, wie beispielsweise Methoxybutanol, Benzylalkohol, Ethyldiglykol oder 1,2-Propylenglykol, enthalten. Bevorzugt sind dabei alle wasserlöslichen organischen Lösemittel.

Das Nuanciermittel kann Oxidationsfarbstoffvorprodukte vom Typ der Entwicklerkomponenten und gegebenenfalls der Kupplerkomponenten enthalten. Als Entwicklerkomponenten werden üblicherweise primäre aromatische Amine mit einer weiteren, in para- oder ortho-Position befindlichen, freien oder substituierten Hydroxy- oder Aminogruppe, Diaminopyridinderivate, heterozyklische Hydrazone, 4-Aminopyrazolderivate sowie 2,4,5,6-Tetraaminopyrimidin und dessen Derivate eingesetzt.

Es kann erfindungsgemäß bevorzugt sein, als Entwicklerkomponente ein p-Phenylendiaminderivat oder eines seiner physiologisch verträglichen Salze einzusetzen. Besonders bevorzugt sind p-Phenylendiaminderivate der Formel (E1) 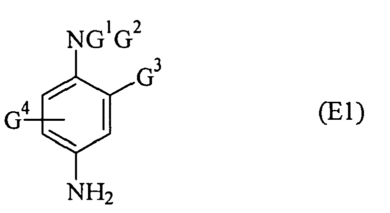 wobei
- G¹ steht für ein Wasserstoffatom, einen C₁- bis C₄-Alkylrest, einen C₁- bis C₄-Monohydroxyalkylrest, einen C₂- bis C₄-Polyhydroxyalkylrest, einen (C₁- bis C₄)-Alkoxy-(C₁- bis C₄)-alkylrest, einen 4'-Aminophenylrest oder einen C₁- bis C₄-Alkylrest, der mit einer stickstoffhaltigen Gruppe, einem Phenyl- oder einem 4'-Aminophenylrest substituiert ist;
- G² steht für ein Wasserstoffatom, einen C₁- bis C₄-Alkylrest, einen C₁- bis C₄-Monohydroxyalkylrest, einen C₂- bis C₄-Polyhydroxyalkylrest, einen (C₁- bis C₄)-Alkoxy-(C₁- bis C₄)-alkylrest oder einen C₁- bis C₄-Alkylrest, der mit einer stickstoffhaltigen Gruppe substituiert ist;
- G³ steht für ein Wasserstoffatom, ein Halogenatom, wie ein Chlor-, Brom-, lod- oder Fluoratom, einen C₁- bis C₄-Alkylrest, einen C₁- bis C₄-Monohydroxyalkylrest, einen C₂- bis C₄-Polyhydroxyalkylrest, einen C₁- bis C₄-Hydroxyalkoxyrest, einen C₁- bis C₄-Acetylaminoalkoxyrest, einen C₁- bis C₄- Mesylaminoalkoxyrest oder einen C₁-bis C₄-Carbamoylaminoalkoxyrest;
- G⁴ steht für ein Wasserstoffatom, ein Halogenatom oder einen C₁- bis C₄-Alkylrest oder
- wenn G³ und G⁴ in ortho-Stellung zueinander stehen, können sie gemeinsam eine verbrückende α,ω-Alkylendioxogruppe, wie beispielsweise eine Ethylendioxygruppe bilden.

Beispiele für die als Substituenten in den erfindungsgemäßen Verbindungen genannten C₁- bis C₄-Alkylreste sind die Gruppen Methyl, Ethyl, Propyl, Isopropyl und Butyl. Ethyl und Methyl sind bevorzugte Alkylreste. Erfindungsgemäß bevorzugte C₁- bis C₄-Alkoxyreste sind beispielsweise eine Methoxy- oder eine Ethoxygruppe. Weiterhin können als bevorzugte Beispiele für eine C₁- bis C₄-Hydroxyalkylgruppe eine Hydroxymethyl-, eine 2-Hydroxyethyl-, eine 3-Hydroxypropyl- oder eine 4-Hydroxybutylgruppe genannt werden. Eine 2-Hydroxyethylgruppe ist besonders bevorzugt. Eine besonders bevorzugte C₂- bis C₄-Polyhydroxyalkylgruppe ist die 1,2-Dihydroxyethylgruppe. Beispiele für Halogenatome sind erfindungsgemäß F-, Cl- oder Br-Atome, Cl-Atome sind ganz besonders bevorzugt. Die weiteren verwendeten Begriffe leiten sich erfindungsgemäß von den hier gegebenen Definitionen ab. Beispiele für stickstoffhaltige Gruppen der Formel (E1) sind insbesondere die Aminogruppen, C₁- bis C₄-Monoalkylaminogruppen, C₁- bis C₄-Dialkylaminogruppen, C₁- bis C₄-Trialkylammoniumgruppen, C₁- bis C₄-Monohydroxyalkylaminogruppen, Imidazolinium und Ammonium.

Besonders bevorzugte p-Phenylendiamine der Formel (E1) sind ausgewählt aus p-Phenylendiamin, p-Toluylendiamin, 2-Chlor-p-phenylendiamin, 2,3-Dimethyl-p-phenylendiamin, 2,6-Dimethyl-p-phenylendiamin, 2,6-Diethyl-p-phenylendiamin, 2,5-Dimethyl-p-phenylendiamin, N,N-Dimethyl-p-phenylendiamin, N,N-Diethyl-p-phenylendiamin, N,N-Dipropyl-p-phenylendiamin, 4-Amino-3-methyl-(N,N-diethyl)-anilin, N,N-Bis-(β-hydroxyethyl)-p-phenylendiamin, 4-N,N-Bis-(β-hydroxyethyl)amino-2-methylanilin, 4-N,N-Bis-(β-hydroxyethyl)amino-2-chloranilin, 2-(β-Hydroxyethyl)-p-phenylendiamin, 2-(α,β-Dihydroxyethyl)-p-phenylendiamin, 2-Fluor-p-phenylendiamin, 2-Isopropyl-p-phenylendiamin, N-(β-Hydroxypropyl)-p-phenylendiamin, 2-Hydroxymethyl-p-phenylendiamin, N,N-Dimethyl-3-methyl-p-phenylendiamin, N,N-(Ethyl,β-hydroxyethyl)-p-phenylendiamin, N-(β,y-Dihydroxypropyl)-p-phenylendiamin, N-(4'-Aminophenyl)-p-phenylendiamin, N-Phenyl-p-phenylendiamin, 2-(β-Hydroxyethyloxy)-p-phenylendiamin, 2-(β-Acetylaminoethyloxy)-p-phenylendiamin, N-(β-Methoxyethyl)-p-phenylendiamin und 5,8-Diaminobenzo-1,4-dioxan sowie ihren physiologisch verträglichen Salzen.

Erfindungsgemäß ganz besonders bevorzugte p-Phenylendiaminderivate der Formel (E1) sind p-Phenylendiamin, p-Toluylendiamin, 2-(β-Hydroxyethyl)-p-phenylendiamin, 2-(α,β-Dihydroxyethyl)-p-phenylendiamin und N,N-Bis-(β-hydroxyethyl)-p-phenylendiamin.

Es kann erfindungsgemäß weiterhin bevorzugt sein, als Entwicklerkomponente Verbindungen einzusetzen, die mindestens zwei aromatische Kerne enthalten, die mit Amino- und/oder Hydroxylgruppen substituiert sind.

Unter den zweikernigen Entwicklerkomponenten, die in den Nuanciermitteln gemäß der Erfindung verwendet werden können, kann man insbesondere die Verbindungen nennen, die der folgenden Formel (E2) entsprechen, sowie ihre physiologisch verträglichen Salze: 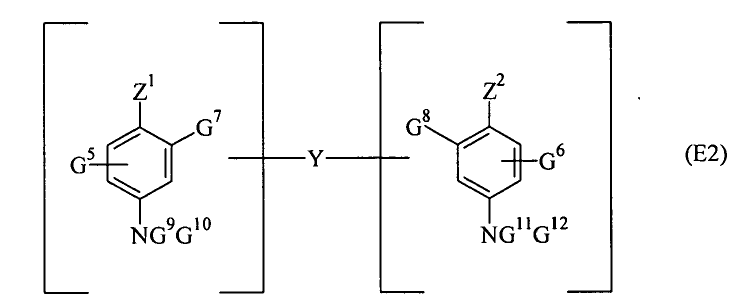 wobei:
- Z¹ und Z² stehen unabhängig voneinander für einen Hydroxyl- oder NH₂-Rest, der gegebenenfalls durch einen C₁- bis C₄-Alkylrest, durch einen C₁- bis C₄-Hydroxyalkylrest und/oder durch eine Verbrückung Y substituiert ist oder der gegebenenfalls Teil eines verbrückenden Ringsystems ist,
- die Verbrückung Y steht für eine Alkylengruppe mit 1 bis 14 Kohlenstoffatomen, wie beispielsweise eine lineare oder verzweigte Alkylenkette oder einen Alkylenring, die von einer oder mehreren stickstoffhaltigen Gruppen und/oder einem oder mehreren Heteroatomen wie Sauerstoff-, Schwefel- oder Stickstoffatomen unterbrochen oder beendet sein kann und eventuell durch einen oder mehrere Hydroxyl- oder C₁- bis C₈-Alkoxyreste substituiert sein kann, oder eine direkte Bindung,
- G⁵ und G⁶ stehen unabhängig voneinander für ein Wasserstoff- oder Halogenatom, einen C₁- bis C₄-Alkylrest, einen C₁- bis C₄-Monohydroxyalkylrest, einen C₂- bis C₄-Polyhydroxyalkylrest, einen C₁- bis C₄-Aminoalkylrest oder eine direkte Verbindung zur Verbrückung Y,
- G⁷, G⁸, G⁹, G¹⁰, G¹¹ und G¹² stehen unabhängig voneinander für ein Wasserstoffatom, eine direkte Bindung zur Verbrückung Y oder einen C₁- bis C₄-Alkylrest,
mit den Maßgaben, dass
- die Verbindungen der Formel (E2) nur eine Verbrückung Y pro Molekül enthalten und
- die Verbindungen der Formel (E2) mindestens eine Aminogruppe enthalten, die mindestens ein Wasserstoffatom trägt.

Die in Formel (E2) verwendeten Substituenten sind erfindungsgemäß analog zu den obigen Ausführungen definiert.

Bevorzugte zweikernige Entwicklerkomponenten der Formel (E2) sind insbesondere: N,N'-Bis-(β-hydroxyethyl)-N,N'-bis-(4'-aminophenyl)-1,3-diamino-propan-2-ol, N,N'-Bis-(β-hydroxyethyl)-N,N'-bis-(4'-aminophenyl)-ethylendiamin, N,N'-Bis-(4-aminophenyl)-tetramethylendiamin, N,N'-Bis-(β-hydroxyethyl)-N,N'-bis-(4-aminophenyl)-tetramethylendiamin, N,N'-Bis-(4-methyl-aminophenyl)-tetramethylendiamin, N,N'-Diethyl-N,N'-bis-(4'-amino-3'-methylphenyl)-ethylendiamin, Bis-(2-hydroxy-5-aminophenyl)-methan, 1,3-Bis-(2,5-diaminophenoxy)-propan-2-ol, N,N'-Bis-(4'-aminophenyl)-1,4-diazacycloheptan, N,N'-Bis-(2-hydroxy-5-aminobenzyl)-piperazin, N-(4'-Aminophenyl)-p-phenylendiamin und 1,10-Bis-(2',5'-diaminophenyl)-1,4,7,10-tetraoxadecan und ihre physiologisch verträglichen Salze.

Ganz besonders bevorzugte zweikernige Entwicklerkomponenten der Formel (E2) sind N,N'-Bis-(β-hydroxyethyl)-N,N'-bis-(4'-aminophenyl)-1,3-diamino-propan-2-ol, Bis-(2-hydroxy-5-aminophenyl)-methan, 1,3-Bis-(2,5-diaminophenoxy)-propan-2-ol, N,N'-Bis-(4'-aminophenyl)-1,4-diazacycloheptan und 1,10-Bis-(2',5'-diaminophenyl)-1,4,7,10-tetraoxadecan oder eines ihrer physiologisch verträglichen Salze.

Weiterhin kann es erfindungsgemäß bevorzugt sein, als Entwicklerkomponente ein p-Aminophenolderivat oder eines seiner physiologisch verträglichen Salze einzusetzen. Besonders bevorzugt sind p-Aminophenolderivate der Formel (E3) 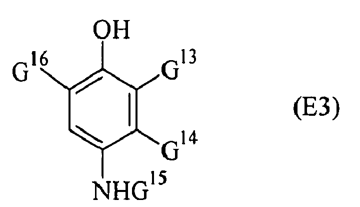 wobei:
- G¹³ steht für ein Wasserstoffatom, ein Halogenatom, einen C₁- bis C₄-Alkylrest, einen C₁- bis C₄-Monohydroxyalkylrest, einen C₂- bis C₄-Polyhydroxyalkylrest, einen (C₁- bis C₄)-Alkoxy-(C₁- bis C₄)-alkylrest, einen C₁- bis C₄-Aminoalkylrest, einen Hydroxy-(C₁- bis C₄)-alkylaminorest, einen C₁- bis C₄-Hydroxyalkoxyrest, einen C₁-bis C₄-Hydroxyalkyl-(C₁-bis C₄)-aminoalkylrest oder einen (Di-C₁- bis C₄-Alkylamino)-(C₁- bis C₄)-alkytrest, und
- G¹⁴ steht für ein Wasserstoff- oder Halogenatom, einen C₁- bis C₄-Alkylrest, einen C₁- bis C₄-Monohydroxyalkylrest, einen C₂- bis C₄-Polyhydroxyalkylrest, einen (C₁-bis C₄)-Alkoxy-(C₁- bis C₄)-alkylrest, einen C₁- bis C₄-Aminoalkylrest oder einen C₁-bis C₄-Cyanoalkylrest,
- G¹⁵ steht für Wasserstoff, einen C₁- bis C₄-Alkylrest, einen C₁- bis C₄-Monohydroxyalkylrest, einen C₂- bis C₄-Polyhydroxyalkylrest, einen Phenylrest oder einen Benzylrest, und
- G¹⁶ steht für Wasserstoff oder ein Halogenatom.

Die in Formel (E3) verwendeten Substituenten sind erfindungsgemäß analog zu den obigen Ausführungen definiert.

Bevorzugte p-Aminophenole der Formel (E3) sind insbesondere p-Aminophenol, N-Methyl-p-aminophenol, 4-Amino-3-methyl-phenol, 4-Amino-3-fluorphenol, 2-Hydroxymethylamino-4-aminophenol, 4-Amino-3-hydroxymethylphenol, 4-Amino-2-(β-hydroxyethoxy)-phenol, 4-Amino-2-methylphenol, 4-Amino-2-hydroxymethylphenol, 4-Amino-2-methoxymethyl-phenol, 4-Amino-2-aminomethylphenol, 4-Amino-2-(β-hydroxyethyl-aminomethyl)-phenol, 4-Amino-2-(α,β-dihydroxyethyl)-phenol, 4-Amino-2-fluorphenol, 4-Amino-2-chlorphenol, 4-Amino-2,6-dichlorphenol, 4-Amino-2-(diethylaminomethyl)-phenol sowie ihre physiologisch verträglichen Salze.

Ganz besonders bevorzugte Verbindungen der Formel (E3) sind p-Aminophenol, 4-Amino-3-methylphenol, 4-Amino-2-aminomethylphenol, 4-Amino-2-(α,β-dihydroxyethyl)-phenol und 4-Amino-2-(diethyl-aminomethyl)-phenol.

Ferner kann die Entwicklerkomponente ausgewählt sein aus o-Aminophenol und seinen Derivaten, wie beispielsweise 2-Amino-4-methylphenol, 2-Amino-5-methylphenol oder 2-Amino-4-chlorphenol.

Weiterhin kann die Entwicklerkomponente ausgewählt sein aus heterozyklischen Entwicklerkomponenten, wie beispielsweise den Pyridin-, Pyrimidin-, Pyrazol-, Pyrazol-Pyrimidin-Derivaten und ihren physiologisch verträglichen Salzen.

Bevorzugte Pyridin-Derivate sind insbesondere die Verbindungen, die in den Patenten GB 1 026 978 und GB 1 153 196 beschrieben werden, wie 2,5-Diamino-pyridin, 2-(4'-Methoxyphenyl)amino-3-amino-pyridin, 2,3-Diamino-6-methoxy-pyridin, 2-(β-Methoxyethyl)amino-3-amino-6-methoxy-pyridin und 3,4-Diamino-pyridin.

Bevorzugte Pyrimidin-Derivate sind insbesondere die Verbindungen, die im deutschen Patent DE 2 359 399, der japanischen Offenlegungsschrift JP 02019576 A2 oder in der Offenlegungsschrift WO 96/15765 beschrieben werden, wie 2,4,5,6-Tetraaminopyrimidin, 4-Hydroxy-2,5,6-triaminopyrimidin, 2-Hydroxy-4,5,6-triaminopyrimidin, 2-Dimethylamino-4,5,6-triaminopyrimidin, 2,4-Dihydroxy-5,6-diaminopyrimidin und 2,5,6-Triaminopyrimidin.

Bevorzugte Pyrazol-Derivate sind insbesondere die Verbindungen, die in den Patenten DE 3 843 892, DE 4 133 957 und Patentanmeldungen WO 94/08969, WO 94/08970, EP-740 931 und DE 195 43 988 beschrieben werden, wie 4,5-Diamino-1-methylpyrazol, 4,5-Diamino-1-(β-hydroxyethyl)-pyrazol, 3,4-Diaminopyrazol, 4,5-Diamino-1-(4'-chlorbenzyl)-pyrazol, 4,5-Diamino-1,3-dimethylpyrazol, 4,5-Diamino-3-methyl-1-phenylpyrazol, 4,5-Diamino-1-methyl-3-phenylpyrazol, 4-Amino-1,3-dimethyl-5-hydrazinopyrazol, 1-Benzyl-4,5-diamino-3-methylpyrazol, 4,5-Diamino-3-tert.-butyl-1-methylpyrazol, 4,5-Diamino-1-tert.-butyl-3-methylpyrazol, 4,5-Diamino-1-(β-hydroxyethyl)-3-methylpyrazol, 4,5-Diamino-1-ethyl-3-methylpyrazol, 4,5-Diamino-1-ethyl-3-(4'-methoxyphenyl)-pyrazol, 4,5-Diamino-1-ethyl-3-hydroxymethylpyrazol, 4,5-Diamino-3-hydroxymethyl-1-methylpyrazol, 4,5-Diamino-3-hydroxymethyl-1-isopropylpyrazol, 4,5-Diamino-3-methyl-1-isopropylpyrazol, 4-Amino-5-(2-aminoethyl)amino-1,3-dimethylpyrazol, 3,4,5-Triaminopyrazol, 1-Methyl-3,4,5-triaminopyrazol, 3,5-Diamino-1-methyl-4-methylaminopyrazol und 3,5-Diamino-4-(β-hydroxyethyl)amino-1-methylpyrazol.

Bevorzugte PyrazoloPyrimidin-Derivate sind insbesondere die Derivate des Pyrazolo[1,5-a]pyrimidin der folgenden Formel (E4) und dessen tautomeren Formen, sofern ein tautomeres Gleichgewicht besteht: 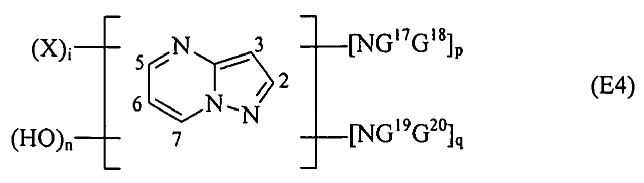 wobei:
- G¹⁷, G¹⁸, G¹⁹ und G²⁰ unabhängig voneinander stehen für ein Wasserstoffatom, einen C₁- bis C₄-Alkylrest, einen Aryl-Rest, einen C₁- bis C₄-Hydroxyalkylrest, einen C₂- bis C₄-Polyhydroxyalkylrest einen (C₁- bis C₄)-Alkoxy-(C₁- bis C₄)-alkylrest, einen C₁- bis C₄-Aminoalkylrest, der gegebenenfalls durch ein Acetyl-Ureid- oder einen Sulfonyl-Rest geschützt sein kann, einen (C₁- bis C₄)-Alkylamino-(C₁- bis C₄)-alkylrest, einen Di-[(C₁- bis C₄)-alkyl]-(C₁- bis C₄)-aminoalkylrest, wobei die Dialkyl-Reste gegebenenfalls einen Kohlenstoffzyklus oder einen Heterozyklus mit 5 oder 6 Kettengliedern bilden, einen C₁- bis C₄-Hydroxyalkyl- oder einen Di-(C₁- bis C₄)-[Hydroxyalkyl]-(C₁- bis C₄)-aminoalkylrest,
- die X-Reste stehen unabhängig voneinander für ein Wasserstoffatom, einen C₁- bis C₄-Alkylrest, einen Aryl-Rest, einen C₁- bis C₄-Hydroxyalkylrest, einen C₂- bis C₄-Polyhydroxyalkylrest, einen C₁- bis C₄-Aminoalkylrest, einen (C₁- bis C₄)-Alkylamino-(C₁- bis C₄)-alkylrest, einen Di-[(C₁- bis C₄)alkyl]- (C₁- bis C₄)-aminoalkylrest, wobei die Dialkyl-Reste gegebenenfalls einen Kohlenstoffzyklus oder einen Heterozyklus mit 5 oder 6 Kettengliedern bilden, einen C₁- bis C₄-Hydroxyalkyl- oder einen Di-(C₁-bis C₄-hydroxyalkyl)aminoalkylrest, einen Aminorest, einen C₁- bis C₄-Alkyl- oder Di-(C₁- bis C₄-hydroxyalkyl)aminorest, ein Halogenatom, eine Carboxylsäuregruppe oder eine Sulfonsäuregruppe,
- i hat den Wert 0, 1, 2 oder 3,
- p hat den Wert 0 oder 1,
- q hat den Wert 0 oder 1 und
- n hat den Wert 0 oder 1,
mit der Maßgabe, dass
- die Summe aus p + q ungleich 0 ist,
- wenn p + q gleich 2 ist, n den Wert 0 hat, und die Gruppen NG¹⁷G¹⁸ und NG¹⁹G²⁰ belegen die Positionen (2,3); (5,6); (6,7); (3,5) oder (3,7);
- wenn p + q gleich 1 ist, n den Wert 1 hat, und die Gruppen NG¹⁷G¹⁸ (oder NG¹⁹G²⁰) und die Gruppe OH belegen die Positionen (2,3); (5,6); (6,7); (3,5) oder (3,7);

Die in Formel (E4) verwendeten Substituenten sind erfindungsgemäß analog zu den obigen Ausführungen definiert.

Wenn das Pyrazolo[1,5-a]pyrimidin der obenstehenden Formel (E4) eine Hydroxygruppe an einer der Positionen 2, 5 oder 7 des Ringsystems enthält, besteht ein tautomeres Gleichgewicht, das zum Beispiel im folgenden Schema dargestellt wird:

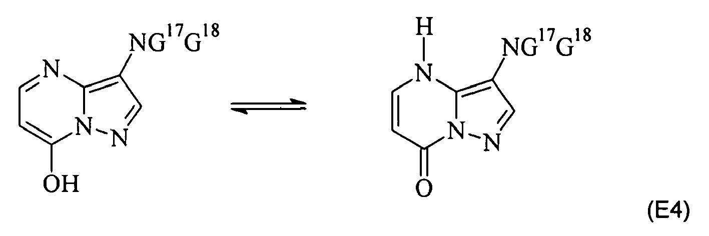

Unter den Pyrazolo[1,5-a]pyrimidinen der obenstehenden Formel (E4) kann man insbesondere nennen:
- Pyrazolo[1,5-a]pyrimidin-3,7-diamin;
- 2,5-Dimethyl-pyrazolo[1,5-a]pyrimidin-3,7-diamin;
- Pyrazolo[1,5-a]pyrimidin-3,5-diamin;
- 2,7-Dimethyl-pyrazolo[1,5-a]pyrimidin-3,5-diamin;
- 3-Aminopyrazolo[1,5-a]pyrimidin-7-ol;
- 3-Aminopyrazolo[1,5-a]pyrimidin-5-ol;
- 2-(3-Aminopyrazolo[1,5-a]pyrimidin-7-ylamino)-ethanol;
- 2-(7-Aminopyrazolo[1,5-a]pyrimidin-3-ylamino)-ethanol;
- 2-[(3-Aminopyrazolo[1,5-a]pyrimidin-7-yl)-(2-hydroxy-ethyl)amino]-ethanol;
- 2-[(7-Aminopyrazolo[1,5-a]pyrimidin-3-yl)-(2-hydroxy-ethyl)amino]-ethanol;
- 5,6-Dimethylpyrazolo[1,5-a]pyrimidin-3,7-diamin;
- 2,6-Dimethylpyrazolo[1,5-a]pyrimidin-3,7-diamin;
- 3-Amino-7-dimethylamino-2,5-dimethylpyrazolo[1,5-a]pyrimidin;
sowie ihre physiologisch verträglichen Salze und ihre tautomeren Formen, wenn ein tautomers Gleichgewicht vorhanden ist.

Die Pyrazolo[1,5-a]pyrimidine der obenstehenden Formel (E4) können wie in der Literatur beschrieben durch Zyklisierung ausgehend von einem Aminopyrazol oder von Hydrazin hergestellt werden.

Als Vorstufen naturanaloger Farbstoffe werden bevorzugt solche Indole und Indoline eingesetzt, die mindestens eine Hydroxy- oder Aminogruppe, bevorzugt als Substituent am Sechsring, aufweisen. Diese Gruppen können weitere Substituenten tragen, z. B. in Form einer Veretherung oder Veresterung der Hydroxygruppe oder eine Alkylierung der Aminogruppe. Diese Indole bzw. Indoline werden gemäß der vorliegenden Erfindung der Einfachheit halber unter Entwicklerkomponenten der Oxidationshaarfarben subsumiert. Besonders gut als Vorstufen naturanaloger Haarfarbstoffe geeignet sind Derivate des 5,6-Dihydroxyindolins der Formel (la), 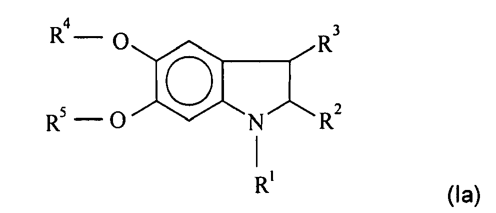 in der unabhängig voneinander
- R¹ steht für Wasserstoff, eine C₁-C₄-Alkylgruppe oder eine C₁-C₄-Hydroxy-alkylgruppe,
- R² steht für Wasserstoff oder eine -COOH-Gruppe, wobei die -COOH-Gruppe auch als Salz mit einem physiologisch verträglichen Kation vorliegen kann,
- R³ steht für Wasserstoff oder eine C₁-C₄-Alkylgruppe,
- R⁴ steht für Wasserstoff, eine C₁-C₄-Alkylgruppe oder eine Gruppe -CO-R⁶, in der R⁶ steht für eine C₁-C₄-Alkylgruppe, und
- R⁵ steht für eine der unter R⁴ genannten Gruppen,
sowie physiologisch verträgliche Salze dieser Verbindungen mit einer organischen oder anorganischen Säure.

Besonders bevorzugte Derivate des Indolins sind das 5,6-Dihydroxyindolin, N-Methyl-5,6-dihydroxyindolin, N-Ethyl-5,6-dihydroxyindolin, N-Propyl-5,6-dihydroxyindolin, N-Butyl-5,6-dihydroxyindolin, 5,6-Dihydroxyindolin-2-carbonsäure sowie das 6-Hydroxyindolin, das 6-Aminoindolin und das 4-Aminoindolin.

Besonders hervorzuheben sind innerhalb dieser Gruppe N-Methyl-5,6-dihydroxyindolin, N-Ethyl-5,6-dihydroxyindolin, N-Propyl-5,6-dihydroxyindolin, N-Butyl-5,6-dihydroxyindolin und insbesondere das 5,6-Dihydroxyindolin.

Als Vorstufen naturanaloger Haarfarbstoffe hervorragend geeignet sind weiterhin Derivate des 5,6-Dihydroxyindols der Formel (Ib), 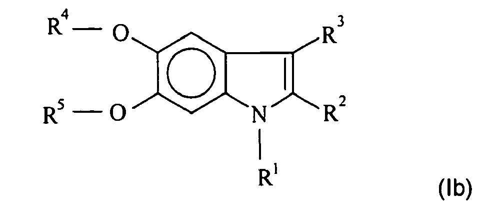 in der unabhängig voneinander
- R¹ steht für Wasserstoff, eine C₁-C₄-Alkylgruppe oder eine C₁-C₄-Hydroxyalkylgruppe,
- R² steht für Wasserstoff oder eine -COOH-Gruppe, wobei die -COOH-Gruppe auch als Salz mit einem physiologisch verträglichen Kation vorliegen kann,
- R³ steht für Wasserstoff oder eine C₁-C₄-Alkylgruppe,
- R⁴ steht für Wasserstoff, eine C₁-C₄-Alkylgruppe oder eine Gruppe -CO-R⁶, in der R⁶ steht für eine C₁-C₄-Alkylgruppe, und
- R⁵ steht für eine der unter R⁴ genannten Gruppen,
- sowie physiologisch verträgliche Salze dieser Verbindungen mit einer organischen oder anorganischen Säure.

Besonders bevorzugte Derivate des Indols sind 5,6-Dihydroxyindol, N-Methyl-5,6-dihydroxyindol, N-Ethyl-5,6-dihydroxyindol, N-Propyl-5,6-dihydroxyindol, N-Butyl-5,6-dihydroxyindol, 5,6-Dihydroxyindol-2-carbonsäure, 6-Hydroxyindol, 6-Aminoindol und 4-Aminoindol.

Innerhalb dieser Gruppe hervorzuheben sind N-Methyl-5,6-dihydroxyindol, N-Ethyl-5,6-dihydroxyindol, N-Propyl-5,6-dihydroxyindol, N-Butyl-5,6-dihydroxyindol sowie insbesondere das 5,6-Dihydroxyindol.

Die Indolin- und Indol-Derivate können in den im Rahmen des erfindungsgemäßen Verfahrens eingesetzten Nuanciermitteln sowohl als freie Basen als auch in Form ihrer physiologisch verträglichen Salze mit anorganischen oder organischen Säuren, z. B. der Hydrochloride, der Sulfate und Hydrobromide, eingesetzt werden. Die Indol- oder Indolin-Derivate sind in diesen üblicherweise in Mengen von 0,05-10 Gew.-%, vorzugsweise 0,2-5 Gew.-% enthalten.

In einer weiteren Ausführungsform kann es erfindungsgemäß bevorzugt sein, das Indolin- oder Indolderivat in dem Nuanciermittel in Kombination mit mindestens einer Aminosäure oder einem Oligopeptid einzusetzen. Die Aminosäure ist vorteilhafterweise eine α-Aminosäure; ganz besonders bevorzugte α-Aminosäuren sind Arginin, Ornithin, Lysin, Serin und Histidin, insbesondere Arginin.

In einer weiteren Ausführungsform enthalten die Nuanciermittel des erfindungsgemäßen Verfahrens mindestens eine Kupplerkomponente.

Als Kupplerkomponenten werden in der Regel m-Phenylendiaminderivate, Naphthole, Resorcin und Resorcinderivate, Pyrazolone und m-Aminophenolderivate sowie heterozyklische Verbindungen verwendet.

Erfindungsgemäß bevorzugte Kupplerkomponenten sind
- m-Aminophenol und dessen Derivate wie beispielsweise 5-Amino-2-methylphenol, N-Cyclopentyl-3-aminophenol, 3-Amino-2-chlor-6-methylphenol, 2-Hydroxy-4-aminophenoxyethanol, 2,6-Dimethyl-3-aminophenol, 3-Trifluoroacetylamino-2-chlor-6-methylphenol, 5-Amino-4-chlor-2-methylphenol, 5-Amino-4-methoxy-2-methylphenol, 5-(2'-Hydroxyethyl)-amino-2-methylphenol, 3-(Diethylamino)-phenol, N-Cyclopentyl-3-aminophenol, 1,3-Dihydroxy-5-(methylamino)-benzol, 3-Ethylamino-4-methylphenol und 2,4-Dichlor-3-aminophenol,
- o-Aminophenol und dessen Derivate,
- m-Diaminobenzol und dessen Derivate wie beispielsweise 2,4-Diaminophenoxyethanol, 1,3-Bis-(2',4'-diaminophenoxy)-propan, 1-Methoxy-2-amino-4-(2'-hydroxyethylamino)benzol, 1,3-Bis-(2',4'-diaminophenyl)-propan, 2,6-Bis-(2'-hydroxyethylamino)-1-methylbenzol, 2-({3-[(2-Hydroxyethyl)amino]-4-methoxy-5-methylphenyl}amino)ethanol, 2-({3-[(2-Hydroxyethyl)amino]-2-methoxy-5-methylphenyl}amino)ethanol, 2-({3-[(2-Hydroxyethyl)amino]-4,5-dimethylphenyl}-amino)ethanol, 2-[3-Morpholin-4-ylphenyl)amino]ethanol, 3-Amino-4-(2-methoxyethoxy)-5-methylphenylamin und 1-Amino-3-bis-(2'-hydroxyethyl)-aminobenzol,
- o-Diaminobenzol und dessen Derivate wie beispielsweise 3,4-Diaminobenzoesäure und 2,3-Diamino-1-methylbenzol,
- Di- beziehungsweise Trihydroxybenzolderivate wie beispielsweise Resorcin, Resorcinmonomethylether, 2-Methylresorcin, 5-Methylresorcin, 2,5-Dimethylresorcin, 2-Chlorresorcin, 4-Chlorresorcin, Pyrogallol und 1,2,4-Trihydroxybenzol,
- Pyridinderivate wie beispielsweise 2,6-Dihydroxypyridin, 2-Amino-3-hydroxypyridin, 2-Amino-5-chlor-3-hydroxypyridin, 3-Amino-2-methylamino-6-methoxypyridin, 2,6-Dihydroxy-3,4-dimethylpyridin, 2,6-Dihydroxy-4-methylpyridin, 2,6-Diaminopyridin, 2,3-Diamino-6-methoxypyridin und 3,5-Diamino-2,6-dimethoxypyridin,
- Naphthalinderivate wie beispielsweise 1-Naphthol, 2-Methyl-1-naphthol, 2-Hydroxymethyl-1-naphthol, 2-Hydroxyethyl-1-naphthol, 1,5-Dihydroxynaphthalin, 1,6-Dihydroxynaphthalin, 1,7-Dihydroxynaphthalin, 1,8-Dihydroxynaphthalin, 2,7-Dihydroxynaphthalin und 2,3-Dihydroxynaphthalin,
- Morpholinderivate wie beispielsweise 6-Hydroxybenzomorpholin und 6-Amino-benzomorpholin,
- Chinoxalinderivate wie beispielsweise 6-Methyl-1,2,3,4-tetrahydrochinoxalin,
- Pyrazolderivate wie beispielsweise 1-Phenyl-3-methylpyrazol-5-on,
- Indolderivate wie beispielsweise 4-Hydroxyindol, 6-Hydroxyindol und 7-Hydroxyindol,
- Pyrimidinderivate, wie beispielsweise 4,6-Diaminopyrimidin, 4-Amino-2,6-dihydroxypyrimidin, 2,4-Diamino-6-hydroxypyrimidin, 2,4,6-Trihydroxypyrimidin, 2-Amino-4-methylpyrimidin, 2-Amino-4-hydroxy-6-methylpyrimidin und 4,6-Dihydroxy-2-methylpyrimidin, oder
- Methylendioxybenzolderivate wie beispielsweise 1-Hydroxy-3,4-methylendioxybenzol, 1-Amino-3,4-methylendioxybenzol und 1-(2'-Hydroxyethyl)-amino-3,4-methylendioxybenzol
sowie deren physiologisch verträglichen Salze.

Erfindungsgemäß besonders bevorzugte Kupplerkomponenten sind 1-Naphthol, 1,5-, 2,7- und 1,7-Dihydroxynaphthalin, 3-Aminophenol, 5-Amino-2-methylphenol, 2-Amino-3-hydroxypyridin, Resorcin, 4-Chlorresorcin, 2-Chlor-6-methyl-3-aminophenol, 2-Methyl-resorcin, 5-Methylresorcin, 2,5-Dimethylresorcin und 2,6-Dihydroxy-3,4-dimethylpyridin und die physiologisch verträglichen Salzen der vorgenannten Verbindungen.

Die Nuanciermittel des erfindungsgemäßen Verfahrens enthalten sowohl die Entwicklerkomponenten als auch die Kupplerkomponenten bevorzugt in einer Menge von 0,005 bis 10 Gew.-%, vorzugsweise von 0,1 bis 5 Gew.-%, jeweils bezogen auf das gesamte Mittel.

Dabei werden Entwicklerkomponenten und Kupplerkomponenten im allgemeinen in etwa molaren Mengen zueinander eingesetzt. Wenn sich auch der molare Einsatz als zweckmäßig erwiesen hat, so ist ein gewisser Überschuß einzelner Oxidationsfarbstoffvorprodukte nicht nachteilig, so daß Entwicklerkomponenten und Kupplerkomponenten in einem Mol-Verhältnis von 1:0,5 bis 1:3, insbesondere 1:1 bis 1:2, enthalten sein können.

Die Nuanciermittel des erfindungsgemäßen Verfahrens können einen oder mehrere direktziehende Farbstoffe enthalten. Direktziehende Farbstoffe sind üblicherweise Nitrophenylendiamine, Nitroaminophenole, Azofarbstoffe, Anthrachinone oder Indophenole. Bevorzugte direktziehende Farbstoffe sind die unter den internationalen Bezeichnungen bzw. Handelsnamen HC Yellow 2, HC Yellow 4, HC Yellow 5, HC Yellow 6, HC Yellow 12, Acid Yellow 1, Acid Yellow 10, Acid Yellow 23, Acid Yellow 36, HC Orange 1, Disperse Orange 3, Acid Orange 7, HC Red 1, HC Red 3, HC Red 10, HC Red 11, HC Red 13, Acid Red 33, Acid Red 52, HC Red BN, Pigment Red 57:1, HC Blue 2, HC Blue 12, Disperse Blue 3, Acid Blue 7, Acid Green 50, HC Violet 1, Disperse Violet 1, Disperse Violet 4, Acid Violet 43, Disperse Black 9, Acid Black 1, und Acid Black 52 bekannten Verbindungen sowie 1,4-Diamino-2-nitrobenzol, 2-Amino-4-nitrophenol, 1,4-Bis-(β-hydroxyethyl)amino-2-nitrobenzol, 3-Nitro-4-(β-hydroxyethyl)aminophenol, 2-(2'-Hydroxyethyl)amino-4,6-dinitrophenol, 1-(2'-Hydroxyethyl)amino-4-methyl-2-nitrobenzol, 1-Amino-4-(2'-hydroxyethyl)amino-5-chlor-2-nitrobenzol, 4-Amino-3-nitrophenol, 1-(2'-Ureidoethyl)amino-4-nitrobenzol, 4-Amino-2-nitrodiphenylamin-2'-carbonsäure, 6-Nitro-1,2,3,4-tetrahydrochinoxalin, 2-Hydroxy-1,4-naphthochinon, Pikraminsäure und deren Salze, 2-Amino-6-chloro-4-nitrophenol, 4-Ethylamino-3-nitrobenzoesäure und 2-Chloro-6-ethylamino-1-hydroxy-4-nitrobenzol.

Ferner können die Nuanciermittel einen kationischen direktziehenden Farbstoff enthalten. Besonders bevorzugt sind dabei
(a) kationische Triphenylmethanfarbstoffe, wie beispielsweise Basic Blue 7, Basic Blue 26, Basic Violet 2 und Basic Violet 14,
(b) aromatischen Systeme, die mit einer quaternären Stickstoffgruppe substituiert sind, wie beispielsweise Basic Yellow 57, Basic Red 76, Basic Blue 99, Basic Brown 16 und Basic Brown 17, sowie
(c) direktziehende Farbstoffe, die einen Heterozyklus enthalten, der mindestens ein quaternäres Stickstoffatom aufweist, wie sie beispielsweise in der EP-A2-998 908, auf die an dieser Stelle explizit Bezug genommen wird, in den Ansprüchen 6 bis 11 genannt werden.

Bevorzugte kationische direktziehende Farbstoffe der Gruppe (c) sind insbesondere die folgenden Verbindungen:

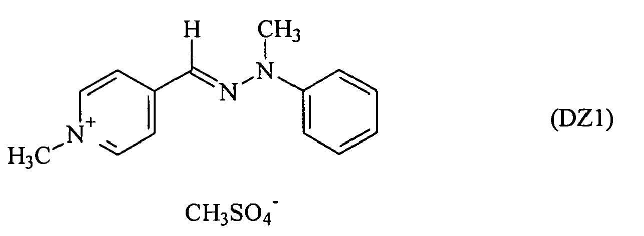

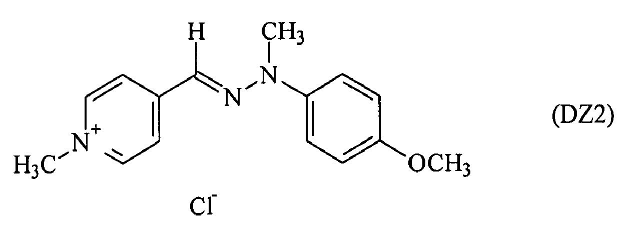

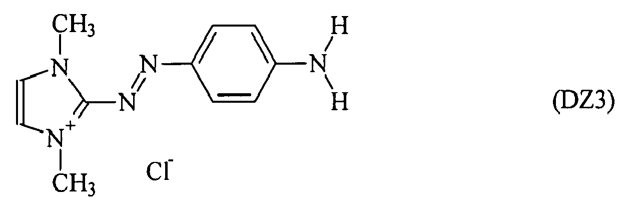

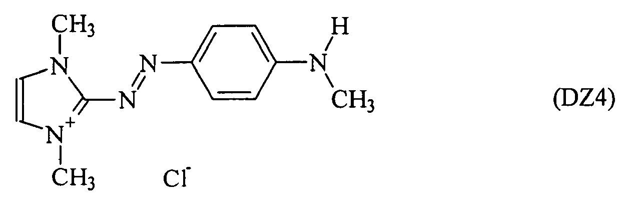

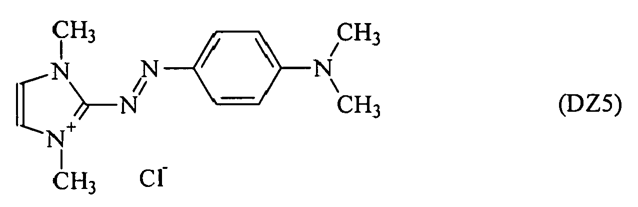

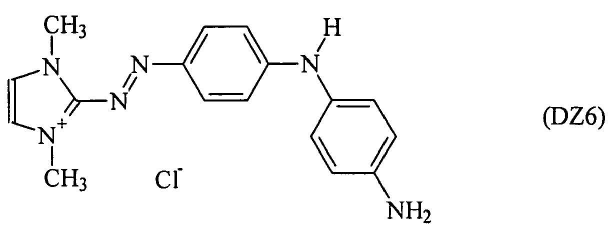

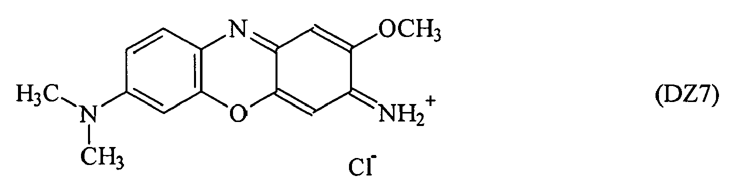

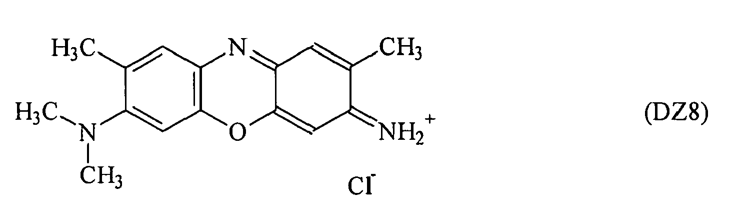

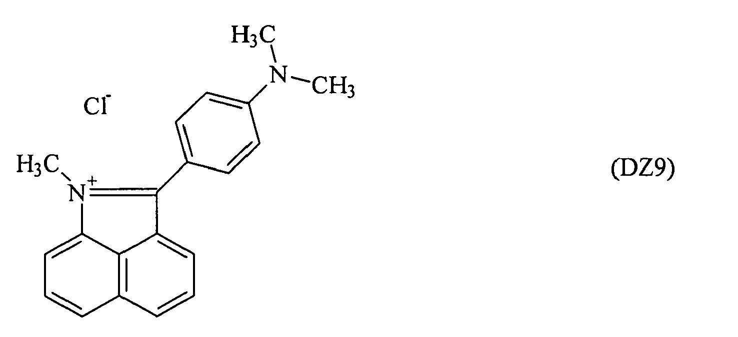

Die Verbindungen der Formeln (DZ1), (DZ3) und (DZ5), die auch unter den Bezeichnungen Basic Yellow 87, Basic Orange 31 und Basic Red 51 bekannt sind, sind ganz besonders bevorzugte kationische direktziehende Farbstoffe der Gruppe (c).

Die kationischen direktziehenden Farbstoffe, die unter dem Warenzeichen Arianor^{®} vertrieben werden, sind erfindungsgemäß ebenfalls ganz besonders bevorzugte kationische direktziehende Farbstoffe.

Die Nuanciermittel können die direktziehenden Farbstoffe in einer Menge von 0,1 bis 5 Gew.-%, bezogen auf das gesamte Mittel, enthalten.

Weiterhin können die Nuanciermittel des erfindungsgemäßen Verfahrens auch in der Natur vorkommende Farbstoffe wie sie beispielsweise in Henna rot, Henna neutral, Henna schwarz, Kamillenblüte, Sandelholz, schwarzem Tee, Faulbaumrinde, Salbei, Blauholz, Krappwurzel, Catechu, Sedre und Alkannawurzel enthalten sind, enthalten.

Es ist jedoch erfindungsgemäß bevorzugt, daß die in dem erfindungsgemäßen Verfahren verwendeten Nuanciermittel keine direktziehenden Farbstoffe enthalten.

Es ist nicht erforderlich, dass die Oxidationsfarbstoffvorprodukte oder die direktziehenden Farbstoffe jeweils einheitliche Verbindungen darstellen. Vielmehr können erfindungsgemäß in den Nuanciermitteln, bedingt durch die Herstellungsverfahren für die einzelnen Farbstoffe, in untergeordneten Mengen noch weitere Komponenten enthalten sein, soweit diese nicht das Färbeergebnis nachteilig beeinflussen oder aus anderen Gründen, z.B. toxikologischen, ausgeschlossen werden müssen.

Bezüglich der in dem Nuanciermittel des erfindungsgemäßen Verfahrens einsetzbaren Farbstoffe wird weiterhin ausdrücklich auf die Monographie Ch. Zviak, The Science of Hair Care, Kapitel 7 (Seiten 248-250; direktziehende Farbstoffe) sowie Kapitel 8, Seiten 264-267; Oxidationsfarbstoffvorprodukte), erschienen als Band 7 der Reihe "Dermatology" (Hrg.: Ch., Culnan und H. Maibach), Verlag Marcel Dekker Inc., New York, Basel, 1986, sowie das "Europäische Inventar der Kosmetik-Rohstoffe", herausgegeben von der Europäischen Gemeinschaft, erhältlich in Diskettenform vom Bundesverband Deutscher Industrie- und Handelsunternehmen für Arzneimittel, Reformwaren und Körperpflegemittel e.V., Mannheim, Bezug genommen.

Das Nuanciermittel des erfindungsgemäßen Verfahrens kann als farbgebende Komponente in Form der Oxofarbstoffvorprodukte mindestens eine Kombination, aus mindestens einer Verbindung der Komponente
- A: Verbindungen, die eine reaktive Carbonylgruppe enthalten mit mindestens einer Verbindung der Komponente
- B: Verbindungen, ausgewählt aus (a) CH-aciden Verbindungen, (b) Verbindungen mit primärer oder sekundärer Aminogruppe oder Hydroxygruppe, ausgewählt aus primären oder sekundären aromatischen Aminen, stickstoffhaltigen heterozyklischen Verbindungen und aromatischen Hydroxyverbindungen, (c) Aminosäuren und/oder (d) aus 2 bis 9 Aminosäuren aufgebauten Oligopeptiden,
enthalten.

Erfindungsgemäße Verbindungen mit einer reaktiven Carbonylgruppe (im Folgenden auch reaktive Carbonylverbindungen oder Komponente A genannt) besitzen mindestens eine Carbonylgruppe als reaktive Gruppe, welche mit den Verbindungen der Komponente B unter Ausbildung einer beide Komponenten verknüpfenden chemischen Bindung reagiert. Ferner sind erfindungsgemäß auch solche Verbindungen als Komponente A umfaßt, in denen die reaktive Carbonylgruppe derart derivatisiert bzw. maskiert ist, daß die Reaktivität des Kohlenstoffatoms der derivatisierten bzw. maskierten Carbonylgruppe gegenüber der Komponente B stets vorhanden ist. Diese Derivate sind bevorzugt Kondensationsverbindungen von reaktiven Carbonylverbindungen mit
a) Aminen und deren Derivate unter Bildung von Iminen oder Oximen als Kondensationsverbindung
b) Alkoholen unter Bildung von Acetalen oder Ketalen als Kondensationsverbindung
c) Wasser unter Bildung von Hydraten als Kondensationsverbindung von Aldehyden.

Die Komponente A wird bevorzugt ausgewählt aus der Gruppe, die gebildet wird aus Acetophenon, Propiophenon, 2-Hydroxyacetophenon, 3-Hydroxyacetophenon, 4-Hydroxyacetophenon, 2-Hydroxypropiophenon, 3-Hydroxypropiophenon, 4-Hydroxypropiophenon, 2-Hydroxybutyrophenon, 3-Hydroxybutyrophenon, 4-Hydroxybutyrophenon, 2,4-Dihydroxyacetophenon, 2,5-Dihydroxyacetophenon, 2,6-Dihydroxyacetophenon, 2,3,4-Trihydroxyacetophenon, 3,4,5-Trihydroxyacetophenon, 2,4,6-Trihydroxyacetophenon, 2,4,6-Trimethoxyacetophenon, 3,4,5-Trimethoxyacetophenon, 3,4,5-Trimethoxyacetophenon-diethylketal, 4-Hydroxy-3-methoxy-acetophenon, 3,5-Dimethoxy-4-hydroxyacetophenon, 4-Aminoacetophenon, 4-Dimethylaminoacetophenon, 4-Morpholinoacetophenon, 4-Piperidinoacetophenon, 4-Imidazolinoacetophenon, 2-Hydroxy-5-brom-acetophenon, 4-Hydroxy-3-nitroacetophenon, Acetophenon-2-carbonsäure, Acetophenon-4-carbonsäure, Benzophenon, 4-Hydroxybenzophenon, 2-Aminobenzophenon, 4,4'-Dihydroxybenzophenon, 2,4-Dihydroxy-benzophenon, 2,4,4'-Trihydroxybenzophenon, 2,3,4-Trihydroxybenzophenon, 2-Hydroxy-1-acetonaphthon, 1-Hydroxy-2-acetonaphthon, Chromon, Chromon-2-carbonsäure, Flavon, 3-Hydroxyflavon, 3,5,7-Trihydroxyflavon, 4',5,7-Trihydroxyflavon, 5,6,7-Trihydroxyflavon, Quercetin, 1-Indanon, 9-Fluorenon, 3-Hydroxyfluorenon, Anthron, 1,8-Dihydroxyanthron, Vanillin, Coniferylaldehyd, 2-Methoxybenzaldehyd, 3-Methoxybenzaldehyd, 4-Methoxybenzaldehyd, 2-Ethoxybenzaldehyd, 3-Ethoxybenzaldehyd, 4-Ethoxybenzaldehyd, 4-Hydroxy-2,3-dimethoxy-benzaldehyd, 4-Hydroxy-2,5-dimethoxybenzaldehyd, 4-Hydroxy-2,6-dimethoxy-benzaldehyd, 4-Hydroxy-2-methyl-benzaldehyd, 4-Hydroxy-3-methyl-benzaldehyd, 4-Hydroxy-2,3-dimethyl-benzaldehyd, 4-Hydroxy-2,5-dimethyl-benzaldehyd, 4-Hydroxy-2,6-dimethyl-benzaldehyd, 4-Hydroxy-3,5-dimethoxybenzaldehyd, 4-Hydroxy-3,5-dimethyl-benzaldehyd, 3,5-Diethoxy-4-hydroxybenzaldehyd, 2,6-Diethoxy-4-hydroxy-benzaldehyd, 3-Hydroxy-4-methoxy-benzaldehyd, 2-Hydroxy-4-methoxy-benzaldehyd, 2-Ethoxy-4-hydroxy-benzaldehyd, 3-Ethoxy-4-hydroxy-benzaldehyd, 4-Ethoxy-2-hydroxy-benzaldehyd, 4-Ethoxy-3-hydroxybenzaldehyd, 2,3-Dimethoxybenzaldehyd, 2,4-Dimethoxybenzaldehyd, 2,5-Dimethoxybenzaldehyd, 2,6-Dimethoxybenzaldehyd, 3,4-Dimethoxybenzaldehyd, 3,5-Dimethoxybenzaldehyd, 2,3,4-Trimethoxybenzaldehyd, 2,3,5-Trimethoxybenzaldehyd, 2,3,6-Trimethoxybenzaldehyd, 2,4,6-Trimethoxybenzaldehyd, 2,4,5-Trimethoxybenzaldehyd, 2,5,6-Trimethoxybenzaldehyd, 2-Hydroxybenzaldehyd, 3-Hydroxybenzaldehyd, 4-Hydroxybenzaldehyd, 2,3-Dihydroxybenzaldehyd, 2,4-Dihydroxybenzaldehyd, 2,5-Dihydroxybenzaldehyd, 2,6-Dihydroxybenzaldehyd, 3,4-Dihydroxybenzaldehyd, 3,5-Dihydroxybenzaldehyd, 2,3,4-Trihydroxybenzaldehyd, 2,3,5-Trihydroxybenzaldehyd, 2,3,6-Trihydroxybenzaldehyd, 2,4,6-Trihydroxybenzaldehyd, 2,4,5-Trihydroxybenzaldehyd, 2,5,6-Trihydroxybenzaldehyd, 4-Hydroxy-2-methoxybenzaldehyd, 4-Dimethylaminobenzaldehyd, 4-Diethylaminobenzaldehyd, 4-Dimethylamino-2-hydroxybenzaldehyd, 4-Diethylamino-2-hydroxybenzaldehyd, 4-Pyrrolidinobenzaldehyd, 4-Morpholinobenzaldehyd, 2-Morpholinobenzaldehyd, 4-Piperidinobenzaldehyd, 2-Methoxy-1-naphthaldehyd, 4-Methoxy-1-naphthaldehyd, 2-Hydroxy-1-naphthaldehyd, 2,4-Dihydroxy-1-napthaldehyd, 4-Hydroxy-3-methoxy-1-naphthaldehyd, 2-Hydroxy-4-methoxy-1-naphthaldehyd, 3-Hydroxy-4-methoxy-1-naphthaldehyd, 2,4-Dimethoxy-1-naphthaldehyd, 3,4-Dimethoxy-1-naphthaldehyd, 4-Hydroxy-1-naphthaldehyd, 4-Dimethylamino-1-naphthaldehyd, 2-Methoxy-zimtaldehyd, 4-Methoxy-zimtaldehyd, 4-Hydroxy-3-methoxy-zimtaldehyd, 3,5-Dimethoxy-4-hydroxyzimtaldehyd, 4-Dimethylaminozimtaldehyd, 2-Dimethylaminobenzaldehyd, 2-Chlor-4-dimethylaminobenzaldehyd, 4-Dimethylamino-2-methylbenzaldehyd, 4-Diethylaminozimtaldehyd, 4-Dibutylamino-benzaldehyd, 4-Diphenylamino-benzaldehyd, 4-Dimethylamino-2-methoxybenzaldehyd, 4-(1-Imidazolyl)-benzaldehyd, Piperonal, 2,3,6,7-Tetrahydro-1H,5H-benzo[ij]chinolizin-9-carboxaldehyd, 2,3,6,7-Tetrahydro-8-hydroxy-1 H,5H-benzo[ij]chinolizin-9-carboxaldehyd, N-Ethylcarbazol-3-aldehyd, 2-Formylmethylen-1,3,3-trimethylindolin (Fischers Aldehyd oder Tribasen Aldehyd), 2-Indolaldehyd, 3-Indolaldehyd, 1-Methylindol-3-aldehyd, 2-Methylindol-3-aldehyd, 1-Acetylindol-3-aldehyd, 3-Acetylindol, 1-Methyl-3-acetylindol, 2-(1',3',3'-Trimethyl-2-indolinyliden)-acetaldehyd, 1-Methylpyrrol-2-aldehyd, 1-Methyl-2-acetylpyrrol, 4-Pyridinaldehyd, 2-Pyridinaldehyd, 3-Pyridinaldehyd, 4-Acetylpyridin, 2-Acetylpyridin, 3-Acetylpyridin, Pyridoxal, Chinolin-3-aldehyd, Chinolin-4-aldehyd, Antipyrin-4-aldehyd, Furfural, 5-Nitrofurfural, 2-Thenoyl-trifluor-aceton, Chromon-3-aldehyd, 3-(5'-Nitro-2'-furyl)-acrolein, 3-(2'-Furyl)-acrolein und Imidazol-2-aldehyd, 1,3-Diacetylbenzol, 1,4-Diacetylbenzol, 1,3,5-Triacetylbenzol, 2-Benzoyl-acetophenon, 2-(4'-Methoxybenzoyl)-acetophenon, 2-(2'-Furoyl)-acetophenon, 2-(2'-Pyridoyl)-acetophenon und 2-(3'-Pyridoyl)-acetophenon, Benzylidenaceton, 4-Hydroxybenzylidenaceton, 2-Hydroxybenzylidenaceton, 4-Methoxybenzylidenaceton, 4-Hydroxy-3-methoxybenzylidenaceton, 4-Dimethylaminobenzylidenaceton, 3,4-Methylendioxybenzylidenaceton, 4-Pyrrolidinobenzylidenaceton, 4-Piperidinobenzylidenaceton, 4-Morpholinobenzylidenaceton, 4-Diethylaminobenzylidenaceton, 3-Benzyliden-2,4-pentandion, 3-(4'-Hydroxybenzyliden)-2,4-pentandion, 3-(4'-Dimethylaminobenzyliden)-2,4-pentandion, 2-Benzylidencyclohexanon, 2-(4'-Hydroxybenzyliden)-cyclohexanon, 2-(4'-Dimethylaminobenzyliden)-cyclohexanon, 2-Benzyliden-1,3-cyclohexandion, 2-(4'-Hydroxybenzyliden)-1,3-cyclohexandion, 3-(4'-Dimethylaminobenzyliden)-1,3-cyclohexandion, 2-Benzyliden-5,5-dimethyl-1,3-cyclohexandion, 2-(4'-Hydroxybenzyliden)-5,5-dimethyl-1,3-cyclohexandion, 2-(4'-Hydroxy-3-methoxybenzyliden)-5,5-dimethyl-1,3-cyclohexandion, 2-(4'-Dimethylaminobenzyliden)-5,5-dimethyl-1,3-cyclohexandion, 2-Benzylidencyclopentanon, 2'-(4-Hydroxybenzyliden)-cyclopentanon, 2-(4'-Dimethylaminobenzyliden)-cyclopentanon, 5-(4-Dimethylaminophenyl)penta-2,4-dienal, 5-(4-Diethylaminophenyl)penta-2,4-dienal, 5-(4-Methoxyphenyl)penta-2,4-dienal, 5-(3,4-Dimethoxyphenyl)penta-2,4-dienal, 5-(2,4-Dimethoxyphenyl)penta-2,4-dienal, 5-(4-Piperidinophenyl)penta-2,4-dienal, 5-(4-Morpholinophenyl)penta-2,4-dienal, 5-(4-Pyrrolidinophenyl)penta-2,4-dienal, 6-(4-Dimethylaminophenyl)hexa-3,5-dien-2-on, 6-(4-Diethylaminophenyl)hexa-3,5-dien-2-on, 6-(4-Methoxyphenyl)hexa-3,5-dien-2-on, 6-(3,4-Dimethoxyphenyl)hexa-3,5-dien-2-on, 6-(2,4-Dimethoxyphenyl)hexa-3,5-dien-2-on, 6-(4-Piperidinophenyl)hexa-3,5-dien-2-on, 6-(4-Morpholinophenyl)hexa-3,5-dien-2-on, 6-(4-Pyrrolidinophenyl)hexa-3,5-dien-2-on, 5-(4-Dimethylamino-1-naphthyl)penta-3,5-dienal, 2-Nitrobenzaldehyd, 3-Nitrobenzaldehyd, 4-Nitrobenzaldehyd, 4-Methyl-3-nitrobenzaldehyd, 3-Hydroxy-4-nitrobenzaldehyd, 4-Hydroxy-3-nitrobenzaldehyd, 5-Hydroxy-2-nitrobenzaldehyd, 2-Hydroxy-5-nitrobenzaldehyd, 2-Hydroxy-3-nitrobenzaldehyd, 2-Fluor-3-nitrobenzaldehyd, 3-Methoxy-2-nitrobenzaldehyd, 4-Chlor-3-nitrobenzaldehyd, 2-Chlor-6-nitrobenzaldehyd, 5-Chlor-2-nitrobenzaldehyd, 4-Chlor-2-nitrobenzaldehyd, 2,4-Dinitrobenzaldehyd, 2,6-Dinitrobenzaldehyd, 2-Hydroxy-3-methoxy-5-nitrobenzaldehyd, 4,5-Dimethoxy-2-nitrobenzaldehyd, 6-Nitropiperonal, 2-Nitropiperonal, 5-Nitrovanillin, 2,5-Dinitrosalicylaldehyd, 5-Brom-3-nitrosalicylaldehyd, 3-Nitro-4-formylbenzolsulfonsäure, 4-Nitro-1-naphthaldehyd, 2-Nitrozimtaldehyd, 3-Nitrozimtaldehyd, 4-Nitrozimtaldehyd, 9-Methyl-3-carbazolaldehyd, 9-Ethyl-3-carbazolaldehyd, 3-Acetylcarbazol, 3,6-Diacetyl-9-ethylcarbazol, 3-Acetyl-9-methylcarbazol, 1,4-Dimethyl-3-carbazolaldehyd, 1,4,9-Trimethyl-3-carbazolaldehyd, 4-Formyl-1-methylpyridinium-, 2-Formyl-1-methylpyridinium-, 4-Formyl-1-ethylpyridinium, 2-Formyl-1-ethylpyridinium-, 4-Formyl-1-benzylpyridinium-, 2-Formyl-1-benzylpyridinium-, 4-Formyl-1,2-dimethylpyridinium-, 4-Formyl-1,3-dimethylpyridinium-, 4-Formyl-1-methylchinolinium-, 2-Formyl-1-methylchinolinium-, 4-Acetyl-1-methylpyridinium-, 2-Acetyl-1-methylpyridinium-, 4-Acetyl-1-methylchinolinium-, 5-Formyl-1-methylchinolinium-, 6-Formyl-1-methylchinolinium-, 7-Formyl-1-methylchinolinium-, 8-Formyl-1-methylchinolinium, 5-Formyl-1-ethylchinolinium-, 6-Formyl-1-ethylchinolinium-, 7-Formyl-1-ethylchinolinium-, 8-Formyl-1-ethylchinolinium, 5-Formyl-1-benzylchinolinium-, 6-Formyl-1-benzylchinolinium-, 7-Formyl-1-benzylchinolinium-, 8-Formyl-1-benzylchinolinium, 5-Formyl-1-allylchinolinium-, 6-Formyl-1-allylchinolinium-, 7-Formyl-1-allylchinolinium- und 8-Formyl-1-allylchinolinium-, 5-Acetyl-1-methylchinolinium-, 6-Acetyl-1-methylchinolinium-, 7-Acetyl-1-methylchinolinium-, 8-Acetyl-1-methylchinolinium, 5-Acetyl-1-ethylchinolinium-, 6-Acetyl-1-ethylchinolinium-, 7-Acetyl-1-ethylchinolinium-, 8-Acetyl-1-ethylchinolinium, 5-Acetyl-1-benzylchinolinium-, 6-Acetyl-1-benzylchinolinium-, 7-Acetyl-1-benzylchinolinium-, 8-Acetyl-1-benzylchinolinium, 5-Acetyl-1-allylchinolinium-, 6-Acetyl-1-allylchinolinium-, 7-Acetyl-1-allylchinolinium- und 8-Acetyl-1-allylchinolinium, 9-Formyl-10-methylacridinium-, 4-(2'-Formylvinyl)-1-methylpyridinium-, 1,3-Dimethyl-2-(4'-formylphenyl)-benzimidazolium-, 1,3-Dimethyl-2-(4'-formylphenyl)-imidazolium-, 2-(4'-Formylphenyl)-3-methylbenzothiazolium-, 2-(4'-Acetylphenyl)-3-methylbenzothiazolium-, 2-(4'-Formylphenyl)-3-methylbenzoxazolium-, 2-(5'-Formyl-2'-furyl)-3-methylbenzothiazolium-, 2-(5'-Formyl-2'-furyl)-3-methylbenzothiazolium-, 2-(5'-Formyl-2'-thienyl)-3-methylbenzothiazolium-, 2-(3'-Formylphenyl)-3-methylbenzothiazolium-, 2-(4'-Formyl-1-naphthyl)-3-methylbenzothiazolium-, 5-Chlor-2-(4'-formylphenyl)-3-methylbenzothiazolium-, 2-(4'-Formylphenyl)-3,5-dimethylbenzothiazolium-Salze, bevorzugt mit Benzolsulfonat, p-Toluolsulfonat, Methansulfonat, Perchlorat, Sulfat, Chlorid, Bromid, lodid, Tetrachlorozinkat, Methylsulfat, Trifluormethansulfonat oder Tetrafluoroborat als Gegenion, Isatin, 1-Methyl-isatin, 1-Allyl-isatin, 1-Hydroxymethyl-isatin, 5-Chlor-isatin, 5-Methoxyisatin, 5-Nitroisatin, 6-Nitro-isatin, 5-Sulfo-isatin, 5-Carboxy-isatin, Chinisatin, 1-Methylchinisatin, sowie beliebigen Gemischen der voranstehenden Verbindungen.

Als CH-acide werden im allgemeinen solche Verbindungen angesehen, die ein an ein aliphatisches Kohlenstoffatom gebundenes Wasserstoffatom tragen, wobei aufgrund von elektronenziehenden Substituenten eine Aktivierung der entsprechenden Kohlenstoff-Wasserstoff-Bindung bewirkt wird. Unter CH-acide Verbindungen fallen erfindungsgemäß auch Enamine, die durch alkalische Behandlung von quaternierten N-Heterozyklen mit einer in Konjugation zum quartären Stickstoff stehenden CH-aciden Alkylgruppe entstehen.

Die CH-aciden Verbindungen der Komponente B sind bevorzugt ausgewählt aus der Gruppe bestehend aus 1,2,3,3-Tetramethyl-3H-indoliumiodid, 1,2,3,3-Tetramethyl-3H-indolium-p-toluolsulfonat, 1,2,3,3-Tetramethyl-3H-indolium-methansulfonat, 1,3,3-Trimethyl-2-methylenindolin (Fischersche Base), 2,3-Dimethyl-benzothiazoliumiodid, 2,3-Dimethyl-benzothiazolium-p-toluolsulfonat, 2,3-Dimethyl-naphtho[1,2-d]thiazolium-p-toluolsulfonat, 3-Ethyl-2-methyl-naphtho[1,2-d]thiazolium-p-toluolsulfonat, Rhodanin, Rhodanin-3-essigsäure, 1,4-Dimethylchinolinium-iodid, 1,2-Dimethylchinolinium-iodid, Barbitursäure, Thiobarbitursäure, 1,3-Dimethylthiobarbitursäure, 1,3-Diethylthiobarbitursäure, 1,3-Diethylbarbitursäure, Oxindol, 3-Indoxylacetat, 2-Cumaranon, 5-Hydroxy-2-cumaranon, 6-Hydroxy-2-cumaranon, 3-Methyl-1-phenyl-pyrazolin-5-on, Indan-1,2-dion, Indan-1,3-dion, Indan-1-on, Benzoylacetonitril, 3-Dicyanmethylenindan-1-on, 2-Amino-4-imino-1,3-thiazolin-hydrochlorid, 5,5-Dimethylcyclohexan-1,3-dion, 2H-1,4-Benzoxazin-4H-3-on, 3-Ethyl-2-methyl-benzoxazoliumiodid, 3-Ethyl-2-methyl-benzothiazoliumiodid, 1-Ethyl-4-methyl-chinoliniumiodid, 1-Ethyl-2-methylchinoliniumiodid, 1,2,3-Trimethylchinoxaliniumiodid, 3-Ethyl-2-methyl-benzoxazolium-p-toluolsulfonat, 3-Ethyl-2-methyl-benzothiazolium-p-toluolsulfonat, 1-Ethyl-4-methyl-chinolinium-p-toluolsulfonat, 1-Ethyl-2-methylchinolinium-p-toluolsulfonat, und 1,2,3-Trimethylchinoxalinium-p-toluolsulfonat.

Bevorzugte primäre oder sekundäre aromatische Amine der Komponente B sind ausgewählt aus N,N-Dimethyl-p-phenylendiamin, N,N-Diethyl-p-phenylendiamin, N-(2-Hydroxyethyl)-N-ethyl-p-phenylendiamin, N,N-Bis-(2-hydroxyethyl)-p-phenylendiamin, N-(2-Methoxyethyl)-p-phenylendiamin, 2,3-Dichlor-p-phenylendiamin, 2,4-Dichlor-p-phenylendiamin, 2,5-Dichlor-p-phenylendiamin, 2-Chlor-p-phenylendiamin, 2,5-Dihydroxy-4-morpholinoanilin, 2-Aminophenol, 3-Aminophenol, 4-Aminophenol, 2-Aminomethyl-4-aminophenol, 2-Hydroxymethyl-4-aminophenol, o-Phenylendiamin, m-Phenylendiamin, p-Phenylendiamin, 2,5-Diaminotoluol, 2,5,-Diaminophenol, 2,5-Diaminoanisol, 2,5,Diaminophenethol, 4-Amino-3-methylphenol, 2-(2,5-Diaminophenyl)-ethanol, 2,4-Diaminophenoxyethanol, 2-(2,5-Diaminophenoxy)-ethanol, 3-Amino-4-(2-hydroxyethyloxy)phenol, 3,4-Methylendioxyphenol, 3,4-Methylendioxyanilin, 3-Amino-2,4-dichlorphenol, 4-Methylaminophenol, 2-Methyl-5-aminophenol, 3-Methyl-4-aminophenol, 2-Methyl-5-(2-hydroxyethylamino)phenol, 3-Amino-2-chlor-6-methylphenol, 2-Methyl-5-amino-4-chlorphenol, 5-(2-Hydroxyethylamino)-4-methoxy-2-methylphenol, 4-Amino-2-hydroxymethylphenol, 2-(Diethylaminomethyl)-4-aminophenol, 4-Amino-1-hydroxy-2-(2-hydroxyethylaminomethyl)-benzol, 1-Hydroxy-2-amino-5-methyl-benzol, 1-Hydroxy-2-amino-6-methyl-benzol, 2-Amino-5-acetamido-phenol, 1,3-Dimethyl-2,5-diaminobenzol, 5-(3-Hydroxypropylamino-)2-methylphenol, 5-Amino-4-methoxy-2-methylphenol, N,N-Dimethyl-3-aminophenol, N-Cyclopentyl-3-aminophenol, 5-Amino-4-fluor-2-methylphenol, 2,4-Diamino-5-fluortoluot, 2,4-Diamino-5-(2-hydroxyethoxy)-toluol, 2,4-Diamino-5-methylphenetol, 3,5-Diamino-2-methoxy-1-methylbenzol, 2-Amino-4-(2-hydroxyethylamino)-anisol, 2,6-Bis-(2-hydroxyethylamino)-1-methylbenzol, 1,3-Diamino-2,4-dimethoxybenzol, 3,5-Diamino-2-methoxy-toluol, 2-Aminobenzoesäure, 3-Aminobenzoesäure, 4-Aminobenzoesäure, 2-Aminophenylessigsäure, 3-Aminophenylessigsäure, 4-Aminophenylessigsäure, 2,3-Diaminobenzoesäure, 2,4-Diaminobenzoesäure, 2,5-Diaminobenzoesäure, 3,4-Diaminobenzoesäure 3,5-Diaminobenzoesäure, 4-Aminosalicylsäure, 5-Aminosaücyisäure, 3-Amino-4-hydroxybenzoesäure, 4-Amino-3-hydroxy-benzoesäure, 2-Aminobenzolsulfonsäure, 3-Aminobenzolsulfonsäure, 4-Aminobenzolsulfonsäure, 3-Amino-4-hydroxybenzolsulfonsäure, 4-Amino-3-hydroxynaphthalin-1-sulfonsäure, 6-Amino-7-hydroxynaphthalin-2-sulfonsäure, 7-Amino-4-hydroxynaphthalin-2-sulfonsäure, 4-Amino-5-hydroxynaphthalin-2,7-disulfonsäure, 3-Amino-2-naphthoesäure, 3-Aminophthalsäure, 5-Aminoisophthalsäure, 1,3,5-Triaminobenzol, 1,2,4-Triaminobenzol, 1,2,4,5-Tetraaminobenzol, 2,4,5-Triaminophenol, Pentaaminobenzol, Hexaaminobenzol, 2,4,6-Triaminoresorcin, 4,5-Diaminobrenzcatechin, 4,6-Diaminopyrogallol, 1-(2-Hydroxy-5-aminobenzyl)-2-imidazolidinon, 4-Amino-2-((4-[(5-amino-2-hydroxyphenyl)methyl]-piperazinyl)methyl)phenol, 3,5-Diamino-4-hydroxybrenzcatechin, 1,4-Bis-(4-aminophenyl)-1,4-diazacycloheptan, aromatische Nitrile, wie 2-Amino-4-hydroxybenzonitril, 4-Amino-2-hydroxybenzonitril, 4-Aminobenzonitril, 2,4-Diaminobenzonitril, Nitrogruppen-haltige Aminoverbindungen, wie 3-Amino-6-methylamino-2-nitro-pyridin, Pikraminsäure, [8-[(4-Amino-2-nitrophenyl)-azo]-7-hydroxy-naphth-2-yl]-trimethylammoniumchlorid, [8-((4-Amino-3-nitrophenyl)-azo)-7-hydroxy-naphth-2-yl]-trimethylammoniumchlorid (Basic Brown 17), 1-Hydroxy-2-amino-4,6-dinitrobenzol, 1-Amino-2-nitro-4-[bis-(2-hydroxyethyl)amino]-benzol, 1-Amino-2-[(2-hydroxyethyl)amino]-5-nitrobenzol (HC Yellow Nr.5), 1-Amino-2-nitro-4-[(2-hydroxyethyl)amino]-benzol (HC Red Nr. 7), 2-Chlor-5-nitro-N-2-hydroxyethyl-1,4-phenylendiamin, 1-[(2-Hydroxyethyl)amino]-2-nitro-4-amino-benzol (HC Red Nr. 3), 4-Amino-3-nitrophenol, 4-Amino-2-nitrophenol, 6-Nitro-o-toluidin, 1-Amino-3-methyl-4-[(2-hydroxyethyl)amino]-6-nitrobenzol (HC Violet Nr. 1), 1-Amino-2-nitro-4-[(2,3-dihydroxypropyl)amino]-5-chlor-benzol (HC Red Nr. 10), 2-(4-Amino-2-nitroanilino)-benzoesäure, 6-Nitro-2,5-diaminopyridin, 2-Amino-6-chlor-4-nitrophenol, 1-Amino-2-(3-nitrophenylazo)-7-phenylazo-8-naphthol-3,6-disulfonsäure Dinatriumsalz (Acid blue Nr. 29), 1-Amino-2-(2-hydroxy-4-nitrophenylazo)-8-naphthol-3,6-disulfonsäure Dinatriumsalz (Palatinchrome green), 1-Amino-2-(3-chlor-2-hydroxy-5-nitrophenylazo)-8-naphthol-3,6-disulfonsäure Dinatriumsalz (Gallion), 4-Amino-4'-nitrostilben-2,2'-disulfonsäure Dinatriumsalz, 2,4-Diamino-3',5'-dinitro-2'-hydroxy-5-methyl-azobenzol (Mordant brown 4), 4'-Amino-4-nitrodiphenylamin-2-sulfonsäure, 4'-Amino-3'-nitrobenzophenon-2-carbonsäure, 1-Amino-4-nitro-2-(2-nitrobenzylidenamino)-benzol, 2-[2-(Diethylamino)ethylamino]-5-nitroanilin, 3-Amino-4-hydroxy-5-nitrobenzolsulfonsäure, 3-Amino-3'-nitrobiphenyl, 3-Amino-4-nitro-acenaphthen, 2-Amino-1-nitronaphthalin, 5-Amino-6-nitrobenzo-1,3-dioxol, Aniline, insbesondere Nitrogruppen-haltige Aniline, wie 4-Nitroanilin, 2-Nitroanilin, 1,4-Diamino-2-nitrobenzol, 1,2-Diamino-4-nitrobenzol, 1-Amino-2-methyl-6-nitrobenzol, 4-Nitro-1,3-phenylendiamin, 2-Nitro-4-amino-1-(2-hydroxyethylamino)-benzol, 2-Nitro-1-amino-4-[bis-(2-hydroxyethyl)-amino]-benzol, 4-Amino-2-nitrodiphenylamin-2'-carbonsäure, 1-Amino-5-chlor-4-(2-hydroyethylamino)-2-nitrobenzol, aromatische Aniline bzw. Phenole mit einem weiteren aromatischen Rest, wie sie in der Formel II dargestellt sind 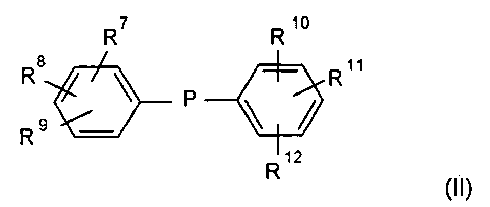 in der
- R⁷ für eine Hydroxy- oder eine Aminogruppe, die durch C₁₋₄-Alkyl-, C₁₋₄-Hydroxyalkyl, C₁₋₄-Alkoxy- oder C₁₋₄-Alkoxy-C₁₋₄-alkylgruppen substituiert sein kann, steht,
- R⁸, R⁹, R¹⁰, R¹¹ und R¹² unabhängig voneinander für ein Wasserstoffatom, eine Hydroxy- oder eine Aminogruppe, die durch C₁-C₄-Alkyl-, C₁-C₄-Hydroxyalkyl, C₁-C₄-Alkoxy-, C₁C₄-Aminoalkyl- oder C₁-C₄-Alkoxy-C₁-C₄-alkylgruppen substituiert sein kann, stehen, und
- P für eine direkte Bindung, eine gesättigte oder ungesättigte, ggf. durch Hydroxygruppen substituierte Kohlenstoffkette mit 1 bis 4 Kohlenstoffatomen, eine Carbonyl-, Sulfoxy-, Sulfonyl- oder Iminogruppe, ein Sauerstoff- oder Schwefelatom, oder eine Gruppe mit der Formel III

   -Q'-(CH₂-Q-CH₂-Q")ₒ- (III)

   in der
   - Q eine direkte Bindung, eine CH₂- oder CHOH-Gruppe bedeutet,
   - Q' und Q" unabhängig voneinander für ein Sauerstoffatom, eine NR¹³-Gruppe, worin R¹³ ein Wasserstoffatom, eine C₁₋₄-Alkyl- oder eine Hydroxy-C₁₋₄-alkylgruppe, wobei auch beide Gruppen zusammen mit dem Restmolekül einen 5-, 6- oder 7-Ring bilden können, bedeutet, die Gruppe O-(CH₂)ₚ-NH oder NH-(CH₂)_{p'}-O, worin p und p' 2 oder 3 sind, stehen und
   - o eine Zahl von 1 bis 4 bedeutet,
wie beispielsweise 4,4'-Diaminostilben und dessen Hydrochlorid, 4,4'-Diaminostilben-2,2'-disulfonsäure-mono- oder -di-Na-Salz, 4-Amino-4'-dimethylaminostilben und dessen Hydrochlorid, 4,4'-Diaminodiphenylmethan, 4,4'-Diaminodiphenylsuifid, 4,4'-Diaminodiphenylsulfoxid, 4,4'-Diaminodiphenylamin, 4,4'-Diaminodiphenylamin-2-sulfonsäure, 4,4'-Diaminobenzophenon, 4,4'-Diaminodiphenylether, 3,3',4,4'-Tetraaminodiphenyl, 3,3',4,4'-Tetraamino-benzophenon, 1,3-Bis-(2,4-diaminophenoxy)-propan, 1,8-Bis-(2,5-diaminophenoxy)-3,6-dioxaoctan, 1,3-Bis-(4-aminophenylamino)propan, , 1,3-Bis-(4-aminophenylamino)-2-propanol, 1,3-Bis-[N-(4-aminophenyl)-2-hydroxyethylamino]-2-propanol, N,N-Bis-[2-(4-aminophenoxy)-ethyl]-methylamin, N-Phenyl-1,4-phenylendiamin und Bis-(5-amino-2-hydroxyphenyl)-methan.

Die vorgenannten Verbindungen können sowohl in freier Form als auch in Form ihrer physiologisch verträglichen Salze, insbesondere als Salze anorganischer Säuren, wie Salz- oder Schwefelsäure, eingesetzt werden.

Geeignete stickstoffhaltige heterocyclische Verbindungen sind z. B. 2-Aminopyridin, 3-Aminopyridin, 4-Aminopyridin, 2-Amino-3-hydroxy-pyridin, 2,6-Diamino-pyridin, 2,5-Diamino-pyridin, 2-(Aminoethylamino)-5-aminopyridin, 2,3-Diamino-pyridin, 2-Dimethylamino-5-amino-pyridin, 2-Methylamino-3-amino-6-methoxy-pyridin, 2,3-Diamino-6-methoxy-pyridin, 2,6-Dimethoxy-3,5-diamino-pyridin, 2,4,5-Triamino-pyridin, 2,6-Dihydroxy-3,4-dimethylpyridin, N-[2-(2,4-Diaminophenyl)aminoethyl]-N-(5-amino-2-pyridyl)-amin, N-[2-(4-Aminophenyl)aminoethyl]-N-(5-amino-2-pyridyl)-amin, 2,4-Dihydroxy-5,6-diaminopyrimidin, 4,5,6-Triaminopyrimidin, 4-Hydroxy-2,5,6-triaminopyrimidin, 2-Hydroxy-4,5,6-triaminopyrimidin, 2,4,5,6-Tetraaminopyrimidin, 2-Methylamino-4,5,6-triaminopyrimidin, 2,4-Diaminopyrimidin, 4,5-Diaminopyrimidin, 2-Amino-4-methoxy-6-methylpyrimidin, 3,5-Diaminopyrazol, 3,5-Diamino-1,2,4-triazol, 3-Aminopyrazol, 3-Amino-5-hydroxypyrazol, 1-Phenyl-4,5-diaminopyrazol, 1-(2-Hydroxyethyl)-4,5-diaminopyrazol, 1-Phenyl-3-methyl-4,5-diaminopyrazol, 4-Amino-2,3-dimethyl-1-phenyl-3-pyrazolin-5-on (4-Aminoantipyrin), 1-Phenyl-3-methylpyrazol-5-on, 2-Aminochinolin, 3-Aminochinolin, 8-Aminochinolin, 4-Aminochinaldin, 2-Aminonicotinsäure, 6-Aminonicotinsäure, 5-Aminoisochinolin, 5-Aminoindazol, 6-Aminoindazol, 5-Aminobenzimidazol, 7-Aminobenzimidazol, 5-Aminobenzothiazol, 7-Aminobenzothiazol, 2,5-Dihydroxy-4-morpholino-anilin sowie Indol- und Indolinderivaten, wie 4-Aminoindol, 5-Aminoindol, 6-Aminoindol, 7-Aminoindol, 5,6-Dihydroxyindol, 5,6-Dihydroxyindolin und 4-Hydroxyindolin. Weiterhin als heterocyclische Verbindungen können erfindungsgemäß die in der DE-U1-299 08 573 offenbarten Hydroxypyrimidine eingesetzt werden. Die vorgenannten Verbindungen können sowohl in freier Form als auch in Form ihrer physiologisch verträglichen Salze, z. B. als Salze anorganischer Säuren, wie Salz- oder Schwefelsäure, eingesetzt werden.

Geeignete aromatische Hydroxyverbindungen sind z. B. 2-Methylresorcin, 4-Methylresorcin, 5-Methylresorcin, 2,5-Dimethylresorcin, Resorcin, 3-Methoxyphenol, Brenzkatechin, Hydrochinon, Pyrogallol, Phloroglucin, Hydroxyhydrochinon, 2-Methoxyphenol, 3-Methoxyphenol, 4-Methoxyphenol, 3-Dimethylaminophenol, 2-(2-Hydroxyethyl)phenol, 3,4-Methylendioxyphenol, 2,4-Dihydroxybenzoesäure, 3,4-Dihydroxybenzoesäure, 2,4-Dihydroxy-phenylessigsäure, 3,4-Dihydroxy-phenylessigsäure, Gallussäure, 2,4,6-Trihydroxybenzoesäure, 2,4,6-Trihydroxyacetophenon, 2-Chlorresorcin, 4-Chlorresorcin, 1-Naphthol, 1,5-Dihydroxynaphthalin, 2,3-Dihydroxynaphthalin, 2,7-Dihydroxynaphthalin, 6-Dimethylamino-4-hydroxy-2-naphthalinsulfonsäure und 3,6-Dihydroxy-2,7-naphthalinsulfonsäure.

Die Verbindungen der Komponente A und die Verbindungen der Komponente B werden vorzugsweise in den erfindungsgemäßen Nuanciermitteln jeweils in einer Menge von 0,03 bis 65 mmol, insbesondere von 1 bis 40 mmol, bezogen auf 100 g des gesamten Nuanciermittels, verwendet. Das molare Verhältnis von der Verbindung der Komponente A und der Verbindung der Komponente B kann im Bereich von 0,5 bis 2,0 liegen, wobei vorzugsweise äquimolare Mengen eingesetzt werden. Das eigentliche Nuanciermittel wird bei getrennter Lagerung der Komponenten A und B unmittelbar vor den Anwendung durch Mischen hergestellt.

Eine oxidative Färbung der Fasern kann in Gegenwart von Oxidationsfarbstoffvorprodukten grundsätzlich mit Luftsauerstoff erfolgen. Bevorzugt wird jedoch ein chemisches Oxidationsmittel eingesetzt, besonders dann, wenn neben der Färbung ein Aufhelleffekt der natürlichen Pigmente des menschlichen Haars gewünscht ist. Dieser Aufhelleffekt kann unabhängig von der Färbemethode gewünscht sein. Die Gegenwart von Oxidationsfarbstoffvorprodukten ist demnach keine zwingende Voraussetzung für einen Einsatz von Oxidationsmitteln in den Nuanciermitteln des erfindungsgemäßen Verfahrens. Als Oxidationsmittel kommt insbesondere Wasserstoffperoxid und/oder mindestens ein Anlagerungsprodukt davon, insbesondere an anorganische oder organische Verbindungen, wie beispielsweise Natriumperborat, Natriumpercarbonat, Magnesiumpercarbonat, Natriumpercarbamid, Polyvinylpyrrolidon·n H₂O₂ (n ist eine positive ganze Zahl größer 0), Harnstoffperoxid und Melaminperoxid, dem Nuanciermittel zugesetzt, in Frage. Das eigentliche Nuanciermittel wird bei getrennter Lagerung der Farbstoffvorprodukte und des Oxidationsmittels unmittelbar vor der Anwendung durch Mischen hergestellt.

Erfindungsgemäß kann aber das Nuanciermittel auch zusammen mit einem Katalysator auf das Haar aufgebracht werden, der die Oxidation der Farbstoffvorprodukte, z.B. durch Luftsauerstoff, aktiviert. Solche Katalysatoren sind z.B. Metallionen, lodide, Chinone oder bestimmte Enzyme.

Geeignete Metallionen sind beispielsweise Zn²⁺, Cu²⁺, Fe²⁺, Fe³⁺, Mn²⁺, Mn⁴⁺, Li⁺, Mg²⁺, Ca²⁺ und Al³⁺. Besonders geeignet sind dabei Zn²⁺, Cu²⁺ und Mn²⁺. Die Metallionen können prinzipiell in der Form eines beliebigen, physiologisch verträglichen Salzes oder in Form einer Komplexverbindung eingesetzt werden. Bevorzugte Salze sind die Acetate, Sulfate, Halogenide, Lactate und Tartrate. Durch Verwendung dieser Metallsalze kann sowohl die Ausbildung der Färbung beschleunigt als auch die Farbnuance gezielt beeinflusst werden.

Geeignete Enzyme sind z.B. Peroxidasen, die die Wirkung geringer Mengen an Wasserstoffperoxid deutlich verstärken können. Weiterhin sind solche Enzyme erfindungsgemäß geeignet, die mit Hilfe von Luftsauerstoff die Oxidationsfarbstoffvorprodukte direkt oxidieren, wie beispielsweise die Laccasen, oder *in situ* geringe Mengen Wasserstoffperoxid erzeugen und auf diese Weise die Oxidation der Farbstoffvorprodukte biokatalytisch aktivieren. Besonders geeignete Katalysatoren für die Oxidation der Farbstoffvorläufer sind die sogenannten 2-Elektronen-Oxidoreduktasen in Kombination mit den dafür spezifischen Substraten, z.B.
- Pyranose-Oxidase und z.B. D-Glucose oder Galactose,
- Glucose-Oxidase und D-Glucose,
- Glycerin-Oxidase und Glycerin,
- Pyruvat-Oxidase und Benztraubensäure oder deren Salze,
- Alkohol-Oxidase und Alkohol (MeOH, EtOH),
- Lactat-Oxidase und Milchsäure und deren Salze,
- Tyrosinase-Oxidase und Tyrosin,
- Uricase und Harnsäure oder deren Salze,
- Cholinoxidase und Cholin,
- Aminosäure-Oxidase und Aminosäuren.

Bei einer Anwendung von Oxidationsmitteln wird das eigentliche Nuanciermittel zweckmäßigerweise unmittelbar vor der Anwendung durch Mischung der Zubereitung des Oxidationsmittels mit der Zubereitung, enthaltend Farbstoffvorprodukte, hergestellt. Das dabei entstehende gebrauchsfertige Haarfärbepräparat sollte bevorzugt einen pH-Wert im Bereich von 6 bis 12 aufweisen. Besonders bevorzugt ist die Anwendung der Nuanciermittel, wenn Sie als Haarfärbemittel Verwendung finden, in einem schwach alkalischen Milieu. Die Anwendungstemperaturen können in einem Bereich zwischen 15 und 40 °C liegen. Nach einer Einwirkungszeit von 5 bis 45 Minuten wird das Haarfärbemittel durch Ausspülen von dem zu färbenden Haar entfernt. Das Nachwaschen mit einem Shampoo entfällt, wenn ein stark tensidhaltiger Träger, z.B. ein Färbeshampoo, verwendet wurde.

Insbesondere bei schwer färbbarem Haar kann ein erfindungsgemäßes Nuanciermittel gegebenenfalls mit zusätzlichen Farbstoffvorprodukten aber auch ohne vorherige Vermischung mit der Oxidationskomponente auf das Haar aufgebracht werden. Nach einer Einwirkdauer von 20 bis 30 Minuten wird dann - gegebenenfalls nach einer Zwischenspülung - die Oxidationskomponente aufgebracht. Nach einer weiteren Einwirkdauer von 10 bis 20 Minuten wird dann gespült und gewünschtenfalls nachshampooniert. Bei dieser Ausführungsform wird gemäß einer ersten Variante, bei der das vorherige Aufbringen der Farbstoffvorprodukte eine bessere Penetration in das Haar bewirken soll, das entsprechende Mittel auf einen pH-Wert von etwa 4 bis 7 eingestellt. Gemäß einer zweiten Variante wird zunächst eine Luftoxidation angestrebt, wobei das aufgebrachte Mittel bevorzugt einen pH-Wert von 7 bis 10 aufweist. Bei der anschließenden beschleunigten Nachoxidation kann die Verwendung von sauer eingestellten Peroxidisulfat-Lösungen als Oxidationsmittel bevorzugt sein.

Es ist erfindungsgemäß bevorzugt, ein Nuanciermittel des erfindungsgemäßen Verfahrens als Bleichmittel für keratinhaltige Fasern, insbesondere menschliche Haare, zu formulieren. In dieser Ausführungsform enthalten die Nuanciermittel mindestens ein Oxidationsmittel und bevorzugt zusätzlich mindestens einen Bleichverstärker.

Als Oxidationsmittel eignet sich bevorzugt Wasserstoffperoxid. Das Wasserstoffperoxid wird als Lösung und/oder als feste Anlagerungsprodukte, insbesondere die im Rahmen der Ausführungsform des Nuanciermittels ais oxidatives Färbemittel definierten Anlagerungsprodukte, dem Nuanciermittel zugesetzt. In den Nuanciermitteln ist Wasserstoffperoxid bevorzugt in einer Menge von 0,5 bis 12,0 Gew.%, insbesondere 6 bis 12 Gew.-%, jeweils bezogen auf das Gewicht des Nuanciermittels, enthalten.

Die Nuanciermittel des erfindungsgemäßen Verfahrens enthalten bevorzugt zusätzlich mindestens einen Bleichverstärker. Die Bleichverstärker werden bevorzugt in Blondiermitteln zur Steigerung der Blondierwirkung des Oxidationsmittels, insbesondere des Wasserstoffperoxids, eingesetzt.

Als Bleichverstärker können Verbindungen, die unter Perhydrolysebedingungen aliphatische Peroxocarbonsäuren mit vorzugsweise 1 bis 10 C-Atomen, insbesondere 2 bis 4 C-Atomen, und/oder gegebenenfalls substituierte Perbenzoesäure ergeben, eingesetzt werden. Geeignet sind Substanzen, die O- und/oder N-Acylgruppen der genannten C-Atomzahl und/oder gegebenenfalls substituierte Benzoylgruppen tragen. Bevorzugt sind mehrfach acylierte Alkylendiamine, insbesondere Tetraacetylethylendiamin (TAED), acylierte Triazinderivate, insbesondere 1,5-Diacetyl-2,4-dioxohexahydro-1,3,5-triazin (DADHT), acylierte Glykolurile, insbesondere Tetraacetylglykoluril (TAGU), N-Acylimide, insbesondere N-Nonanoylsuccinimid (NOSI), acylierte Phenolsulfonate, insbesondere n-Nonanoyl- oder lsononanoyloxybenzolsulfonat (n- bzw. iso-NOBS), Carbonsäureanhydride, insbesondere Phthalsäureanhydrid, acylierte mehrwertige Alkohole, insbesondere Triacetin, Ethylenglykoldiacetat und 2,5-Diacetoxy-2,5-dihydrofuran.

Bleichverstärker sind bevorzugt feste Peroxoverbindungen. Unter erfindungsgemäß bleichverstärkende, feste Peroxoverbindungen fallen keine Anlagerungsprodukte von Wasserstoffperoxid an andere Komponenten. Die Auswahl dieser festen Peroxoverbindung unterliegt prinzipiell keinen Beschränkungen; übliche, dem Fachmann bekannte Peroxoverbindungen sind beispielsweise Ammoniumperoxidisulfat, Kaliumperoxidisulfat, Natriumperoxidisulfat, Ammoniumpersulfat, Kaliumpersulfat, Natriumpersulfat, Kaliumperoxidiphosphat und Peroxide wie Bariumperoxid und Magnesiumperoxid. Unter diesen Peroxoverbindungen, die auch in Kombination eingesetzt werden können, sind erfindungsgemäß die anorganischen Verbindungen bevorzugt. Besonders bevorzugt sind die Peroxidisulfate, insbesondere Ammoniumperoxidisulfat.

Die erfindungsgemäßen Nuanciermittel werden im Rahmen der Ausführungsform als Bleichmittel bevorzugt kurz vor der Anwendung auf der Faser durch Vermengen von mindestens zwei Komponenten hergestellt. Zu diesem Zweck wird eine Komponente 1, welche einen Bleichverstärker enthalten kann, mit einer Wasserstoffperoxid-Lösung als Komponente 2 vermischt. Komponente 1 liegt bevorzugt als Creme, Paste, W/O-Emulsion, O/W-Emulsion oder als Öl vor.

Die Komponente 1 wiederum kann jedoch in einer weiteren erfindungsgemäßen Variante dieser Ausführungsform aus einer Komponente 1 a und einer Komponente 1 b, enthaltend mindestens einen Bleichverstärker, durch Mischung hergestellt werden. Dabei liegt die Komponente 1 a bevorzugt in Form einer W/O- oder O/W-Emulsion und die Komponente 1 b als Feststoff, z.B. als Pulver oder Granulat, vor.

Die Konzentration der Wasserstoffperoxid-Lösung der Komponente 2 wird einerseits von den gesetzlichen Vorgaben und andererseits von dem gewünschten Effekt bestimmt; in der Regel werden 6- bis 12-prozentige Lösungen in Wasser verwendet. Die Mengenverhältnisse von Komponente 1 und der Komponente 2 liegen dabei üblicherweise im Bereich 1.5:1 bis 1:2, wobei ein Überschuß an Komponente 1 insbesondere dann gewählt wird, wenn keine zu ausgeprägte Blondierwirkung erwünscht ist.

In der Ausführungsform als Blondiermittel sind die Bleichverstärker in den erfindungsgemäßen anwendungsfertigen Mitteln bevorzugt in Mengen von 5-30 Gew.-%, insbesondere in Mengen von 8-20 Gew.-%, enthalten.

Die Nuanciermittel des erfindungsgemäßen Verfahrens enthalten, wenn sie als Bleichmittel fungieren, als bevorzugtes Alkalisierungsmittel mindestens eine Verbindung, ausgewählt aus Ammonium-, Alkalimetall- und Erdalkalimetallhydroxiden, -carbonaten, -hydrogencarbonaten, -hydroxycarbonaten und -carbamiden, sowie Alkaliphosphaten.

Der pH-Wert der erfindungsgemäß verwendeten Nuanciermittel liegt bevorzugt in einem pH-Bereich von pH 2.5 bis 12.0, besonders bevorzugt von pH 8.5 bis 11.0.

Ferner kann das Nuanciermittel im Allgemeinen zusätzlich mindestens eine SiO₂-Verbindung, die gegebenenfalls hydratisiert sein kann, enthalten. Es kann erfindungsgemäß bevorzugt sein, die gegebenenfalls hydratisierten SiO₂-Verbindungen in Mengen von 0,05 Gew.-% bis 15 Gew.-%, besonders bevorzugt in Mengen von 0,15 Gew.-% bis 10 Gew.-% und ganz besonders bevorzugt in Mengen von 0,2 Gew.-% bis 5 Gew.-%, jeweils bezogen auf das gesamte Nuanciermittel, einzusetzen. Die Mengenangaben geben dabei jeweils den Gehalt der SiO₂-Verbindungen (ohne deren Wasseranteil) in den Mitteln wieder.

Hinsichtlich der gegebenenfalls hydratisierten SiO₂-Verbindungen unterliegt die vorliegende Erfindung prinzipiell keinen Beschränkungen. Bevorzugt sind Kieselsäuren, deren Oligomeren und Polymeren sowie deren Salze. Bevorzugte Salze sind die Alkalisalze, insbesondere die Kalium und Natriumsalze. Die Natriumsalze sind ganz besonders bevorzugt.

Die gegebenenfalls hydratisierten SiO₂-Verbindungen können in verschiedenen Formen vorliegen. Erfindungsgemäß bevorzugt werden die SiO₂-Verbindungen in Form von Kieselgelen (Silicagel) oder besonders bevorzugt als Wasserglas eingesetzt. Diese SiO₂-Verbindungen können teilweise in wäßriger Lösung vorliegen.

Erfindungsgemäß ganz besonders bevorzugt sind Wassergläser, die aus einem Silikat der Formel (SiO₂)ₙ(Na₂O)ₘ(K₂O)ₚ gebildet werden, wobei n steht für eine positive rationale Zahl und m und p stehen unabhängig voneinander für eine positive rationale Zahl oder für 0, mit den Maßgaben, daß mindestens einer der Parameter m oder p von 0 verschieden ist und das Verhältnis zwischen n und der Summe aus m und p zwischen 1:4 und 4:1 liegt.

Neben den durch die Summenformel beschriebenen Komponenten können die Wassergläser in geringen Mengen noch weitere Zusatzstoffe, wie beispielsweise Phosphate oder Magnesiumsalze, enthalten.

Erfindungsgemäß besonders bevorzugte Wassergläser werden unter anderem von der Firma Henkel unter den Bezeichnungen Ferrosil^{®} 119, Natronwasserglas 40/42, Portil^{®} A, Portil^{®} AW, Portil^{®} N und Portil^{®} W und von der Firma Akzo unter der Bezeichnung Britesil^{®} C20 vertrieben.

Das Nuanciermittel besitzt bevorzugt eine Viskosität von 5000 bis 100000 mPa·s, besonders bevorzugt von 30000 bis 80000 mPa·s (Brookfield Rotationsviskosimeter, 25°C, Spindel #4, 20 rpm).

Die Viskosität kann durch ein Verdickungsmittel eingestellt werden, welches in dem Nuanciermittel zusätzlich enthalten sein kann. Polymere können die Viskosität von wäßrigen und nicht-wäßrigen Phasen in kosmetischen Zubereitungen erhöhen. In wäßrigen Phasen beruht ihre die Viskosität erhöhende Funktion auf ihrer Löslichkeit in Wasser oder ihrer hydrophilen Natur. Sie werden sowohl in tensidischen als auch in emulsionsförmigen Systemen angewendet. Im folgenden werden einige Beispiele bevorzugter polymerer Verdicker angeführt, die in dem Nuanciermittel des erfindungsgemäßen Verfahrens enthalten sein können:
Acrylamides Copolymer, Acrylamide/Sodium Acrylate Copolymer, Acrylamide/Sodium Acryloyldimethyltaurate Copolymer, Acrylates/Acetoacetoxyethyl Methacrylate Copolymer, Acrylates/Beheneth-25 Methacrylate Copolymer, Acrylates/C10-30 Alkyl Acrylate Crosspolymer, Acrylates/Ceteth-20 Itaconate Copolymer, Acrylates/Ceteth-20 Methacrylate Copolymer, Acrylates/Laureth-25 Methacrylate Copolymer, Acrylates/Palmeth-25 Acrylate Copolymer, Acrylates/Palmeth-25 Itaconate Copolymer, Acrylates/Steareth-50 Acrylate Copolymer, Acrylates/Steareth-20 Itaconate Copolymer, Acrylates/Steareth-20 Methacrylate Copolymer, Acrylates/Stearyl Methacrylate Copolymer, Acrylates/Vinyl Isodecanoate Crosspolymer, Acrylic Acid/Acrylonitrogens Copolymer, Agar, Agarose, Alcaligenes Polysaccharides, Algin, Alginic Acid, Ammonium Acrylates/Acrylonitrogens Copolymer, Ammonium Acrylates Copolymer, Ammonium Acryloyldimethyltaurate/Vinyl Formamide Copolymer, Ammonium Acryloyldimethyltaurate/VP Copolymer, Ammonium Alginate, Ammonium Polyacryloyldimethyl Taurate, Amylopectin, Ascorbyl Methylsilanol Pectinate, Astragalus Gummifer Gum, Attapulgite, Avena Sativa (Oat) Kernel Flour, Bentonite, Butoxy Chitosan, Caesalpinia Spinosa Gum, Calcium Alginate, Calcium Carboxymethyl Cellulose, Calcium Carrageenan, Calcium Potassium Carbomer, Calcium Starch Octenylsuccinate, C20-40 Alkyl Stearate, Carbomer, Carboxybutyl Chitosan, Carboxymethyl Chitin, Carboxymethyl Chitosan, Carboxymethyl Dextran, Carboxymethyl Hydroxyethylcellulose, Carboxymethyl Hydroxypropyl Guar, Cellulose Acetate Propionate Carboxylate, Cellulose Gum, Ceratonia Siliqua Gum, Cetyl Hydroxyethylcellulose, Cholesterol/HDI/Pullulan Copolymer, Cholesteryl Hexyl Dicarbamate Pullulan, Cyamopsis Tetragonoloba (Guar) Gum, Diglycol/CHDM/Isophthalates/SIP Copolymer, Dihydrogenated Tallow Benzylmonium Hectorite, Dimethicone Crosspolymer-2, Dimethicone Propyl PG-Betaine, DMAPA Acrylates/Acrylic Acid/Acrylonitrogens Copolymer, Ethylene/Sodium Acrylate Copolymer, Gelatin, Gellan Gum, Glyceryl Alginate, Glycine Soja (Soybean) Flour, Guar Hydroxypropyltrimonium Chloride, Hectorite, Hydrated Silica, Hydrogenated Potato Starch, Hydroxybutyl Methylcellulose, Hydroxyethyl Acrylate/Sodium Acryloyldimethyl Taurate Copolymer, Hydroxyethylcellulose, Hydroxyethyl Chitosan, Hydroxyethyl Ethylcellulose, Hydroxypropylcellulose, Hydroxypropyl Chitosan, Hydroxypropyl Ethylenediamine Carbomer, Hydroxypropyl Guar, Hydroxypropyl Methylcellulose, Hydroxypropyl Methylcellulose Stearoxy Ether, Hydroxypropyl Starch, Hydroxypropyl Starch Phosphate, Hydroxypropyl Xanthan Gum, Hydroxystearamide MEA, Isobutylene/Sodium Maleate Copolymer, Lithium Magnesium Silicate, Lithium Magnesium Sodium Silicate, Macrocystis Pyrifera (Kelp), Magnesium Alginate, Magnesium Aluminum Silicate, Magnesium Silicate, Magnesium Trisilicate, Methoxy PEG-22/Dodecyl Glycol Copolymer, Methylcellulose, Methyl Ethylcellulose, Methyl Hydroxyethylcellulose, Microcrystalline Cellulose, Montmorillonite, Moroccan Lava Clay, Natto Gum, Nonoxynyl Hydroxyethylcellulose, Octadecene/MA Copolymer, Pectin, PEG-800, PEG-Crosspolymer, PEG-150/Decyl Alcohol/SMDI Copolymer, PEG-175 Diisostearate, PEG-190 Distearate, PEG-15 Glyceryl Tristearate, PEG-140 Glyceryl Tristearate, PEG-240/HDI Copolymer Bis-Decyltetradeceth-20 Ether, PEG-100/IPDI Copolymer, PEG-180/Laureth-50/TMMG Copolymer, PEG-10/Lauryl Dimethicone Crosspolymer, PEG-15/Lauryl Dimethicone Crosspolymer, PEG-2M, PEG-5M, PEG-7M, PEG-9M, PEG-14M, PEG-20M, PEG-23M, PEG-25M, PEG-45M, PEG-65M, PEG-90M, PEG-115M, PEG-160M, PEG-120 Methyl Glucose Trioleate, PEG-180/Octoxynol-40/TMMG Copolymer, PEG-150 Pentaerythrityl Tetrastearate, PEG-4 Rapeseedamide, PEG-150/Stearyl Alcohol/SMDI Copolymer, Polyacrylate-3, Polyacrylic Acid, Polycyclopentadiene, Polyether-1, Polyethylene/Isopropyl Maleate/MA Copolyol, Polymethacrylic Acid, Polyquaternium-52, Polyvinyl Alcohol, Potassium Alginate, Potassium Aluminum Polyacrylate, Potassium Carbomer, Potassium Carrageenan, Potassium Polyacrylate, Potato Starch Modified, PPG-14 Laureth-60 Hexyl Dicarbamate, PPG-14 Laureth-60 lsophoryl Dicarbamate, PPG-14 Palmeth-60 Hexyl Dicarbamate, Propylene Glycol Alginate, PVP/Decene Copolymer, PVP Montmorillonite, Rhizobian Gum, Ricinoleic Acid/Adipic Acid/AEEA Copolymer, Sclerotium Gum, Sodium Acrylate/Acryloyldimethyl Taurate Copolymer, Sodium Acrylates/Acrolein Copolymer, Sodium Acrylates/Acrylonitrogens Copolymer, Sodium Acrylates Copolymer, Sodium Acrylates/Vinyl Isodecanoate Crosspolymer, Sodium Acrylate/Vinyl Alcohol Copolymer, Sodium Carbomer, Sodium Carboxymethyl Chitin, Sodium Carboxymethyl Dextran, Sodium Carboxymethyl Beta-Glucan, Sodium Carboxymethyl Starch, Sodium Carrageenan, Sodium Cellulose Sulfate, Sodium Cyclodextrin Sulfate, Sodium Hydroxypropyl Starch Phosphate, Sodium Isooctylene/MA Copolymer, Sodium Magnesium Fluorosilicate, Sodium Polyacrylate, Sodium Polyacrylate Starch, Sodium Polyacryloyldimethyl Taurate, Sodium Polymethacrylate, Sodium Polystyrene Sulfonate, Sodium Silicoaluminate, Sodium Starch Octenylsuccinate, Sodium Stearoxy PG-Hydroxyethylcellulose Sulfonate, Sodium Styrene/Acrylates Copolymer, Sodium Tauride Acrylates/Acrylic Acid/Acrylonitrogens Copolymer, Solanum Tuberosum (Potato) Starch, Starch/Acrylates/Acrylamide Copolymer, Starch Hydroxypropyltrimonium Chloride, Steareth-60 Cetyl Ether, Steareth-100/PEG-136/HDI Copolymer, Sterculia Urens Gum, Synthetic Fluorphlogopite, Tamarindus Indica Seed Gum, Tapioca Starch, TEA-Alginate, TEA-Carbomer, Triticum Vulgare (Wheat) Starch, Tromethamine Acrylates/Acrylonitrogens Copolymer, Tromethamine Magnesium Aluminum Silicate, Welan Gum, Xanthan Gum, Yeast Beta-Glucan, Yeast Polysaccharides, Zea Mays (Corn) Starch.

In einer weiteren Ausführungsform des erfindungsgemäßen Verfahrens können die keratinhaltigen Fasern vor Schritt (i) komplett einer Färbung mit einem Färbemittel unterzogen werden. Ein Verfahren einer Nuancierung von bereits gefärbtem Haar wird in der Druckschrift WO-A1-2005/051336 beschrieben auf die ausdrücklich und vollinhaltlich Bezug genommen wird. In dieser Ausführungsform ist es bevorzugt, durch die Nuancierung eine Aufhellung des zuvor gefärbten Haars gemäß WO-A1-2005/051336 zu erzielen. Daher findet gemäß dieser Ausführungsform bevorzugt eine Färbemittel/Nuanciermittel-Kombination gemäß WO-A1-2005/051336 Verwendung.

Darüber hinaus können die keratinhaltigen Fasern in einer anderen Ausführungsform des erfindungsgemäßen Verfahrens nach dem Schritt (v) einer kompletten Färbung mit einem Färbemittel unterzogen werden, wenn das verwendete Nuanciermittel ein Bleichmittel für keratinhaltige Fasern ist. Ein Verfahren einer kompletten Färbung von bereits mit einem Nuanciermittel, insbesondere einem Bleichmittel für keratinhaltige Fasern, in Teilbereichen nuanciertem Haar wird in der Patentanmeldung DE102004045414.0 beschrieben auf die ausdrücklich und vollinhaltlich Bezug genommen wird.

In beiden o.g. Ausführungsformen ist es bevorzugt, wenn das Färbemittel in einem kosmetischen Träger mindestens eine Verbindung enthält, ausgewählt
(a) aus Oxidationsfarbstoffvorprodukten vom Typ der Entwicklerkomponenten und gegebenenfalls der Kupplerkomponenten und/oder
(b) aus reaktiven Carbonylverbindungen und/oder
(c) aus direktziehenden Farbstoffen und/oder
(d) aus Vorstufen naturanaloger Farbstoffe und/oder
(e) aus Oxidationsmitteln.
Als bevorzugte Verbindungen der entsprechenden Klassen (a) bis (e) des Färbemittels gelten die im Rahmen der Ausführungsform des Nuanciermittels entsprechend genannten Vertreter.

Die Färbemittel sowie die Nuanciermittel des erfindungsgemäßen Verfahrens können weiterhin alle für solche Zubereitungen bekannten Wirk-, Zusatz- und Hilfsstoffe enthalten.

In vielen Fällen enthalten die Färbemittel und/oder die Nuanciermittel mindestens ein Tensid, wobei prinzipiell sowohl anionische als auch zwitterionische, ampholytische, nichtionische und kationische Tenside geeignet sind. In vielen Fällen hat es sich aber als vorteilhaft erwiesen, die Tenside aus anionischen, zwitterionischen oder nichtionischen Tensiden auszuwählen.

Als anionische Tenside eignen sich in erfindungsgemäßen Zubereitungen alle für die Verwendung am menschlichen Körper geeigneten anionischen oberflächenaktiven Stoffe. Diese sind gekennzeichnet durch eine wasserlöslichmachende, anionische Gruppe wie z. B. eine Carboxylat-, Sulfat-, Sulfonat- oder Phosphat-Gruppe und eine lipophile Alkylgruppe mit etwa 10 bis 22 C-Atomen. Zusätzlich können im Molekül Glykol- oder Polyglykolether-Gruppen, Ester-, Ether- und Amidgruppen sowie Hydroxylgruppen enthalten sein. Beispiele für geeignete anionische Tenside sind, jeweils in Form der Natrium-, Kalium- und Ammonium- sowie der Mono-, Di- und Trialkanolammoniumsalze mit 2 oder 3 C-Atomen in der Alkanolgruppe,
- lineare Fettsäuren mit 10 bis 22 C-Atomen (Seifen),
- Ethercarbonsäuren der Formel R-O-(CH₂-CH₂O)ₓ -CH₂-COOH, in der R eine lineare Alkylgruppe mit 10 bis 22 C-Atomen und x = 0 oder 1 bis 16 ist,
- Acylsarcoside mit 10 bis 18 C-Atomen in der Acylgruppe,
- Acyltauride mit 10 bis 18 C-Atomen in der Acylgruppe,
- Acylisethionate mit 10 bis 18 C-Atomen in der Acylgruppe,
- Sulfobernsteinsäuremono- und -dialkylester mit 8 bis 18 C-Atomen in der Alkylgruppe und Sulfobernsteinsäuremono-alkylpolyoxyethylester mit 8 bis 18 C-Atomen in der Alkylgruppe und 1 bis 6 Oxyethylgruppen,
- lineare Alkansulfonate mit 12 bis 18 C-Atomen,
- lineare Alpha-Olefinsulfonate mit 12 bis 18 C-Atomen,
- Alpha-Sulfofettsäuremethylester von Fettsäuren mit 12 bis 18 C-Atomen,
- Alkylsulfate und Alkylpolyglykolethersulfate der Formel R-O(CH₂-CH₂O)ₓ-SO₃H, in der R eine bevorzugt lineare Alkylgruppe mit 10 bis 18 C-Atomen und x = 0 oder 1 bis 12 ist,
- Gemische oberflächenaktiver Hydroxysulfonate gemäß DE-A-37 25 030,
- sulfatierte Hydroxyalkylpolyethylen- und/oder Hydroxyalkylenpropylenglykolether gemäß DE-A-37 23 354,
- Sulfonate ungesättigter Fettsäuren mit 12 bis 24 C-Atomen und 1 bis 6 Doppelbindungen gemäß DE-A-39 26 344,
- Ester der Weinsäure und Zitronensäure mit Alkoholen, die Anlagerungsprodukte von etwa 2-15 Molekülen Ethylenoxid und/oder Propylenoxid an Fettalkohole mit 8 bis 22 C-Atomen darstellen.

Bevorzugte anionische Tenside sind Alkylsulfate, Alkylpolyglykolethersulfate und Ethercarbonsäuren mit 10 bis 18 C-Atomen in der Alkylgruppe und bis zu 12 Glykolethergruppen im Molekül sowie insbesondere Salze von gesättigten und insbesondere ungesättigten C₈-C₂₂-Carbonsäuren, wie Ölsäure, Stearinsäure, Isostearinsäure und Palmitinsäure.

Nichtionogene Tenside enthalten als hydrophile Gruppe z. B. eine Polyolgruppe, eine Polyalkylenglykolethergruppe oder eine Kombination aus Polyol- und Polyglykolethergruppe. Solche Verbindungen sind beispielsweise
- Anlagerungsprodukte von 2 bis 30 Mol Ethylenoxid und/oder 0 bis 5 Mol Propylenoxid an lineare Fettalkohole mit 8 bis 22 C-Atomen, an Fettsäuren mit 12 bis 22 C-Atomen und an Aikyiphenoie mit 8 bis 15 C-Atomen in der Alkylgruppe,
- C₁₂-C₂₂-Fettsäuremono- und -diester von Anlagerungsprodukten von 1 bis 30 Mol Ethylenoxid an Glycerin,
- C₈-C₂₂-Alkylmono- und -oligoglycoside und deren ethoxylierte Analoga sowie
- Anlagerungsprodukte von 5 bis 60 Mol Ethylenoxid an Rizinusöl und gehärtetes Rizinusöl.

Bevorzugte nichtionische Tenside sind Alkylpolyglykoside der allgemeinen Formel R¹O-(Z)ₓ. Diese Verbindungen sind durch die folgenden Parameter gekennzeichnet.

Der Alkylrest R¹ enthält 6 bis 22 Kohlenstoffatome und kann sowohl linear als auch verzweigt sein. Bevorzugt sind primäre lineare und in 2-Stellung methylverzweigte aliphatische Reste. Solche Alkylreste sind beispielsweise 1-Octyl, 1-Decyl, 1-Lauryl, 1-Myristyl, 1-Cetyl und 1-Stearyl. Besonders bevorzugt sind 1-Octyl, 1-Decyl, 1-Lauryl, 1-Myristyl. Bei Verwendung sogenannter "Oxo-Alkohole" als Ausgangsstoffe überwiegen Verbindungen mit einer ungeraden Anzahl von Kohlenstoffatomen in der Alkylkette.

Die erfindungsgemäß verwendbaren Alkylpolyglykoside können beispielsweise nur einen bestimmten Alkylrest R¹ enthalten. Üblicherweise werden diese Verbindungen aber ausgehend von natürlichen Fetten und Ölen oder Mineralölen hergestellt. In diesem Fall liegen als Alkylreste R Mischungen entsprechend den Ausgangsverbindungen bzw. entsprechend der jeweiligen Aufarbeitung dieser Verbindungen vor.

Besonders bevorzugt sind solche Alkylpolyglykoside, bei denen R¹
- im wesentlichen aus C₈- und C₁₀-Alkylgruppen,
- im wesentlichen aus C₁₂- und C₁₄-Alkylgruppen,
- im wesentlichen aus C₈- bis C₁₆-Alkylgruppen oder
- im wesentlichen aus C₁₂- bis C₁₆-Alkylgruppen besteht.

Als Zuckerbaustein Z können beliebige Mono- oder Oligosaccharide eingesetzt werden. Üblicherweise werden Zucker mit 5 bzw. 6 Kohlenstoffatomen sowie die entsprechenden Oligosaccharide eingesetzt. Solche Zucker sind beispielsweise Glucose, Fructose, Galactose, Arabinose, Ribose, Xylose, Lyxose, Allose, Altrose, Mannose, Gulose, Idose, Talose und Sucrose. Bevorzugte Zuckerbausteine sind Glucose, Fructose, Galactose, Arabinose und Sucrose; Glucose ist besonders bevorzugt.

Die erfindungsgemäß verwendbaren Alkylpolyglykoside enthalten im Schnitt 1,1 bis 5 Zuckereinheiten. Alkylpolyglykoside mit x-Werten von 1,1 bis 1,6 sind bevorzugt. Ganz besonders bevorzugt sind Alkylglykoside, bei denen x 1,1 bis 1,4 beträgt.

Die Alkylglykoside können neben ihrer Tensidwirkung auch dazu dienen, die Fixierung von Duftkomponenten auf dem Haar zu verbessern. Der Fachmann wird also für den Fall, dass eine über die Dauer der Haarbehandlung hinausgehende Wirkung des Parfümöles auf dem Haar gewünscht wird, bevorzugt zu dieser Substanzklasse als weiterem Inhaltsstoff der erfindungsgemäßen Zubereitungen zurückgreifen.

Auch die alkoxylierten Homologen der genannten Alkylpolyglykoside können erfindungsgemäß eingesetzt werden. Diese Homologen können durchschnittlich bis zu 10 Ethylenoxid- und/oder Propylenoxideinheiten pro Alkylglykosideinheit enthalten.

Weiterhin können, insbesondere als Co-Tenside, zwitterionische Tenside verwendet werden. Als zwitterionische Tenside werden solche oberflächenaktive Verbindungen bezeichnet, die im Molekül mindestens eine quartäre Ammoniumgruppe und mindestens eine -COO⁽⁻⁾- oder -SO₃⁽⁻⁾-Gruppe tragen. Besonders geeignete zwitterionische Tenside sind die sogenannten Betaine wie die N-Alkyl-N,N-dimethylammonium-glycinate, beispielsweise das Kokosalkyl-dimethylammonium-glycinat, N-Acyl-aminopropyl-N,N-dimethylammoniumglycinate, beispielsweise das Kokosacylaminopropyl-dimethylammoniumglycinat, und 2-Alkyl-3-carboxylmethyl-3-hydroxyethyl-imidazoline mit jeweils 8 bis 18 C-Atomen in der Alkyl- oder Acylgruppe sowie das Kokosacylaminoethylhydroxyethylcarboxymethylglycinat. Ein bevorzugtes zwitterionisches Tensid ist das unter der INCI-Bezeichnung Cocamidopropyl Betaine bekannte Fettsäureamid-Derivat.

Ebenfalls insbesondere als Co-Tenside geeignet sind ampholytische Tenside. Unter ampholytischen Tensiden werden solche oberflächenaktiven Verbindungen verstanden, die außer einer C₈-C₁₈-Alkyl- oder Acylgruppe im Molekül mindestens eine freie Aminogruppe und mindestens eine -COOH- oder -SO₃H-Gruppe enthalten und zur Ausbildung innerer Salze befähigt sind. Beispiele für geeignete ampholytische Tenside sind N-Alkylglycine, N-Alkylpropionsäuren, N-Alkylaminobuttersäuren, N-Alkyliminodipropionsäuren, N-Hydroxyethyl-N-alkylamidopropylglycine, N-Alkyltaurine, N-Alkylsarcosine, 2-Alkylaminopropionsäuren und Alkylaminoessigsäuren mit jeweils etwa 8 bis 18 C-Atomen in der Alkylgruppe. Besonders bevorzugte ampholytische Tenside sind das N-Kokosalkylaminopropionat, das Kokosacylaminoethylaminopropionat und das C₁₂₋₁₈-Acylsarcosin.

Erfindungsgemäß werden als kationische Tenside insbesondere solche vom Typ der quartären Ammoniumverbindungen, der Esterquats und der Amidoamine eingesetzt.

Bevorzugte quaternäre Ammoniumverbindungen sind Ammoniumhalogenide, insbesondere Chloride und Bromide, wie Alkyltrimethylammoniumchloride, Dialkyldimethylammoniumchloride und Trialkylmethylammoniumchloride, z. B. Cetyltrimethylammoniumchlorid, Stearyltrimethylammoniumchlorid, Distearyldimethylammoniumchlorid, Lauryldimethylammoniumchlorid, Lauryldimethylbenzylammoniumchlorid und Tricetylmethylammoniumchlorid, sowie die unter den INCI-Bezeichnungen Quaternium-27 und Quaternium-83 bekannten Imidazolium-Verbindungen. Die langen Alkylketten der oben genannten Tenside weisen bevorzugt 10 bis 18 Kohlenstoffatome auf.

Bei Esterquats handelt es sich um bekannte Stoffe, die sowohl mindestens eine Esterfunktion als auch mindestens eine quartäre Ammoniumgruppe als Strukturelement enthalten. Bevorzugte Esterquats sind quaternierte Estersalze von Fettsäuren mit Triethanolamin, quaternierte Estersalze von Fettsäuren mit Diethanolalkylaminen und quaternierten Estersalze von Fettsäuren mit 1,2-Dihydroxypropyldialkylaminen. Solche Produkte werden beispielsweise unter den Warenzeichen Stepantex^{®}, Dehyquart^{®} und Armocare^{®} vertrieben. Die Produkte Armocare^{®} VGH-70, ein N,N-Bis(2-Palmitoyloxyethyl)dimethylammoniumchlorid, sowie Dehyquart^{®} F-75 und Dehyquart^{®} AU-35 sind Beispiele für solche Esterquats.

Die Alkylamidoamine werden üblicherweise durch Amidierung natürlicher oder synthetischer Fettsäuren und Fettsäureschnitte mit Dialkylaminoaminen hergestellt. Eine erfindungsgemäß besonders geeignete Verbindung aus dieser Substanzgruppe stellt das unter der Bezeichnung Tegoamid^{®} S 18 im Handel erhältliche Stearamidopropyldimethylamin dar.

Weitere erfindungsgemäß verwendbare kationische Tenside stellen die quaternisierten Proteinhydrolysate dar.

Erfindungsgemäß ebenfalls geeignet sind kationische Silikonöle wie beispielsweise die im Handel erhältlichen Produkte Q2-7224 (Hersteller: Dow Corning; ein stabilisiertes Trimethylsilylamodimethicon), Dow Corning 929 Emulsion (enthaltend ein hydroxylamino-modifiziertes Silicon, das auch als Amodimethicone bezeichnet wird), SM-2059 (Hersteller: General Electric), SLM-55067 (Hersteller: Wacker) sowie Abil^{®}-Quat 3270 und 3272 (Hersteller: Th. Goldschmidt; diquaternäre Polydimethylsiloxane, Quaternium-80).

Ein Beispiel für ein als kationisches Tensid einsetzbares quaternäres Zuckerderivat stellt das Handelsprodukt Glucquat^{®}100 dar, gemäß INCI-Nomenklatur ein "Lauryl Methyl Gluceth-10 Hydroxypropyl Dimonium Chloride".

Bei den als Tensid eingesetzten Verbindungen mit Alkylgruppen kann es sich jeweils um einheitliche Substanzen handeln. Es ist jedoch in der Regel bevorzugt, bei der Herstellung dieser Stoffe von nativen pflanzlichen oder tierischen Rohstoffen auszugehen, so dass man Substanzgemische mit unterschiedlichen, vom jeweiligen Rohstoff abhängigen Alkylkettenlängen erhält.

Bei den Tensiden, die Anlagerungsprodukte von Ethylen- und/oder Propylenoxid an Fettalkohole oder Derivate dieser Anlagerungsprodukte darstellen, können sowohl Produkte mit einer "normalen" Homologenverteilung als auch solche mit einer eingeengten Homologenverteilung verwendet werden. Unter "normaler" Homologenverteilung werden dabei Mischungen von Homologen verstanden, die man bei der Umsetzung von Fettalkohol und Alkylenoxid unter Verwendung von Alkalimetallen, Alkalimetallhydroxiden oder Alkalimetallalkoholaten als Katalysatoren erhält. Eingeengte Homologenverteilungen werden dagegen erhalten, wenn beispielsweise Hydrotalcite, Erdalkalimetallsalze von Ethercarbonsäuren, Erdalkalimetalloxide, -hydroxide oder -alkoholate als Katalysatoren verwendet werden. Die Verwendung von Produkten mit eingeengter Homologenverteilung kann bevorzugt sein.

Ferner können die Färbemittel und die Nuanciermittel des erfindungsgemäßen Verfahrens weitere Wirk-, Hilfs- und Zusatzstoffe, wie beispielsweise
- nichtionische Polymere wie beispielsweise Vinylpyrrolidon/Vinylacrylat-Copolymere, Polyvinylpyrrolidon und Vinylpyrrolidon/Vinylacetat-Copolymere und Polysiloxane,
- kationische Polymere wie quaternisierte Celluloseether, Polysiloxane mit quaternären Gruppen, Dimethyldiallylammoniumchlorid-Polymere, Acrylamid-Dimethyldiallylammoniumchlorid-Copolymere, mit Diethylsulfat quaternierte Dimethylaminoethylmethacrylat-Vinylpyrrolidon-Copolymere, Vinylpyrrolidon-Imidazoliniummethochlorid-Copolymere und quaternierter Polyvinylalkohol,
- zwitterionische und amphotere Polymere wie beispielsweise Acrylamidopropyl-trimethylammoniumchlorid/Acrylat-Copolymere und Octylacrylamid/Methyl-methacrylat/tert-Butylaminoethylmethacrylat/2-Hydroxypropylmethacrylat-Copolymere,
- anionische Polymere wie beispielsweise Polyacrylsäuren, vernetzte Polyacrylsäuren, Vinytacetat/Crotonsäure-Copolymere, Vinylpyrrolidon/Vinylacrylat-Copolymere, Vinylacetat/Butylmaleat/Isobornylacrylat-Copolymere, Methylvinylether/Maleinsäureanhydrid-Copolymere und Acrylsäure/Ethylacrylat/N-tert.Butyl-acrylamid-Terpolymere,
- Strukturanten wie Maleinsäure und Milchsäure,
- haarkonditionierende Verbindungen wie Phospholipide, beispielsweise Sojalecithin, Ei-Lecitin und Kephaline,
- Proteinhydrolysate, insbesondere Elastin-, Kollagen-, Keratin-, Milcheiweiß-, Sojaprotein- und Weizenproteinhydrolysate, deren Kondensationsprodukte mit Fettsäuren sowie quaternisierte Proteinhydrolysate,
- Parfümöle, Dimethylisosorbid und Cyclodextrine,
- Lösungsmittel und -vermittler wie Ethanol, Isopropanol, Ethylenglykol, Propylenglykol, Glycerin und Diethylenglykol,
- faserstrukturverbessernde Wirkstoffe, insbesondere Mono-, Di- und Oligosaccharide wie beispielsweise Glucose, Galactose, Fructose, Fruchtzucker und Lactose,
- quaternierte Amine wie Methyl-1-alkylamidoethyl-2-alkylimidazolinium-methosulfat
- Entschäumer wie Silikone,
- Farbstoffe zum Anfärben des Mittels,
- Antischuppenwirkstoffe wie Piroctone Olamine, Zink Omadine und Climbazol,
- Lichtschutzmittel, insbesondere derivatisierte Benzophenone, Zimtsäure-Derivate und Triazine,
- Substanzen zur Einstellung des pH-Wertes, wie beispielsweise übliche Säuren, insbesondere Genußsäuren und Basen,
- Wirkstoffe wie Allantoin, Pyrrolidoncarbonsäuren und deren Salze sowie Bisabolol,
- Vitamine, Provitamine und Vitaminvorstufen, insbesondere solche der Gruppen A, B₃, B₅, B₆, C, E, F und H,
- Pflanzenextrakte wie die Extrakte aus Grünem Tee, Eichenrinde, Brennessel, Hamamelis, Hopfen, Kamille, Klettenwurzel, Schachtelhalm, Weißdorn, Lindenblüten, Mandel, Aloe Vera, Fichtennadel, Roßkastanie, Sandelholz, Wacholder, Kokosnuß, Mango, Aprikose, Limone, Weizen, Kiwi, Melone, Orange, Grapefruit, Salbei, Rosmarin, Birke, Malve, Wiesenschaumkraut, Quendel, Schafgarbe, Thymian, Melisse, Hauhechel, Huflattich, Eibisch, Meristem, Ginseng und Ingwerwurzel,.
- Cholesterin,
- Konsistenzgeber wie Zuckerester, Polyolester oder Polyolalkylether,
- Fette und Wachse wie Walrat, Bienenwachs, Montanwachs und Paraffine,
- Fettsäurealkanolamide,
- Komplexbildner wie EDTA, NTA, β-Alanindiessigsäure und Phosphonsäuren,
- Quell- und Penetrationsstoffe wie Glycerin, Propylenglykolmonoethylether, Carbonate, Hydrogencarbonate, Guanidine, Harnstoffe sowie primäre, sekundäre und tertiäre Phosphate,
- Trübungsmittel wie Latex, Styrol/PVP- und Styrol/Acrylamid-Copolymere
- Perlglanzmittel wie Ethylenglykolmono- und -distearat sowie PEG-3-distearat,
- Pigmente,
- Stabilisierungsmittel für Wasserstoffperoxid und andere Oxidationsmittel,
- Treibmittel wie Propan-Butan-Gemische, N₂O, Dimethylether, CO₂ und Luft,
- Antioxidantien,
enthalten.

Bezüglich weiterer fakultativer Komponenten sowie die eingesetzten Mengen dieser Komponenten wird ausdrücklich auf die dem Fachmann bekannten einschlägigen Handbücher, z. B. Kh. Schrader, Grundlagen und Rezepturen der Kosmetika, 2. Auflage, Hüthig Buch Verlag, Heidelberg, 1989, verwiesen.

Die Anwendungstemperaturen des Färbemittels können in einem Bereich zwischen 15 und 40 °C liegen. Nach einer Einwirkungszeit Z2 von bevorzugt 2 bis 60 Minuten, besonders bevorzugt von 5 bis 45 Minuten, wird das Haarfärbemittel durch Ausspülen von dem zu färbenden Haar entfernt. Das Nachwaschen mit einem Shampoo entfällt, wenn ein stark tensidhaltiger Träger, z.B. ein Färbeshampoo, verwendet wurde.

Es wird ein schärferer Kontrastübergang zwischen den nuancierten Haarbereichen und dem restlichen Haar bewirkt, wenn das Nuanciermittel auf trockenes Haar appliziert wird.

Haar ist im Sinne der Erfindung auch dann trocken, wenn es eine gewisse Restfeuchtigkeit hat, wie beispielsweise handtuchtrockenes Haar.

Die Einwirkzeit Z1 beträgt bevorzugt 5 bis 60 Minuten, besonders bevorzugt 20 bis 45 Minuten.

Ein zweiter Gegenstand der vorliegenden Anmeldung ist eine Verkaufseinheit (Kit), enthaltend
- gegebenenfalls einen Applikator,
- mindestens einen Container C1, enthaltend ein Nuanciermittel, wobei das Nuanciermittel bei mehreren Containern C1 gleich oder verschieden sein kann,
- mindestens einen rohrförmigen Körper,
wobei
- die rohrförmigen Körper als Ausgangsform die Form eines bereits vorgefertigten, an beiden Grund- und Deckfläche offenen, durch einen Mantel begrenzten Hohlzylinders besitzen, wobei der Mantel eine Naht, verlaufend über die vollständige Höhe des Hohlzylinders entlang der Mantelfläche aufweist,
- die Ausgangsform ohne äußere Krafteinwirkung beständig ist,
- die verwendeten rohrförmigen Körper sich unter äußerer Krafteinwirkung zu einer geöffneten Form entlang der Naht öffnen lassen und
- die rohrförmigen Körper aus der geöffneten Form heraus ohne äußere Krafteinwirkung selbsttätig ihre Ausgangsform annehmen.

Als Applikatoren gelten die im ersten Erfindungsgegenstand genannten Vertreter.

Mindestens ein Nuanciermittel wird statt in einem Container C1 bevorzugt als Zweikomponentenmittel in einem Containern C1a und einem Container C1b konfektioniert, wobei Container C1a eine Zusammensetzung, enthaltend in einem kosmetischen Träger mindestens eine Verbindung, ausgewählt
(a) aus Oxidationsfarbstoffvorprodukten vom Typ der Entwicklerkomponenten und gegebenenfalls der Kupplerkomponenten und/oder
(b) aus Oxofarbstoffvorprodukten und/oder
(c) aus direktziehenden Farbstoffen und/oder
(d) aus Vorstufen naturanaloger Farbstoffe und/oder
(e) aus Bleichverstärkern.
und Container C1b eine Zusammensetzung, enthaltend in einem kosmetischen Träger mindestens ein Oxidationsmittel, enthält.

Enthält das Kit mehrere Container zur Bereitstellung mehrerer Nuanciermittel des erfindungsgemäßen Verfahrens, so können diese Container mit einer entsprechenden Kennzeichnung, beispielsweise mittels eines Farbcodes oder einer Nummerierung, versehen sein. Auf diese Weise kann der Anwender die Container einem Nuanciermittel zuordnen. Die gleiche Kennzeichnung kann auch auf entsprechenden rohrförmigen Körpern angebracht werden, so dass das Kit unterschiedlich gekennzeichnete rohrförmige Körper enthält, die durch die Kennzeichnung den verschiedenen Nuanciermitteln zugeordnet werden können.

Das Färbemittel und das Nuanciermittel, auch als Mischung der Zusammensetzungen aus Container C1a und C1b, besitzen die bevorzugten Merkmale, wie sie im ersten Gegenstand der Erfindung beschrieben wurden. Als Applikatoren dienen die im ersten Gegenstand der Erfindung genannten Applikatoren.

In einer weiteren Ausführungsform kann das Kit zusätzlich mindestens einen Container C2 umfassen, der ein Färbemittel für keratinhaltige Fasern enthält.

Das Färbemittel liegt bevorzugt als Zweikomponentenmittel in zwei Containern C2a und C2b in dem Kit vor. Wenn es sich um ein oxidatives Färbemittel handelt, befindet sich in Container C2a die sogenannte Färbecreme, welche die Farbstoffvorprodukte enthält, und im Container 2b wird eine wasserstoffperoxidhaltige Zusammensetzung aufbewahrt. Wenn es sich um ein Färbemittel der Oxofärbung handelt, können die Verbindungen der Komponente A in Container C2a und die Verbindungen der Komponente B in Container C2b getrennt gelagert werden.

Gegebenenfalls kann das erfindungsgemäße Kit zusätzlich einen Container C3, enthaltend ein Konditioniermittel für keratinhaltige Fasern, umfassen.

Das Kit kann zusätzlich mindestens eine Schale enthalten, welche sich insbesondere zum Anmischen des Nuancier- bzw. Färbemittels und als Behältnis zur Aufbewahrung des entsprechenden Mittels während der Durchführung des erfindungsgemäßen Verfahrens eignet. Unter einer Schale wird erfindungsgemäß ein offenes Gefäß verstanden, welches eine Basisfläche und eine Wandung auf dieser Basisfläche, bevorzugt entlang des Randes der Basisfläche, besitzt. Die Basisfläche kann beispielsweise dreieckig, viereckig, fünfeckig, sechseckig, siebeneckig, achteckig, kreisrund oder oval sein, oder der Form der Grund- bzw. Deckfläche des rohrförmigen Körpers entsprechen. Die Wandung kann zusätzlich entlang ihrer Ausdehnung, die nicht in der Ebene der Grundfläche liegt, gerade oder geschwungen sein. Die Wandung steht bevorzugt in einem Winkel von 45° bis 135° auf der Basisfläche. Ein Winkel von 75° bis 105°, insbesondere von 90°, ist besonders bevorzugt. Die Schale kann undurchsichtig oder transparent sein. Die Schale kann ebenso ein Bestandteil der Verpackung, z.B. ein sogenanntes tray mit einem Reservoir, sein.

Das Kit kann zusätzlich eine Gebrauchanweisung enthalten, welche die Anwendung des Kits gemäß des Verfahrens des ersten Erfindungsgegenstandes vorschreibt.

Ein dritter Gegenstand der Erfindung ist die Verwendung des Kits gemäß zweitem Gegenstand der Erfindung in einem Verfahren des ersten Gegenstands der Erfindung.

## Patentansprüche

1. Verfahren zum Färben keratinhaltiger Fasern, insbesondere menschlicher Haare, in welchem
(i) aus einer Menge keratinhaltiger Fasern eine Teilmenge abgeteilt wird,
(ii) auf diese Teilmenge vollständig oder teilweise ein Nuanciermittel aufgetragen wird,
(iii) die Teilmenge in einen rohrförmigen Körper eingebracht wird,
(iv) gegebenenfalls die Schritte (i) bis (iii) unter Auswahl weiterer Teilmengen und Verwendung weiterer rohrförmiger Körper wiederholt werden,
(v) nach Ablauf einer Einwirkzeit Z1 alle rohrförmigen Körper entfernt werden und das Nuanciermittel abgespült wird,
wobei
- die rohrförmigen Körper als Ausgangsform die Form eines bereits vorgefertigten Hohlzylinders besitzen, der an Grund- und Deckfläche offen ist und durch einen Mantel begrenzt wird, wobei der Mantel eine Naht, verlaufend über die vollständige Höhe des Hohlzylinders entlang der Mantelfläche, aufweist,
- die Ausgangsform ohne äußere Krafteinwirkung beständig ist,
- die rohrförmigen Körper sich zusätzlich unter äußerer Krafteinwirkung zu einer geöffneten Form entlang der Naht öffnen lassen und
- die rohrförmigen Körper aus der geöffneten Form heraus ohne äußere Krafteinwirkung selbsttätig ihre Ausgangsform annehmen.

2. Verfahren nach Anspruch 1, **dadurch gekennzeichnet, daß** die Grundfläche und die Deckfläche der rohrförmigen Körper rund, insbesondere elliptisch oder kreisrund, sind.

3. Verfahren nach einem der Ansprüche 1 oder 2, **dadurch gekennzeichnet, daß** die rohrförmigen Körper aus einem planaren Material durch Aufrollen gebildet werden.

4. Verfahren nach einem der Ansprüche 1 bis 3, **dadurch gekennzeichnet, daß** die rohrförmigen Körper aus mindestens einem Kunststoff bestehen oder damit beschichtet sind.

5. Verfahren nach einem der Ansprüche 1 bis 4, **dadurch gekennzeichnet, daß** die rohrförmigen Körper an der Naht verlaufend über die vollständige Höhe des Hohlzylinders entlang der Mantelfläche mindestens einen Verschluß besitzen.

6. Verfahren nach einem der Ansprüche 1 bis 5, **dadurch gekennzeichnet, daß** das Nuanciermittel in einem kosmetischen Träger mindestens eine Verbindung enthält, die ausgewählt wird,
(a) aus Oxidationsfarbstoffvorprodukten vom Typ der Entwicklerkomponenten und gegebenenfalls der Kupplerkomponenten und/oder
(b) aus Oxofarbstoffvorprodukten und/oder
(c) aus direktziehenden Farbstoffen und/oder
(d) aus Vorstufen naturanaloger Farbstoffe und/oder
(e) aus Oxidationsmitteln und gegebenenfalls zusätzlichen Bleichverstärkern.

7. Verfahren nach Anspruch 6, **dadurch gekennzeichnet, daß** die Entwicklerkomponenten ausgewählt werden, aus der Liste, die gebildet wird aus p-Aminophenol, N-Methyl-p-aminophenol, 4-Amino-3-methyl-phenol, 4-Amino-3-fluorphenol, 2-Hydroxymethylamino-4-aminophenol, 4-Amino-3-hydroxymethylphenol, 4-Amino-2-(2-hydroxyethoxy)-phenol, 4-Amino-2-methylphenol, 4-Amino-2-hydroxymethylphenol, 4-Amino-2-methoxymethyl-phenol, 4-Amino-2-aminomethylphenot, 4-Amino-2-(β-hydroxyethyl-aminomethyl)-phenol, 4-Amino-2-(α,β-dihydroxyethyl)-phenol, 4-Amino-2-fluorphenol, 4-Amino-2-chlorphenol, 4-Amino-2,6-dichlorphenol, 4-Amino-2-(diethyl-aminomethyl)-phenol, N,N'-Bis-(β-hydroxyethyl)-N,N'-bis-(4'-aminophenyl)-1,3-diamino-propan-2-ol, N,N'-Bis-(β-hydroxyethyl)-N,N'-bis-(4'-aminophenyl)-ethylendiamin, N, N'-Bis-(4-aminophenyl)-tetramethylendiamin, N,N'-Bis-(β-hydroxyethyl)-N,N'-bis-(4-aminophenyl)-tetramethylendiamin, N,N'-Bis-(4-methyl-aminophenyl)-tetramethylend iamin, N, N'-Diethyl-N, N'-bis-(4'-amino-3'-methylphenyl)-ethylendiamin, Bis-(2-hydroxy-5-aminophenyl)-methan, 1,3-Bis-(2,5-diaminophenoxy)-propan-2-ol, N,N'-Bis-(4'-aminophenyl)-1,4-diazacycloheptan, N, N'-Bis-(2-hydroxy-5-aminobenzyl)-piperazin, N-(4'-Aminophenyl)-p-phenylendiamin und 1,10-Bis-(2',5'-diaminophenyl)-1,4,7,10-tetraoxadecan, p-Phenylendiamin, p-Toluylendiamin, 2-Chlor-p-phenylendiamin, 2,3-Dimethyl-p-phenylendiamin, 2,6-Dimethyl-p-phenylendiamin, 2,6-Diethyl-p-phenylendiamin, 2,5-Dimethyl-p-phenylendiamin, N,N-Dimethyl-p-phenylendiamin, N,N-Diethyl-p-phenylendiamin, N,N-Dipropyl-p-phenylendiamin, 4-Amino-3-methyl-(N,N-diethyl)-anilin, N,N-Bis-(β-hydroxyethyl)-p-phenylendiamin, 4-N,N-Bis-(β-hydroxyethyl)amino-2-methylanilin, 4-N,N-Bis-(β-hydroxyethyl)amino-2-chloranilin, 2-(β-Hydroxyethyl)-p-phenylendiamin, 2-(α,β-Dihydroxyethyl)-p-phenylendiamin, 2-Fluor-p-phenylendiamin, 2-Isopropyl-p-phenylendiamin, N-(β-Hydroxypropyl)-p-phenylendiamin, 2-Hydroxymethyl-p-phenylendiamin, N,N-Dimethyl-3-methyl-p-phenylendiamin, N,N-(Ethyl,β-hydroxyethyl)-p-phenylendiamin, N-(β,γ-Dihydroxypropyl)-p-phenylendiamin, N-(4'-Aminophenyl)-p-phenylendiamin, N-Phenyl-p-phenylendiamin, 2-(β-Hydroxyethyloxy)-p-phenylendiamin, 2-(β-Acetylaminoethyloxy)-p-phenylendiamin, N-(β-Methoxyethyl)-p-phenylendiamin und 5,8-Diaminobenzo-1,4-dioxan, 4,5-Diamino-1-methylpyrazol, 4,5-Diamino-1-(β-hydroxyethyl)-pyrazol, 3,4-Diaminopyrazol, 4,5-Diamino-1-(4'-chlorbenzyl)-pyrazol, 4,5-Diamino-1,3-dimethylpyrazol, 4,5-Diamino-3-methyl-1-phenylpyrazol, 4,5-Diamino-1-methyl-3-phenylpyrazol, 4-Amino-1,3-dimethyl-5-hydrazinopyrazol, 1-Benzyl-4,5-diamino-3-methylpyrazol, 4,5-Diamino-3-tert.-butyl-1-methylpyrazol, 4,5-Diamino-1-tert.-butyl-3-methylpyrazol, 4,5-Diamino-1-(β-hydroxyethyl)-3-methylpyrazol, 4,5-Diamino-1-ethyl-3-methylpyrazol, 4,5-Diamino-1-ethyl-3-(4'-methoxyphenyl)-pyrazol, 4,5-Diamino-1-ethyl-3-hydroxymethylpyrazol, 4,5-Diamino-3-hydroxymethyl-1-methylpyrazol, 4,5-Diamino-3-hydroxymethyl-1-isopropylpyrazol, 4,5-Diamino-3-methyl-1-isopropylpyrazol, 4-Amino-5-(2-aminoethyl)amino-1,3-dimethylpyrazol, 3,4,5-Triaminopyrazol, 1-Methyl-3,4,5-triaminopyrazol, 3,5-Diamino-1-methyl-4-methylaminopyrazol, 3,5-Diamino-4-(β-hydroxyethyl)amino-1-methylpyrazol, 2,4,5,6-Tetraaminopyrimidin, 4-Hydroxy-2,5,6-triaminopyrimidin, 2-Hydroxy-4,5,6-triaminopyrimidin, 2-Dimethylamino-4,5,6-triaminopyrimidin, 2,4-Dihydroxy-5,6-diaminopyrimidin, 2,5,6-Triaminopyrimidin, 2,5-Diamino-pyridin, 2-(4'-Methoxyphenyl)amino-3-amino-pyridin, 2,3-Diamino-6-methoxy-pyridin, 2-(β-Methoxyethyl)amino-3-amino-6-methoxy-pyridin und 3,4-Diamino-pyridin und deren physiologisch verträglichen Salze.

8. Verfahren nach einem der Ansprüche 6 oder 7, **dadurch gekennzeichnet, daß** die Kupplerkomponenten ausgewählt werden aus der Liste, die gebildet wird aus 1-Naphthol, 1,5-Dihydroxynaphthalin, 2,7-Dihydroxynaphthalin, 1,7-Dihydroxynaphthalin, 5-Amino-2-methylphenol, m-Aminophenol, Resorcin, m-Phenylendiamin, 1-Phenyl-3-methyl-pyrazol-5-on, 2,4-Dichlor-3-aminophenol, 1,3-Bis-(2,4-diaminophenoxy)-propan, 2-Chlor-resorcin, 4-Chlor-resorcin, 2-Chlor-6-methyl-3-aminophenol, 3-Amino-2-methylamino-6-methoxypyridin, 2-Amino-3-hydroxypyridin, 2,6-Dihydroxy-3,4-dimethylpyridin, 2-Methylresorcin, 5-Methylresorcin, 2-Methyl-4-chlor-5-aminophenol und den physiologisch verträglichen Salzen der vorgenannten Verbindungen.

9. Verfahren nach einem der Ansprüche 6 bis 8, **dadurch gekennzeichnet, daß** die Oxofarbstoffvorprodukte ausgewählt werden aus mindestens einer Kombination, ausgewählt aus mindestens einer Verbindung der Komponente
A Verbindungen, die eine reaktive Carbonylgruppe enthalten mit mindestens einer Verbindung der Komponente
B Verbindungen, ausgewählt aus (a) CH-aciden Verbindungen, (b) Verbindungen mit primärer oder sekundärer Aminogruppe oder Hydroxygruppe, ausgewählt aus primären oder sekundären aromatischen Aminen, stickstoffhaltigen heterozyklischen Verbindungen und aromatischen Hydroxyverbindungen, (c) Aminosäuren und/oder (d) aus 2 bis 9 Aminosäuren aufgebauten Oligopeptiden.

10. Verfahren nach einem der Ansprüche 7 bis 9, **dadurch gekennzeichnet, daß** das Oxidationsmittel Wasserstoffperoxid und/oder mindestens ein Anlagerungsprodukt davon ist.

11. Verfahren nach einem der Ansprüche 7 bis 10, **dadurch gekennzeichnet, daß** der Bleichverstärker ausgewählt wird aus festen Peroxoverbindungen.

12. Verfahren nach einem der Ansprüche 1 bis 11, **dadurch gekennzeichnet, daß** das Nuanciermittel ein Bleichmittel für keratinhaltige Fasern ist.

13. Verfahren nach einem der Ansprüche 1 bis 12, **dadurch gekennzeichnet, daß** die keratinhaltigen Fasern vor Schritt (i) komplett einer Färbung mit einem Färbemittel unterworfen werden.

14. Verfahren nach Anspruch 12, **dadurch gekennzeichnet, daß** die keratinhaltigen Fasern nach dem Schritt (v) komplett einer Färbung mit einem Färbemittel unterworfen werden.

15. Verfahren nach einem der Ansprüche 13 oder 14, **dadurch gekennzeichnet, daß** das Färbemittel in einem kosmetischen Träger mindestens eine Verbindung enthält, ausgewählt
(a) aus Oxidationsfarbstoffvorprodukten vom Typ der Entwicklerkomponenten und gegebenenfalls der Kupplerkomponenten und/oder
(b) aus reaktiven Carbonylverbindungen und/oder
(c) aus direktziehenden Farbstoffen und/oder
(d) aus Vorstufen naturanaloger Farbstoffe und/oder
(e) aus Oxidationsmitteln.

16. Kit enthaltend
• gegebenenfalls einen Applikator,
• mindestens einen Container C1, enthaltend ein Nuanciermittel, wobei das Nuanciermittel bei mehreren Containern C1 gleich oder verschieden sein kann,
• mindestens einen rohrförmigen Körper,
wobei
- die rohrförmigen Körper als Ausgangsform die Form eines bereits vorgefertigten, an beiden Grund- und Deckfläche offenen, durch einen Mantel begrenzten Hohlzylinders besitzen, wobei der Mantel eine Naht, verlaufend über die vollständige Höhe des Hohlzylinders entlang der Mantelfläche aufweist,
- die Ausgangsform ohne äußere Krafteinwirkung beständig ist,
- die verwendeten rohrförmigen Körper sich unter äußerer Krafteinwirkung zu einer geöffneten Form entlang der Naht öffnen lassen und
- die rohrförmigen Körper aus der geöffneten Form heraus ohne äußere Krafteinwirkung selbsttätig ihre Ausgangsform annehmen.

17. Kit nach Anspruch 16, **dadurch gekennzeichnet, daß** es zusätzlich mindestens einen Container C2, enthaltend ein Färbemittel für keratinhaltige Fasern umfasst.

18. Kit nach einem der Ansprüche 16 oder 17, **dadurch gekennzeichnet, daß** es statt Container C1 zur Konfektionierung mindestens eines Nuanciermittels als Zweikomponentenmittel einem Containern C1a und einem Container C1b umfasst, wobei Container C1a eine Zusammensetzung, enthaltend in einem kosmetischen Träger mindestens eine Verbindung, ausgewählt
(a) aus Oxidationsfarbstoffvorprodukten vom Typ der Entwicklerkomponenten und gegebenenfalls der Kupplerkomponenten und/oder
(b) aus reaktiven Carbonylverbindungen und/oder
(c) aus direktziehenden Farbstoffen und/oder
(d) aus Vorstufen naturanaloger Farbstoffe und/oder
(e) aus Bleichverstärkern.
und Container C1 b eine Zusammensetzung, enthaltend in einem kosmetischen Träger mindestens ein Oxidationsmittel, enthält.

19. Verwendung des Kits nach einem der Ansprüche 16 oder 17 in einem Verfahren zum Färben keratinhaltiger Fasern gemäß einem der Ansprüche 1 bis 15.

## Claims

1. Method of dyeing keratin-containing fibres, in particular human hair, in which
(i) a part amount is divided off from an amount of keratin-containing fibres,
(ii) a nuancing agent is applied to all or part of this part amount,
(iii) the part amount is introduced into a tubular body,
(iv) optionally steps (i) to (iii) are repeated with selection of further part amounts and use of further tubular bodies,
(v) after expiry of a contact time T1 all of the tubular bodies are removed and the nuancing agent is rinsed off,
where
- the tubular bodies have, as starting shape, the shape of an already preformed hollow cylinder which is open at the base surface and top surface and is limited by a jacket, where the jacket has a seam running along the complete height of the hollow cylinder along the jacket surface,
- the starting shape is stable without the influence of external force,
- the tubular bodies can additionally be opened along the seam to give an opened form under the influence of external force and
- the tubular bodies automatically adopt their starting shape from the opened form without the influence of external force.

2. Method according to Claim 1, **characterized in that** the base surface and the top surface of the tubular bodies are round, in particular elliptic or circular.

3. Method according to one of Claims 1 or 2, **characterized in that** the tubular bodies are formed from a planar material by rolling up.

4. Method according to one of Claims 1 to 3, **characterized in that** the tubular bodies consist of at least one plastic or are coated therewith.

5. Method according to one of Claims 1 to 4, **characterized in that** the tubular bodies have at least one closure on the seam running the entire height of the hollow cylinder along the jacket surface.

6. Method according to one of Claims 1 to 5, **characterized in that** the nuancing agent comprises, in a cosmetic carrier, at least one compound which is selected
(a) from oxidation dye precursors of the developer component type and optionally of the coupler component type and/or
(b) from oxo dye precursors and/or
(c) from direct dyes and/or
(d) from precursors of nature-analogous dyes and/or
(e) from oxidizing agents and optionally additional bleach boosters.

7. Method according to Claim 6, **characterized in that** the developer components are selected from the list formed from p-aminophenol, N-methyl-p-aminophenol, 4-amino-3-methylphenol, 4-amino-3-fluorophenol, 2-hydroxymethylamino-4-aminophenol, 4-amino-3-hydroxymethylphenol, 4-amino-2-(2-hydroxyethoxy)phenol, 4-amino-2-methylphenol, 4-amino-2-hydroxymethylphenol, 4-amino-2-methoxymethylphenol, 4-amino-2-aminomethylphenol, 4-amino-2-(β-hydroxyethylaminomethyl)phenol, 4-amino-2-(α,β-dihydroxyethyl)phenol, 4-amino-2-fluorophenol, 4-amino-2-chlorophenol, 4-amino-2,6-dichlorophenol, 4-amino-2-(diethylaminomethyl)-phenol, N,N'-bis(β-hydroxyethyl)-N,N,'-bis(4'-aminophenyl)-1,3-diaminopropan-2-ol, N,N'-bis(β-hydroxyethyl)-N,N'-bis(4'-aminophenyl)ethylenediamine, N,N'-bis(4-aminophenyl)tetramethylenediamine, N,N'-bis(β-hydroxyethyl)-N,N'-bis(4-aminophenyl)tetramethylenediamine, N,N'-bis(4-methylaminophenyl)tetramethylenediamine, N,N'-diethyl-N,N'-bis(4'-amino-3'-methylphenyl)ethylenediamine, bis(2-hydroxy-5-aminophenyl)methane, 1,3-bis(2,5-diaminophenoxy)propan-2-ol, N,N'-bis-(4'-aminophenyl)-1,4-diazacycloheptane, N,N'-bis-(2-hydroxy-5-aminobenzyl)piperazine, N-(4'-aminophenyl)-p-phenylenediamine and 1,10-bis(2',5'-diaminophenyl)-1,4,7,10-tetraoxadecane, p-phenylenediamine, p-tolylenediamine, 2-chloro-p-phenylenediamine, 2,3-dimethyl-p-phenylenediamine, 2,6-dimethyl-p-phenylenediamine, 2,6-diethyl-p-phenylenediamine, 2,5-dimethyl-p-phenylenediamine, N,N-dimethyl-p-phenylenediamine, N,N-diethyl-p-phenylenediamine, N,N-dipropyl-p-phenylenediamine, 4-amino-3-methyl-(N,N-diethyl)aniline, N,N-bis(β-hydroxyethyl)-p-phenylenediamine, 4-N,N-bis(β-hydroxyethyl)amino-2-methylaniline, 4-N,N-bis(β-hydroxyethyl)amino-2-chloroaniline, 2-(β-hydroxyethyl)-p-phenylenediamine, 2-(α,β-dihydroxyethyl)-p-phenylenediamine, 2-fluoro-p-phenylenediamine, 2-isopropyl-p-phenylenediamine, N-(β-hydroxypropyl)-p-phenylenediamine, 2-hydroxymethyl-p-phenylenediamine, N,N-dimethyl-3-methyl-p-phenylenediamine, N,N-(ethyl,β-hydroxyethyl)-p-phenylenediamine, N-(β,γ-dihydroxypropyl)-p-phenylenediamine, N-(4'-aminophenyl)-p-phenylenediamine, N-phenyl-p-phenylenediamine, 2-(β-hydroxyethyloxy)-p-phenylenediamine, 2-(β-acetylaminoethyloxy)-p-phenylenediamine, N-(β-methoxyethyl)-p-phenylenediamine and 5,8-diaminobenzo-1,4-dioxane, 4,5-diamino-1-methylpyrazole, 4,5-diamino-1-(β-hydroxyethyl)pyrazole, 3,4-diaminopyrazole, 4,5-diamino-1-(4'-chlorobenzyl)-pyrazole, 4,5-diamino-1,3-dimethylpyrazole, 4,5-diamino-3-methyl-1-phenylpyrazole, 4,5-diamino-l-methyl-3-phenylpyrazole, 4-amino-1,3-dimethyl-5-hydrazinopyrazole, 1-benzyl-4,5-diamino-3-methylpyrazole, 4,5-diamino-3-tert-butyl-1-methylpyrazole, 4,5-diamino-1-tert-butyl-3-methylpyrazole, 4,5-diamino-1-(β-hydroxyethyl)-3-methylpyrazole, 4,5-diamino-1-ethyl-3-methylpyrazole, 4,5-diamino-1-ethyl-3-(4'-methoxyphenyl)pyrazole, 4,5-diamino-1-ethyl-3-hydroxymethylpyrazole, 4,5-diamino-3-hydroxymethyl-1-methylpyrazole, 4,5-diamino-3-hydroxymethyl-1-isopropylpyrazole, 4,5-diamino-3-methyl-1-isopropylpyrazole, 4-amino-5-(2-aminoethyl)amino-1,3-dimethylpyrazole, 3,4,5-triaminopyrazole, 1-methyl-3,4,5-triaminopyrazole, 3,5-diamino-1-methyl-4-methylaminopyrazole, 3,5-diamino-4-(β-hydroxyethyl)amino-1-methylpyrazole, 2,4,5,6-tetraaminopyrimidine, 4-hydroxy-2,5,6-triaminopyrimidine, 2-hydroxy-4,5,6-triaminopyrimidine, 2-dimethylamino-4,5,6-triaminopyrimidine, 2,4-dihydroxy-5,6-diaminopyrimidine, 2,5,6-triaminopyrimidine, 2,5-diaminopyridine, 2-(4'-methoxyphenyl)amino-3-aminopyridine, 2,3-diamino-6-methoxypyridine, 2-(β-methoxyethyl)amino-3-amino-6-methoxypyridine and 3,4-diaminopyridine and physiologically compatible salts thereof.

8. Method according to one of Claims 6 or 7, **characterized in that** the coupler components are selected from the list formed from 1-naphthol, 1,5-dihydroxynaphthalene, 2,7-dihydroxynaphthalene, 1,7-dihydroxynaphthalene, 5-amino-2-methylphenol, m-aminophenol, resorcinol, m-phenylenediamine, 1-phenyl-3-methylpyrazol-5-one, 2,4-dichloro-3-aminophenol, 1,3-bis(2,4-diaminophenoxy)propane, 2-chlororesorcinol, 4-chlororesorcinol, 2-chloro-6-methyl-3-aminophenol, 3-amino-2-methylamino-6-methoxypyridine, 2-amino-3-hydroxypyridine, 2,6-dihydroxy-3,4-dimethylpyridine, 2-methylresorcinol, 5-methylresorcinol, 2-methyl-4-chloro-5-aminophenol and the physiologically compatible salts of the abovementioned compounds.

9. Method according to one of Claims 6 to 8, **characterized in that** the oxo dye precursors are selected from at least one combination selected from at least one compound of component
A compounds which contain a reactive carbonyl group with at least one compound of component
B compounds selected from (a) CH-acidic compounds, (b) compounds with a primary or secondary amino group or hydroxy group, selected from primary or secondary aromatic amines, nitrogen-containing heterocyclic compounds and aromatic hydroxy compounds, (c) amino acids and/or (d) oligopeptides constructed from 2 to 9 amino acids.

10. Method according to one of Claims 7 to 9, **characterized in that** the oxidizing agent is hydrogen peroxide and/or at least one addition product thereof.

11. Method according to one of Claims 7 to 10, **characterized in that** the bleach booster is selected from solid peroxo compounds.

12. Method according to one of Claims 1 to 11, **characterized in that** the nuancing agent is a bleach for keratin-containing fibres.

13. Method according to one of Claims 1 to 12, **characterized in that** the keratin-containing fibres are completely subjected to a dyeing with a colourant before step (i).

14. Method according to Claim 12, **characterized in that** the keratin-containing fibres are completely subjected to a dyeing with a colourant after step (v).

15. Method according to one of Claims 13 or 14, **characterized in that** the colourant comprises, in a cosmetic carrier, at least one compound selected
(a) from oxidation dye precursors of the developer component type and optionally of the coupler component type and/or
(b) from reactive carbonyl compounds and/or
(c) from direct dyes and/or
(d) from precursors of nature-analogous dyes and/or
(e) from oxidizing agents.

16. Kit comprising
• optionally an applicator,
• at least one container C1, comprising a nuancing agent, where the nuancing agent may be identical or different in the case of a plurality of containers C1,
• at least one tubular body,
where
- the tubular bodies have, as starting shape, the shape of an already preformed hollow cylinder open at both base surface and top surface and limited by a jacket, where the jacket has a seam running the entire height of the hollow cylinder along the jacket surface,
- the starting shape is stable without the influence of external force,
- the tubular bodies used can be opened along the seam under the influence of external force to give an opened form and
- the tubular bodies automatically adopt their starting shape from the opened form without the influence of external force.

17. Kit according to Claim 16, **characterized in that** it additionally includes at least one container C2 containing a colourant for keratin-containing fibres.

18. Kit according to one of Claims 16 or 17, **characterized in that** instead of container C1 for the formulation of at least one nuancing agent, it comprises, as two-component means, a container C1a and a container Clb, where container C1a comprises a composition comprising, in a cosmetic carrier, at least one compound selected
(a) from oxidation dye precursors of the developer component type and optionally of the coupler component type and/or
(b) from reactive carbonyl compounds and/or
(c) from direct dyes and/or
(d) from precursors of nature-analogous dyes and/or
(e) from bleach boosters
and container C1b comprises a composition comprising at least one oxidizing agent in a cosmetic carrier.

19. Use of the kit according to one of Claims 16 or 17 in a method for dyeing keratin-containing fibres according to one of Claims 1 to 15.

## Revendications

1. Procédé pour la teinture de fibres kératiniques, en particulier de cheveux humains, dans lequel
(i) on sépare une quantité partielle d'une quantité de fibres kératiniques,
(ii) on applique un agent formant une nuance totalement ou partiellement sur cette quantité partielle,
(iii) on introduit la quantité partielle dans un corps en forme de tube,
(iv) on répète le cas échéant les étapes (i) à (iii) en choisissant d'autres quantités partielles et en utilisant d'autres corps en forme de tube,
(v) on enlève tous les corps en forme de tube après un temps d'action Z1 et on élimine l'agent formant une nuance par rinçage,
où
- les corps en forme de tube présentent, comme forme de départ, la forme d'un cylindre creux déjà réalisé au préalable, qui est ouvert au niveau de la surface de base et supérieure et qui est délimité par une enveloppe, l'enveloppe présentant un joint, s'étendant sur toute la hauteur du cylindre creux le long de la surface de l'enveloppe,
- la forme de départ étant stable, sans influence d'une force extérieure,
- les corps en forme de tube pouvant en outre être ouverts sous l'influence d'une force externe en une forme ouverte le long du joint et
- les corps en forme de tube reprenant, à partir de la forme ouverte, sans l'influence d'une force extérieure, automatiquement, leur forme de départ.

2. Procédé selon la revendication 1, **caractérisé en ce que** la surface de base et la surface supérieure du corps en forme de tube sont rondes, en particulier elliptiques ou circulaires.

3. Procédé selon l'une quelconque des revendications 1 ou 2, **caractérisé en ce que** les corps en forme de tube sont réalisés à partir d'un matériau plan par enroulement.

4. Procédé selon l'une quelconque des revendications 1 à 3, **caractérisé en ce que** les corps en forme de tube sont constitués par au moins un matériau synthétique ou revêtus par celui-ci.

5. Procédé selon l'une quelconque des revendications 1 à 4, **caractérisé en ce que** les corps en forme de tube présentent, au niveau du joint s'étendant sur toute la hauteur du cylindre creux, le long de la surface de l'enveloppe, au moins une fermeture.

6. Procédé selon l'une quelconque des revendications 1 à 5, **caractérisé en ce que** l'agent formant une nuance contient, dans un support cosmétique, au moins un composé qui est choisi parmi
(a) les précurseurs de colorants par oxydation du type des composants de développement et le cas échéant des composants de couplage et/ou
(b) les précurseurs de colorants de type oxo et/ou
(c) les colorants montant directement sur la fibre et/ou
(d) les précurseurs de colorants analogues aux colorants naturels et/ou
(e) les oxydants et le cas échéant des agents de renforcement supplémentaires de décoloration.

7. Procédé selon la revendication 6, **caractérisé en ce que** les composants de développement sont choisis dans la liste formée par le p-aminophénol, le N-méthyl-p-aminophénol, le 4-amino-3-méthylphénol, le 4-amino-3-fluorophénol, le 2-hydroxyméthylamino-4-aminophénol, le 4-amino-3-hydroxyméthylphénol, le 4-amino-2-(2-hydroxyéthoxy)-phénol, le 4-amino-2-méthylphénol, le 4-amino-2-hydroxyméthylphénol, le 4-amino-2-méthoxyméthylphénol, le 4-amino-2-aminométhylphénol, le 4-amino-2-(β-hydroxyéthylaminométhyl)-phénol, le 4-amino-2-(α,β-dihydroxyéthyl)-phénol, le 4-amino-2-fluorophénol, le 4-amino-2-chlorophénol, le 4-amino-2,6-dichlorophénol, le 4-amino-2-(diéthylaminométhyl)-phénol, le N,N'-bis-(β-hydroxyéthyl)-N,N'-bis-(4'-aminophényl)-1,3-diaminopropan-2-ol, la N,N'-bis-(β-hydroxyéthyl)-N,N'-bis-(4'-aminophényl)-éthylènediamine, la N,N'-bis-(4-aminophényl)-tétraméthylènediamine, la N,N'-bis-(β-hydroxyéthyl)-N,N'-bis-(4-aminophényl)-tétraméthylènediamine, la N,N'-bis-(4-méthylaminophényl)-tétraméthylènediamine, la N,N'-diéthyl-N,N'-bis-(4'-amino-3'-méthylphényl)-éthylènediamine, le bis-(2-hydroxy-5-aminophényl)-méthane, le 1,3-bis-(2,5-diaminophénoxy)-propan-2-ol, le N,N'-bis-(4'-aminophényl)-1,4-diazacycloheptane, la N,N'-bis-(2-hydroxy-5-aminobenzyl)-pipérazine, la N-(4'-aminophényl)-p-phénylènediamine et le 1,10-bis-(2',5'-diaminophényl)-1,4,7,10-tétraoxadécane, la p-phénylènediamine, la p-toluylènediamine, la 2-chloro-p-phénylènediamine, la 2,3-diméthyl-p-phénylènediamine, la 2,6-diméthyl-p-phénylènediamine, la 2,6-diéthyl-p-phénylènediamine, la 2,5-diméthyl-p-phénylènediamine, la N,N-diméthyl-p-phénylènediamine, la N,N-diéthyl-p-phénylènediamine, la N,N-dipropyl-p-phénylènediamine, la 4-amino-3-méthyl-(N,N-diéthyl)-aniline, la N,N-bis-(β-hydroxyéthyl)-p-phénylènediamine, la 4-N,N-bis-(β-hydroxyéthyl)amino-2-méthylaniline, la 4-N,N-bis-(β-hydroxyéthyl)amino-2-chloroaniline, la 2-(β-hydroxyéthyl)-p-phénylènediamine, la 2-(α,β-dihydroxyéthyl)-p-phénylènediamine, la 2-fluoro-p-phénylènediamine, la 2-isopropyl-p-phénylènediamine, la N-(β-hydroxypropyl)-p-phénylènediamine, la 2-hydroxyméthyl-p-phénylènediamine, la N,N-diméthyl-3-méthyl-p-phénylènediamine, la N,N-(éthyl,β-hydroxyéthyl)-p-phénylènediamine, la N-(β,γ-dihydroxypropyl)-p-phénylènediamine, la N-(4'-aminophényl)-p-phénylènediamine, la N-phényl-p-phénylènediamine, la 2-(β-hydroxyéthyloxy)-p-phénylènediamine, la 2-(β-acétylaminoéthyloxy)-p-phénylènediamine, la N-(β-méthoxyéthyl)-p-phénylènediamine et le 5,8-diaminobenzo-1,4-dioxane, le 4,5-diamino-1-méthylpyrazole, le 4,5-diamino-1-(β-hydroxyéthyl)-pyrazole, le 3,4-diaminopyrazole, le 4,5-diamino-1-(4'-chlorobenzyl)-pyrazole, le 4,5-diamino-1,3-diméthylpyrazole, le 4,5-diamino-3-méthyl-1-phénylpyrazole, le 4,5-diamino-1-méthyl-3-phénylpyrazole, le 4-amino-1,3-diméthyl-5-hydrazinopyrazole, le 1-benzyl-4,5-diamino-3-méthylpyrazole, le 4,5-diamino-3-tert-butyl-1-méthylpyrazole, le 4,5-diamino-1-tert-butyl-3-méthylpyrazole, le 4,5-diamino-1-(β-hydroxyéthyl)-3-méthylpyrazole, le 4,5-diamino-1-éthyl-3-méthylpyrazole, le 4,5-diamino-1-éthyl-3-(4'-méthoxyphényl)-pyrazole, le 4,5-diamino-1-éthyl-3-hydroxyméthylpyrazole, le 4,5-diamino-3-hydroxyméthyl-1-méthylpyrazole, le 4,5-diamino-3-hydroxyméthyl-1-isopropylpyrazole, le 4,5-diamino-3-méthyl-1-isopropylpyrazole, le 4-amino-5-(2-aminoéthyl)amino-1,3-diméthylpyrazole, le 3,4,5-triaminopyrazole, le 1-méthyl-3,4,5-triaminopyrazole, le 3,5-diamino-1-méthyl-4-méthylaminopyrazole, le 3,5-diamino-4-(β-hydroxyéthyl)amino-1-méthylpyrazole, la 2,4,5,6-tétraaminopyrimidine, la 4-hydroxy-2,5,6-triaminopyrimidine, la 2-hydroxy-4,5,6-triaminopyrimidine, la 2-diméthylamino-4,5,6-triaminopyrimidine, la 2,4-dihydroxy-5,6-diaminopyrimidine, la 2,5,6-triaminopyrimidine, la 2,5-diaminopyridine, la 2-(4'-méthoxyphényl)amino-3-aminopyridine, la 2,3-diamino-6-méthoxypyridine, la 2-(β-méthoxyéthyl)amino-3-amino-6-méthoxypyridine et la 3,4-diaminopyridine et leurs sels physiologiquement acceptables.

8. Procédé selon l'une quelconque des revendications 6 ou 7, **caractérisé en ce que** les composants de couplage sont choisis dans la liste formée par le 1-naphtol, le 1,5-dihydroxynaphtalène, le 2,7-dihydroxynaphtalène, le 1,7-dihydroxynaphtalène, le 5-amino-2-méthylphénol, le m-aminophénol, le résorcinol, la m-phénylènediamine, la 1-phényl-3-méthylpyrazol-5-one, le 2,4-dichloro-3-aminophénol, le 1,3-bis-(2,4-diaminophénoxy)-propane, le 2-chlororésorcinol, le 4-chlororésorcinol, le 2-chloro-6-méthyl-3-aminophénol, la 3-amino-2-méthylamino-6-méthoxypyridine, la 2-amino-3-hydroxypyridine, la 2,6-dihydroxy-3,4-diméthylpyridine, le 2-méthylrésorcinol, le 5-méthylrésorcinol, le 2-méthyl-4-chloro-5-aminophénol et les sels physiologiquement acceptables des composés susmentionnés.

9. Procédé selon l'une quelconque des revendications 6 à 8, **caractérisé en ce que** les précurseurs des colorants oxo sont choisis parmi au moins une combinaison choisie parmi au moins un composé du composant
A des composés qui contiennent un groupe carbonyle réactif avec au moins un composé du composant
B des composés choisis parmi (a) les composés CH-acides, (b) les composés avec un groupe amino primaire ou secondaire ou un groupe hydroxy, choisis parmi les amines primaires ou secondaires, les composés hétérocycliques contenant de l'azote et les composés hydroxy aromatiques, (c) les acides aminés et/ou (d) les oligopeptides élaborés à partir de 2 à 9 acides aminés.

10. Procédé selon l'une quelconque des revendications 7 à 9, **caractérisé en ce que** l'oxydant est le peroxyde d'hydrogène et/ou au moins un produit d'addition de celui-ci.

11. Procédé selon l'une quelconque des revendications 7 à 10, **caractérisé en ce que** l'agent de renforcement de décoloration est choisi parmi les composés peroxo solides.

12. Procédé selon l'une quelconque des revendications 1 à 11, **caractérisé en ce que** l'agent formant une nuance est un agent de blanchiment pour des fibres kératiniques.

13. Procédé selon l'une quelconque des revendications 1 à 12, **caractérisé en ce que** les fibres kératiniques sont soumises avant l'étape (i) complètement à une teinture avec un agent de teinture.

14. Procédé selon la revendication 12, **caractérisé en ce que** les fibres kératiniques sont soumises après l'étape (v) complètement à une teinture avec un agent de teinture.

15. Procédé selon l'une quelconque des revendications 13 ou 14, **caractérisé en ce que** l'agent de teinture contient, dans un support cosmétique, au moins un composé qui est choisi parmi
(a) les précurseurs de colorants par oxydation du type des composants de développement et le cas échéant des composants de couplage et/ou
(b) les composés carbonyle réactifs et/ou
(c) les colorants montant directement sur la fibre et/ou
(d) les précurseurs de colorants analogues aux colorants naturels et/ou
(e) les oxydants.

16. Kit contenant
- le cas échéant un applicateur,
- au moins un conteneur C1, contenant un agent formant une nuance, l'agent formant une nuance pouvant être identique ou différent dans le cas de plusieurs conteneurs C1,
- au moins un corps en forme de tube,
où
- les corps en forme de tube présentent, comme forme de départ, la forme d'un cylindre creux déjà réalisé au préalable, qui est ouvert au niveau des deux surfaces, de base et supérieure, délimité par une enveloppe, l'enveloppe présentant un joint, s'étendant sur toute la hauteur du cylindre creux le long de la surface de l'enveloppe,
- la forme de départ étant stable, sans influence d'une force extérieure,
- les corps en forme de tube utilisés pouvant être ouverts sous l'influence d'une force externe en une forme ouverte le long du joint et
- les corps en forme de tube reprenant, à partir de la forme ouverte, sans l'influence d'une force extérieure, automatiquement, leur forme de départ.

17. Kit selon la revendication 16, **caractérisé en ce qu'**il contient en outre au moins un conteneur C2, contenant un agent de teinture pour fibres kératiniques.

18. Kit selon l'une quelconque des revendications 16 ou 17, **caractérisé en ce qu'**il comprend, au lieu du conteneur C1 pour la confection d'au moins un agent formant une nuance, comme agent à deux composants, un conteneur C1a et un conteneur C1b, le conteneur C1a contenant une composition contenant, dans un support cosmétique, au moins un composé choisi parmi
(a) les précurseurs de colorants par oxydation du type des composants de développement et le cas échéant des composants de couplage et/ou
(b) les composés carbonyle réactifs et/ou
(c) les colorants montant directement sur la fibre et/ou
(d) les précurseurs de colorants analogues aux colorants naturels et/ou
(e) les agents de renforcement de décoloration
et le conteneur C1b contenant une composition, contenant, dans un support cosmétique, au moins un oxydant.

19. Utilisation du kit selon l'une quelconque des revendications 16 ou 17 dans un procédé pour la teinture de fibres kératiniques selon l'une quelconque des revendications 1 à 15.
